# EUROPEAN PATENT APPLICATION

(11) **EP 4 659 733 A1**
(43) Date of publication of application: **10.12.2025**
(21) Application number: 23920143.7
(22) Date of filing: 08.12.2023
(51) Int. Cl.: A61K 8/41, A61K 8/49, A61K 8/27, A61Q 5/00, A61Q 5/02, A61Q 17/00, A01N 63/30, A01N 33/08

(54) **BACTERICIDAL AND PRESERVATIVE COMPOSITION**

(30) Priority: 01.02.2023 KR 20230013650; 01.02.2023 KR 20230013651; 01.02.2023 KR 20230013652; 16.08.2023 KR 20230106974; 06.12.2023 KR 20230175604
(71) Applicant: Bjbiochem Co., Ltd., Daejeon 34025 (KR)
(72) Inventor: JEONG, Gug In, Daejeon 34021 (KR); KIM, Na Rae, Daejeon 34070 (KR); LEE, Soo Hyeok, Daejeon 34048 (KR); KIM, Min Kyung, Daejeon 34014 (KR)
(74) Representative: AWA Sweden AB
(86) International application number: PCT/KR2023/020170
(87) International publication number: WO 2024/162594

(57) **Abstract**

The present invention relates to an antibacterial, bactericidal, and preservative composition and a product containing same, the composition protecting, from microorganisms, the quality of products in various industrial fields including home care products such as clothes detergents, dish detergents, and household cleaning agents, personal care products such as shampoos, body cleansers, wet wipes, and cleansing products, and cosmetic products such as creams and emulsions. The antibacterial and preservative composition according to the present invention comprises a compound represented by chemical formula 1 and a compound represented by chemical formula 2, 3, or 4, thereby solving problems such as harmfulness to the human body and skin irritation, and enhancing antibacterial activity. In addition, an antidandruff hair care cosmetic composition or an antifungal composition, comprising the compound represented by chemical formula 1 of the present invention, has the effect of controlling dandruff-causing bacteria, and has a synergistic effect when the compound represented by chemical formula 1 is used together with a high-cost dandruff therapeutic agent, such as piroctone olamine, zinc pyrithione, or climbazole. In addition, an antiviral composition comprising the compound represented by chemical formula 1 of the present invention effectively controls microorganisms such as viruses and thus can be used as an ecofriendly infectious disease prevention composition for virus control.

## Description

### [Technical Field]

The present invention relates to antibacterial, sterilizing and preservative compositions that protect products in various industrial fields, including home care products such as clothing detergents, dish detergents, and home cleaners, personal care products such as shampoos, body cleansers, wet tissues, and cleansers, and cosmetic products such as creams and emulsions.

Also, the present invention relates to an anti-dandruff hair care cosmetic composition and an anti-viral composition for removing dandruff bacteria.

### [Background Art]

All products containing water require antimicrobial agents because microorganisms readily propagate in water. Antimicrobial agents are agents that inhibit the growth of or kill microorganisms such as bacteria.

In particular, detergent products containing a great amount of water necessarily contain preservatives for preventing the proliferation and decay of microorganisms and antimicrobial agents for removing contaminant microorganisms in order to maintain quality thereof for a long time. These products require antimicrobial components because they have a very long shelf life, are very likely to come into contact with microorganisms during use and storage thereof, are blended with various carbon and nitrogen sources, and contain a high amount of water essential for microbial growth.

Recently, the harmfulness of antimicrobial agents to the human body has been controversial. However, if antimicrobial agents are not used in products or the antimicrobial property thereof is weak, there is a high risk of product deterioration and pathogen transmission, and microbial metabolism causes skin troubles and diseases. Therefore, antimicrobial agents are inevitably used.

Isothiazolinone ingredients such as methylisothiazolinone, chloromethylisothiazolinone, and 1,2-benzisothiazol-3(2H)-one, which are toxic chemicals, are still used as preservatives in clothes, dishes, household cleaning or fragrance products, wet wipes, shampoo, cosmetics (creams, lipstick, etc.) and the like.

The isothiazolinone ingredients, which are toxic chemicals, are most widely used due to economic efficiency and potent effects thereof, but are being increasingly regulated because they may act as carcinogens.

Recently, regulations for respective product groups have been strengthened in Korea as antimicrobial agents may affect public health. The movement to strengthen antimicrobial regulations is accelerating. For example, related laws and regulations are strengthened, such as wet wipes are classified into other sanitary products under the Sanitary Product Management Act, and the Ministry of Food and Drug Safety released a notice associated with usable raw materials for kitchen detergents. However, due to inappropriate use of antimicrobial agents, the application of highly safe and excellent antimicrobial agents is being narrowed.

Meanwhile, in complicate modern society, environmental pollution and stress affect the normal shedding of the skin, hair, and scalp, causing scalp diseases such as dandruff and itchiness. Dandruff is a condition in which the bonds between keratinocytes break and dead cells peel off from the scalp due to scalp abnormalities. Dandruff and itchiness are not life-threatening diseases, but negatively affect daily life, for example, give people a bad image and cause lack of confidence.

Dandruff is known to be the most common scalp skin disease. *Malassezia* fungus is the cause of dandruff and is commonly called "dandruff fungus". Among the *Malassezia* genus, *Malassezia furfur, Malassezia restricta,* and *Malassezia globosa* species are known to be mainly involved in dandruff. In addition, in various research cases around the world, Malassezia fungi were identified as the dominant species on the scalp of dandruff patients, and the population of Malassezia fungi was reported to increase depending on the severity of symptoms.

Shampoos, rinses, hair restorers, and the like for preventing excessive generation of dandruff are mixed with exfoliating agents, anti-seborrheic agents, disinfectants, etc. In addition, anti-inflammatory or anti-inflammatory agents may be added to prevent inflammation from worsening. Piroctone olamine, zinc pyrithione, and climbazole, which are currently known to be effective in treating dandruff, are expensive compounds and are rarely contained in cosmetic formulations such as shampoo.

Meanwhile, various virus quarantine products are used and quarantine substances are becoming more diverse. More effective and safer quarantine is required because these quarantine substances are used in living environments. Because there is a need for quarantine that encompasses not only bacteria but also viruses such as foot-and-mouth disease and coronavirus, which periodically increase in frequency, it is required to develop safer materials compared to current quarantine materials and quarantine agents that have a wide virus quarantine spectrum and are relatively less harmful.

It is stipulated that among currently commercialized products approved by the Ministry of Environment, quaternary ammonium salts such as -n-alkyl dimethyl ethyl benzyl ammonium chloride· alkyl benzyl dimethyl ammonium chloride (1:1), and benzalkonium chloride should not be used for the human body or items in direct contact with the human body and cannot be used to disinfect food or food containers. As such, directly contact or spraying of most of the quarantine products on the human body or livestock is not recommended, but in reality, non-contact is impossible and it is necessary to develop materials that are recognized as being as safe as possible.

Therefore, there is a need for development of a sterilizing and preservative agent that exhibits excellent antibacterial and antiviral properties while being safe to use in products including cosmetics, sanitary products, and household chemical products using substances that are non-toxic to humans and recognized as environmentally safe.

### [Disclosure]

### [Technical Problem]

While researching and developing a method to control microorganisms without using conventional antibacterial agents that harm the human body in various ways, the present inventors found that the compound represented by Formula 1 has an antibacterial effect.

In addition, the present inventors found that a mixture of a compound represented by Formula 1 with a compound represented by Formula 2, Formula 3, or Formula 4 can easily incorporate substances for controlling microorganisms into the product formulation, provide synergistic antibacterial, sterilizing, and preservative effects, and control microorganisms more efficiently, based on the effect of offsetting the decline in antibacterial, sterilizing and preservative functions due to the ionic nature of the product base, compared to the compound alone. Based on this finding, the present invention has been completed.

In addition, the present inventors found that the compound represented by Formula 1 is effective in sterilizing strains of the dandruff genus *Malassezia,* and that a mixture of the compound represented by Formula 1 with piroctone olamine, zinc pyrithione, or climbazole exhibits a synergistic sterilizing effect against strains of the *Malassezia* genus, and can reduce the amount of piroctone olamine, zinc pyrithione, and climbazole used, compared to the compound represented by Formula 1 alone, when applied to hair care cosmetics.

Furthermore, while researching and developing a method of controlling viruses without using conventional antivirals that harm the human body in various ways, the present inventors found that the compound represented by Formula 1 has an antiviral effect in addition to the antibacterial effect. Based on this finding, the present invention has been completed.

Therefore, the present invention is based on development of a sterilizing and preservative agent that exhibits antibacterial and antiviral properties.

### [Technical Solution]

The present invention discloses the following solutions to the above problems.

In accordance with one aspect of the present invention, provided is an antimicrobial and preservative composition containing a compound represented by Formula 1.

In accordance with one aspect of the present invention, provided is an antimicrobial and preservative composition containing (a) a compound represented by Formula 1 and (b) a compound represented by Formula 2, Formula 3 or Formula 4.

In accordance with another aspect of the present invention, provided is a product containing the antibacterial and preservative composition.

In accordance with another aspect of the present invention, provided is an antimicrobial method including treating bacteria with the antimicrobial and preservative composition.

In accordance with another aspect of the present invention, provided is an anti-dandruff hair care cosmetic composition containing the compound represented by Formula 1.

In accordance with another aspect of the present invention, provided is an anti-dandruff hair care cosmetic composition containing the compound represented by Formula 1 and any one compound selected from the group consisting of piroctone olamine, zinc pyrithione, and climbazole.

In accordance with another aspect of the present invention, provided is an antifungal composition against strains of *Malassezia sp.,* containing the compound represented by Formula 1.

In accordance with another aspect of the present invention, provided is a hair care cosmetic product provided in any one of various formulations containing the anti-dandruff hair care cosmetic composition or the anti-fungal composition.

In accordance with another aspect of the present invention, provided is a method of sterilizing dandruff bacteria including treating the dandruff bacteria with the anti-dandruff hair care cosmetic composition or anti-fungal composition.

In accordance with another aspect of the present invention, provided is an antiviral composition containing the compound represented by Formula 1.

In accordance with another aspect of the present invention, provided is an antiviral method including treating bacteria with the antiviral composition.

The antibacterial and preservative composition, the anti-dandruff composition, and antiviral composition using the compound represented by formula 1 of the present invention are advantageously capable of controlling a wide range of microorganisms and of solving problems such as harmfulness to the human body and skin irritation caused by preservatives.

### [Advantageous Effects]

The combination of the ingredients discovered by the present inventors exerts synergistic antimicrobial effects.

In addition, various products such as cosmetics, sanitary products, and household chemical products including the antimicrobial and preservative composition discovered by the present inventors enable emulsification and solubilization suitable for the characteristics of surfactants and exhibit excellent antimicrobial activity and a broad antimicrobial spectrum.

In addition, the antimicrobial and preservative composition of the present invention exhibits a wide range of efficacy in compositions based on anionic and nonionic surfactants and compositions based on cationic surfactants, thus being utilized in a wide range of applications.

In addition, the anti-dandruff hair care cosmetic composition or antifungal composition of the present invention has the effect of controlling dandruff bacteria, has a synergistic effect using a combination of the compound represented by Formula 1 with expensive dandruff-treating agents piroctone olamine, zinc pyrithione, or climbazole, and can reduce consumption of the expensive dandruff-treating agents by more than 50%.

In addition, the antiviral composition of the present invention is effectively capable of controlling microorganisms such as viruses and is thus used as an eco-friendly quarantine composition for virus control.

The effects of the present invention are not limited to those described above and various effects may be derived from the following description within the scope of those skilled in the art.

### [Best Mode]

Hereinafter, embodiments of the present invention will be described in more detail.

The detailed description will be given below. Meanwhile, each description and embodiment disclosed herein may also be applied to other descriptions and embodiments, respectively. That is, all combinations of various elements disclosed herein fall within the scope of the present invention. Also, the detailed description described below should be not construed as limiting the scope of the present invention.

Terms, such as "comprising", used herein should be considered as open-ended terms that may include other embodiments, unless specifically mentioned otherwise in the phrases or sentences including the term.

Terms and words used in the description and the claims are not construed as being limited to conventional meanings in dictionaries, but are construed to have meanings and concepts corresponding to the spirit and scope of the present invention based on a principle that the inventor can properly define concepts of the terms in order to explain the invention in the best way.

### Antimicrobial and preservative composition

The present invention provides an antimicrobial and preservative composition containing a compound represented by Formula 1: 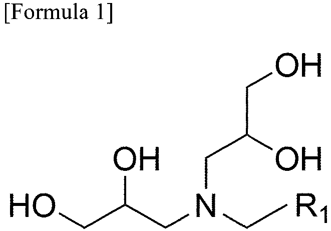 wherein R₁ is substituted or unsubstituted C₇-C₁₇ alkyl or substituted or unsubstituted C₇-C₁₇ alkenyl.

In addition, in Formula 1, R₁ is linear or branched C₇-C₁₇ alkyl, linear or branched C₇-C₁₇ alkenyl, or C₇-C₁₇ hydroxyalkyl.

More specifically, R₁ is linear or branched C₁₀-C₁₄ alkyl, linear or branched C₁₀-C₁₄ alkenyl, or C₁₀-C₁₄ hydroxyalkyl.

In the present invention, the antibacterial and preservative composition contains the compound represented by Formula 1 as an active ingredient.

As used herein, the term "active ingredient" refers to an ingredient that exhibits the desired activity alone or in combination with a carrier that is not active on its own.

The present invention provides an antimicrobial and preservative composition containing (a) a compound represented by Formula 1 and (b) a compound represented by Formula 2, Formula 3, or Formula 4: 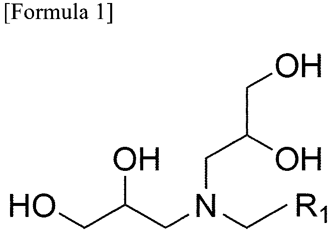 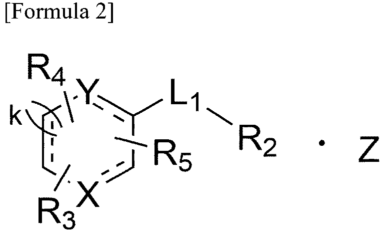 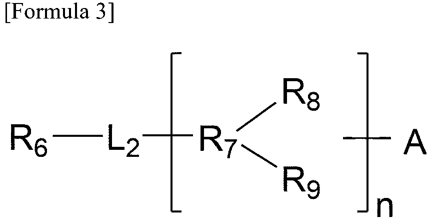 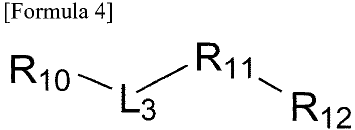 wherein
in Formula 1,
R₁ is substituted or unsubstituted C₇-C₁₇ alkyl, or substituted or unsubstituted C₇-C₁₇ alkenyl,
in Formula 2,
L₁ is -(CH=CH)-, -(CH₂)₁-, -C(=O)- or -O-,
R₂ is -H, -C(=O)-CH₃, -C(=O)H, -OH, -O⁻Na⁺, -(CH₂)ₘ-OH, -CH(CH₃)-CH₂-C(CH₃)₂-CH₃, or substituted or unsubstituted C₁-C₆ alkyl,
R₃, R₄ and R₅ are each independently -H, -OH, C₁-C₃ alkoxy, =O, -O⁻Na⁺, or substituted or unsubstituted C₁-C₆ alkyl,
X and Y are each independently -CH-, -O- or -NRₐ,
Rₐ is -OH,
Z is NH₂-CH₂-CH₂-OH or null,
-̅ -̅ -̅ represents a single or double bond, and
K, 1, and m are each independently an integer of 1 to 4,
in Formula 3,
R₆ is -H, substituted or unsubstituted C₂-C₁₈ alkyl, or substituted or unsubstituted C₂-C₁₈ alkenyl,
L₂ is -(CH₂)ₒ-, -O- or -C(=O)-,
R₇ is -NHCH-, -N- or C(=O)CHNH-,
R₈ is -OH, COOH, -COOCH₃, -COOCH₂CH₃, -(CH₂)₄NH- or 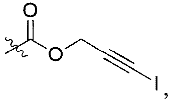
R₉ is -H, -(CH₂)₄NH₂ or -(CH₂)₃C(NH)₂NH₂,
A is -H or null,
N is an integer of 1 to 99, and
O is an integer of 1 to 3, and
in Formula 4,
L₃ is -(CH₂)ₚ-, -O-, -C(=O)- or -O-C(=O)-,
R₁₀ is substituted or unsubstituted C₂-C₁₀ alkyl, -OH or -O⁻Na⁺,
R₁₁ is -(CH₂)_{q}- substituted or unsubstituted with at least one substituent selected from the group consisting of -CH₂-CHOH-CH₂-, hydroxy, unsubstituted C₁-C₃ alkyl, and carboxyl, substituted or unsubstituted -(CH₂)ᵣ-N(R_{b})-(CH₂)ₛ- or unsubstituted phenylene (-C₆H₄-),
R₁₂ is -OH, -COOH, C₁-C₃ alkoxy or -COO⁻Na⁺,
R_{b} is -(CH₂)ₜ-N[(CH₂)ᵤ-CO₂⁻)]₂, and
p, q, r, s, t, and u are each independently an integer of 1 to 3.

As used herein, the term "antimicrobial and preservative composition" refers to an antiseptic composition, may encompass all additives that may be used to prevent deterioration, decay, discoloration, and chemical change of the composition to which it is added, and may also encompass functional antibiotics for suppressing the proliferation of microorganisms such as bacteria, molds, and yeasts to inhibit the growth of or kill decaying microorganisms.

In the present invention, the antimicrobial and preservative composition may have antibacterial or antifungal activity against bacteria or fungi, but is not limited thereto.

As used herein, the term "antibacterial" refers to an action of inhibiting or controlling the growth and proliferation of bacteria.

As used herein, the term "antifungal" means an action of inhibiting or controlling the growth and proliferation of fungi.

In the present invention, the antimicrobial and preservative composition may have antibacterial activity against *Escherichia coli, Pseudomonas aeruginosa, Staphylococcus aureus, Klebsiella pneumoniae,* MRSA (*methicillin-resistant Staphylococcus aureus*), *Bacillus subtilis, Vibrio parahaemolyticus, Salmonella typhimurium, Listeria monocytogenes* and *Shigella flexneri,* or have antifungal activity against *Candida albicans, Aspergillus niger,* or *Aspergillus brasiliensis,* but is not limited thereto.

In the present invention, the compounds represented by Formula 1 or Formulas 2 to 4 are preservative compounds. The term used herein "preservative" refers to a chemical substance that is added to regulate the quality of industrially produced and distributed processed food, beverages, cosmetics, medicines, sanitary products, household chemicals, and the like, and is used to prevent deterioration of quality of products due to decay by microorganisms or decomposition of chemical components. That is, the preservative exhibits antibacterial activity against at least one microorganism of bacteria, molds, and yeasts, and thus is used as an antimicrobial, antibacterial and/or antifungal agent.

In the present invention, the antimicrobial and preservative composition may be used to control at least one microorganism selected from the group consisting of *Escherichia coli, Pseudomonas aeruginosa, Staphylococcus aureus, Klebsiella pneumoniae,* MRSA (*methicillin-resistant Staphylococcus aureus), Bacillus subtilis, Vibrio parahaemolyticus, Salmonella typhimurium, Listeria monocytogenes, Shigella flexneri, Candida albicans, Aspergillus niger,* and *Aspergillus brasiliensis,* but is not limited thereto.

Specifically, the antimicrobial and preservative composition may be used to control at least one microorganism selected from the group consisting of *Escherichia coli, Staphylococcus aureus, Pseudomonas aeruginosa, Aspergillus brasiliensis,* and *Candida albicans.*

In the present invention, the compound represented by Formula 1 and the compound represented by Formula 2, Formula 3, or Formula 4 may be present in a weight ratio of 0.01:9.99 to 9.99:0.01, specifically the compound represented by Formula 1 and the compound represented by Formula 2, Formula 3, or Formula 4 may be present in a weight ratio of 2:8, 4:6, 5:5, 7:3, and/or 8:2, but the present invention is not limited thereto.

Specifically, the compound represented by Formula 1 and the compound represented by Formula 2, Formula 3, or Formula 4 are present in a content ratio within the range defined above, thereby advantageously improving the microbial control effect.

In the present invention, 3,3'-(dodecylimino)bispropane-1,2-diol, which is the compound represented by Formula 1, has the following structure:

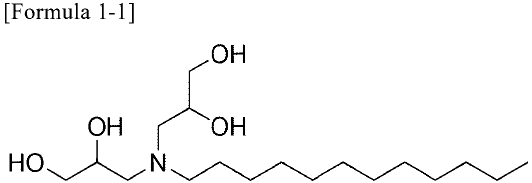

Specifically, the compound represented by Formula 1-1 exhibits excellent emulsification and solubilization effects and is mixed with a compound represented by Formula 2, Formula 3 or Formula 4 to produce an antimicrobial composition, which is capable of inhibiting microorganisms such as *Escherichia coli, Staphylococcus aureus, Pseudomonas aeruginosa, Klebsiella pneumoniae, MRSA (methicillin-resistant Staphylococcus aureus), Bacillus subtilis, Vibrio parahaemolyticus, Salmonella typhimurium, Listeria monocytogenes, Shigella flexneri, Candida albicans, Aspergillus niger,* and *Aspergillus brasiliensis,* thus exhibits antibacterial and antifungal properties against these microorganisms, and is advantageously applicable to various products such as liquid and solid cosmetics, sanitary products, and household chemical products.

In the present invention, the compound represented by Formula 2 may include at least one selected from the group consisting of o-methoxycinnamaldehyde, 4-methoxycinnamaldehyde, raspberry ketone, 3-phenyl-1-propanol, hydroxy acetophenone, sodium benzoate, phenoxyethanol, tropolone, piroctone olamine, and sodium dehydroacetate, but is not limited thereto.

Specifically, the o-methoxycinnamaldehyde has a structure of 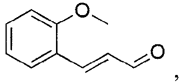 the 4-methoxycinnamaldehyde has a structure of 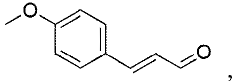 the raspberry ketone has a structure of 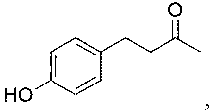 the 3-phenyl-1-propanol has a structure of 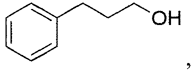 the 4-hydroxy acetophenone has a structure of 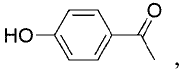 the sodium benzoate has a structure of 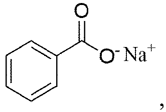 the phenoxyethanol has a structure of 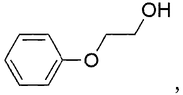 the tropolone has a structure of 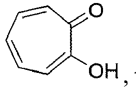 the piroctone olamine has a structure of 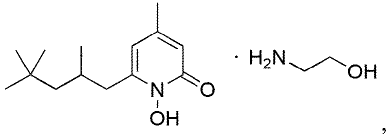 and the sodium dehydroacetate has a structure of

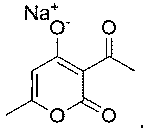

In the present invention, the compound represented by Formula 3 may include at least one selected from the group consisting of lauroyl arginine methyl ester, lauroyl lysine methyl ester, lauroyl arginine ethyl ester, iodopropynyl butylcarbamate, polylysine, lauroyl lysine, and caprylhydroxamic acid, but is not limited thereto.

Specifically, the lauroyl arginine methyl ester has a structure of 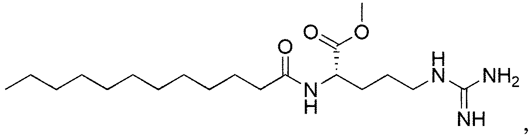 the lauroyl lysine methyl ester has a structure of 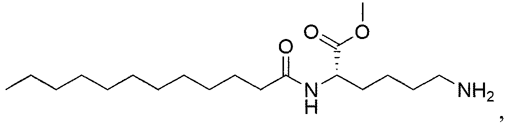 the lauroyl arginine ethyl ester has a structure of 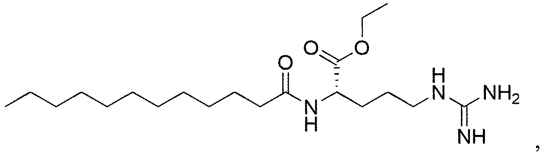 the iodopropynyl butylcarbamate has a structure of 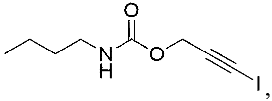 the polylysine has a structure of 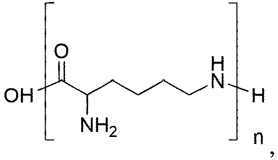 the lauroyl lysine has a structure of 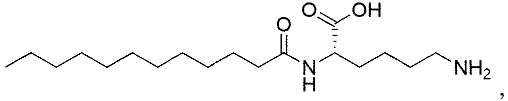 and the caprylhydroxamic acid has a structure of

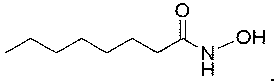

In the present invention, the compound represented by Formula 4 may include at least one selected from the group consisting of hexane-1,2-diol, octane-1,2-diol, decane-1,2-diol, ethylhexylglycerin, glyceryl monocaprylate, octyl glycerin, hexyl glycerin, levulinic acid, citric acid, sodium anisate, disodium EDTA, and tetrasodium EDTA, but is limited thereto.

Specifically, the hexane-1,2-diol has a structure of 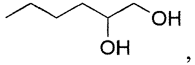 the octane-1,2-diol has a structure of 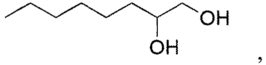 the decane-1,2-diol has a structure of 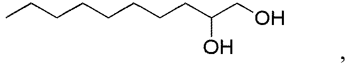 the ethylhexylglycerin has a structure of 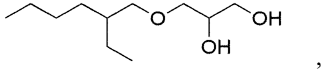 the glyceryl monocaprylate has a structure of 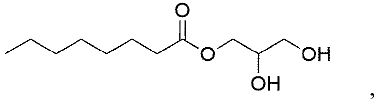 the octyl glycerin has a structure of 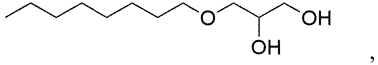 the hexyl glycerin has a structure of 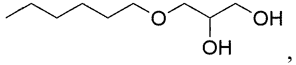 the levulinic acid has a structure of 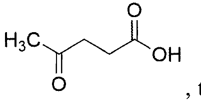 the citric acid has a structure of 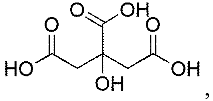 the sodium anisate has a structure of 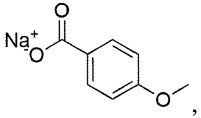 the disodium EDTA has a structure of 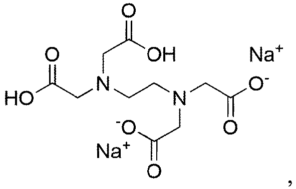 and the tetrasodium EDTA has a structure of

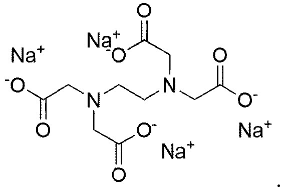

A combination of the compound represented by Formula 2, Formula 3, or Formula 4 with the compound represented by Formula 1 is used as an ingredient for the antibacterial and preservative composition, thereby inhibiting the growth of microorganisms such as bacteria, molds and yeasts, inhibiting the growth of decaying microorganisms, and exhibiting synergistic sterilizing activity, compared to when each compound is used alone.

### Products containing antimicrobial and preservative composition

The present invention provides a product including the antimicrobial and preservative composition.

In the present invention, the product may be a cosmetic, sanitary product or household chemical product, but is not limited thereto.

As used herein, the term "cosmetic" refers to a product that is used such as by applying to the human body, rubbing, or spraying or in a similar manner thereto, to clean and beautify the human body to add attractiveness, brighten the appearance, or maintain or promote the health of the skin and hair, while having almost no or less effects on the human body.

As used herein, the term "sanitary product" refers to a product that requires special sanitary management in order to secure health and sanitary.

As used herein, the term "household chemical product" refers to a chemical product used in everyday living spaces such as homes, offices, and multi-use facilities, which may cause exposure of chemicals to people or the environment.

In the present invention, the cosmetic may be prepared in any formulation conventionally prepared in the art according to the Cosmetics Act, and may be formulated into any one selected from the group consisting of solutions, suspensions, emulsions, pastes, gels, creams, pacts, powders, emulsion foundations, wax foundations, sprays, soaps, face cleansers, body cleansers, hair shampoos and hair rinses, but is not limited thereto. More specifically, the cosmetic may be formulated into a softening lotion, nourishing lotion, cream, nourishing cream, massage cream, lipstick, pact, essence, eye cream, cleansing cream, cleansing foam, cleansing water, pack, or spray formulation.

In the present invention, according to the Sanitary Product Management Act, the sanitary product may be selected from the group consisting of dishwashing detergents, dishwasher detergents, and wet wipe, but is not limited thereto.

In the present invention, according to the Consumer Chemical Products and Biocides Safety Act, the household chemical product may be selected from the group consisting of laundry detergents, residential cleaners, deodorants, air cleaners, indoor air fresheners, fragrances, fabric softeners, multipurpose cleaners, disinfectants, antimicrobials, sanitizers, and fungistats, but is not limited thereto.

In the present invention, the antimicrobial and preservative composition may be present in an amount of 0.01 to 10% by weight with respect to the weight of the cosmetic, sanitary or household chemical product, but is not limited thereto.

Specifically, the product may be selected from the group consisting of a liquid detergent composition for clothes, a dishwashing detergent composition, a stock wet wipe composition, a stock shampoo composition, a stock cosmetic cream composition, and a cosmetic pack composition, but is not limited thereto.

When the product including the antimicrobial and preservative composition is a liquid detergent composition for clothes, a dishwashing detergent composition, a stock wet wipe composition, a stock shampoo composition, a stock cosmetic cream composition, or a cosmetic pack composition, the product includes an antimicrobial and preservative composition containing 3,3'-(dodecylimino)bispropane-1,2-diol represented by Formula 1-1, or a combination of 3,3'-(dodecylimino)bispropane-1,2-diol represented by Formula 1-1 and any one compound of o-methoxycinnamaldehyde, 4-methoxycinnamaldehyde, raspberry ketone, 3-phenyl-1-propanol, hydroxy acetophenone, sodium benzoate, phenoxyethanol, tropolone, piroctone olamine and sodium dehydroacetate, all of which are represented by Formula 2, thereby effectively controlling *E. coli, S. Aureus, P. Aeruginosa, A. brasiliensis* and C. *albicans.*

In addition, when the product including the antimicrobial and preservative composition is a liquid detergent composition for clothes, a dishwashing detergent composition, a stock wet wipe composition, a stock shampoo composition, a stock cosmetic cream composition, or a cosmetic pack composition, the product includes an antimicrobial and preservative composition containing a combination of 3,3'-(dodecylimino)bispropane-1,2-diol represented by Formula 1-1 and any one compound of lauroyl arginine methyl ester, lauroyl lysine methyl ester, lauroyl arginine ethyl ester, iodopropynyl butylcarbamate, polylysine, lauroyl lysine, and caprylhydroxamic acid, all of which are represented by Formula 3, thereby effectively controlling *E. coli, S. aureus, P. aeruginosa, A. brasiliensis* and C. *albicans.*

In addition, when the product including the antimicrobial and preservative composition is a liquid detergent composition for clothes, a dishwashing detergent composition, a stock wet wipe composition, a stock shampoo composition, a stock cosmetic cream composition, or a cosmetic pack composition, the product includes an antimicrobial and preservative composition containing a combination of 3,3'-(dodecylimino)bispropane-1,2-diol represented by Formula 1-1 and any one compound of hexane-1,2-diol, octane-1,2-diol, decane-1,2-diol, ethylhexylglycerin, glyceryl monocaprylate, octyl glycerin, hexyl glycerin, levulinic acid, citric acid, sodium anisate, disodium EDTA, and tetrasodium EDTA, all of which are represented by Formula 4, thereby effectively controlling *E. coli, S. aureus, P. aeruginosa, A. brasiliensis* and C. *albicans.*

### Antimicrobial method

The present invention provides an antimicrobial method including treating bacteria with the antimicrobial and preservative composition.

The method for treating bacteria with the antimicrobial and preservative composition may be appropriately used according to conventional methods well known to those skilled in the art in various fields requiring antibacterial activity. The amount of the antimicrobial and preservative composition that is treated may be appropriately determined depending on the purpose of use.

Matters mentioned in the antimicrobial and preservative composition, the product including the antimicrobial and preservative composition, and the antimicrobial method of the present invention are equally applied unless contradictory to each other.

### Anti-dandruff hair care cosmetic composition

The present invention provides an anti-dandruff hair care cosmetic composition containing a compound represented by Formula 1: 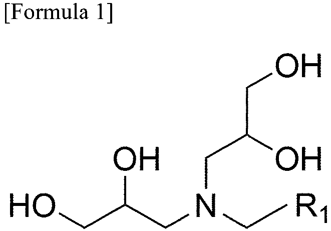 wherein R₁ is substituted or unsubstituted C₇-C₁₇ alkyl or substituted or unsubstituted C₇-C₁₇ alkenyl.

In addition, in Formula 1, R₁ is linear or branched C₇-C₁₇ alkyl, linear or branched C₇-C₁₇ alkenyl, or C₇-C₁₇ hydroxyalkyl.

More specifically, R₁ is linear or branched C₁₀-C₁₄ alkyl, linear or branched C₁₀-C₁₄ alkenyl or C₁₀-C₁₄ hydroxyalkyl.

In the present invention, the anti-dandruff hair care cosmetic composition contains the compound represented by Formula 1 as an active ingredient.

In the present invention, the alkyl of the compound represented by Formula 1 may have 7 to 17 carbon atoms, and specifically, may have 3,3'-(dodecylimino)bispropane-1,2-diol, which has the following Formula 1-1: 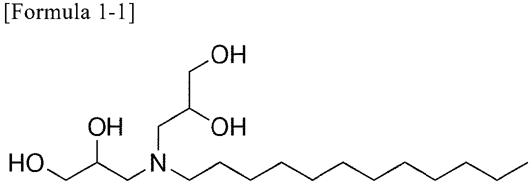 wherein R₁ is C₁₁ alkyl.

In the present invention, the cosmetic composition may further contain a compound represented by Formula 5.

[Formula 5] R₁₃ - L₄ · W

wherein
L₄ is 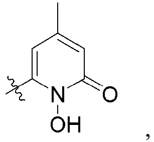 -Zn or 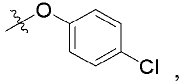
R₁₃ is CH₃-C(CH₃)₂-CH₂-CH(CH₃)-CH₂-, 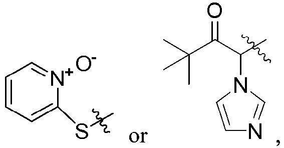
W is NH₂-CH₂-CH₂-OH, 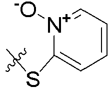 or null.

Specifically, in the compound represented by Formula 5, L₄ is 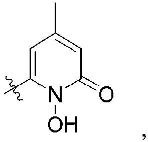 R₁₃ is CH₃-C(CH₃)₂-CH₂-CH(CH₃)-CH₂-, and W is NH₂-CH₂-CH₂-OH.

In the compound represented by Formula 5, L₄ is -Zn, R₁₃ is 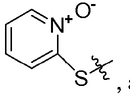 and W is

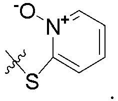

In the compound represented by Formula 5, L₄ is 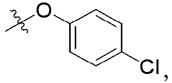 R₁₃ is 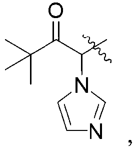 and W is null.

In the present invention, the compound represented by Formula 5 may include at least one selected from the group consisting of piroctone olamine, zinc pyrithione, and climbazole, but is not limited thereto.

Specifically, the piroctone olamine has a structure of 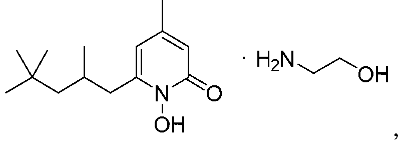 the zinc pyrithione has a structure of 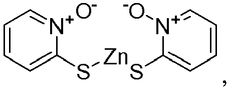 and the climbazole has a structure of

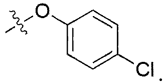

In the present invention, the cosmetic compound may further include any one compound selected from the group consisting of piroctone olamine, zinc pyrithione, and climbazole.

The compound represented by Formula 1 and the compound represented by Formula 5 may be present in the hair care cosmetic composition at a weight ratio of 9:1 to 1:9.

The compound represented by Formula 1 and any one compound selected from the group consisting of piroctone olamine, zinc pyrithione, and climbazole may be present in the hair care cosmetic composition at a weight ratio of 9:1 to 1:9, and at a weight ratio of 7:3 to 1:9 in terms of dandruff bacteria control, and at a weight ratio of 9:1 to 5:5 in terms of economic efficiency.

The cosmetic composition has a sterilizing effect against strains of the *Malassezia sp.,* specifically, at least one fungus selected from the group consisting of *Malassezia furfur, Malassezia restricta, Malassezia globosa, Malassezia sympodialis, Malassezia slooffiae, Malassezia pachydermatis,* and *Malassezia obtusa,* and thus may be applied without limitation to strains belonging to the genus *Malassezia sp.*

Compared to when a cosmetic composition is prepared using the compound represented by Formula 1 alone, when a cosmetic composition is prepared by mixing the compound represented by Formula 1 with any one compound selected from the group consisting of piroctone olamine, zinc pyrithione, and climbazole, the composition exhibits synergistic sterilizing effect against the dandruff fungus, *Malassezia* sp. and exhibits similar or better sterilizing activity against *Malassezia* sp. although the compound represented by Formula 1 is used instead of 50% or more, specifically, 70 to 80% of the expensive dandruff-treating agent, piroctone olamine, zinc pyrithione, or climbazole.

### Antifungal composition

The present invention provides an antifungal composition against strains of *Malassezia sp.,* containing the compound represented by Formula 1.

The compound represented by Formula 1 is described above.

In the present invention, the antifungal compound may further include the compound represented by Formula 5, specifically, any one compound selected from the group consisting of piroctone olamine, zinc pyrithione, and climbazole.

The compound represented by Formula 1 and the compound represented by Formula 5, specifically, any one compound selected from the group consisting of piroctone olamine, zinc pyrithione, and climbazole, may be present at a weight ratio of 7:3 to 1:9 in terms of dandruff bacteria control, and at a weight ratio of 9:1 to 5:5 in terms of economic efficiency.

The compound represented by Formula 1 and any one compound selected from the group consisting of piroctone olamine, zinc pyrithione, and climbazole may be present in the hair care cosmetic composition at a weight ratio of 9:1 to 1:9, and at a weight ratio of 7:3 to 1:9 in terms of dandruff bacteria control, and at a weight ratio of 9:1 to 5:5 in terms of economic efficiency.

The antifungal composition has a sterilizing effect against strains of the *Malassezia sp.,* specifically, at least one fungus selected from the group consisting of *Malassezia furfur, Malassezia restricta, Malassezia globosa, Malassezia sympodialis, Malassezia slooffiae, Malassezia pachydermatis,* and *Malassezia obtusa,* and thus may be applied without limitation to strains belonging to the genus *Malassezia sp.*

Compared to when an antifungal composition is prepared using the compound represented by Formula 1 alone, when an antifungal composition is prepared by mixing the compound represented by Formula 1 with any one compound selected from the group consisting of piroctone olamine, zinc pyrithione, and climbazole, the composition exhibits synergistic sterilizing effect against the dandruff fungus, *Malassezia* sp. and exhibits similar or better sterilizing activity against *Malassezia* sp. although the compound represented by Formula 1 is used instead of 50% or more, specifically, 70 to 80% of the expensive dandruff-treating agent, piroctone olamine, zinc pyrithione, or climbazole.

### Hair care cosmetic

The present invention provides a hair care cosmetic composition containing the anti-dandruff hair care cosmetic composition or the anti-fungal composition.

The hair care cosmetics have an anti-dandruff effect and an anti-fungal effect against dandruff bacteria.

As used herein, the term "cosmetic product" refers to products that are applied, rubbed, sprayed or the like onto the human body to clean and beautify the human body to make more attractive, brighten the appearance, or to maintain or promote skin and hair health, and have a mild effect on the human body.

In the present invention, the cosmetic product may be prepared in any formulation commonly used in the art according to the Cosmetics Act. The hair care cosmetic product may be formulated into any one selected from the group consisting of hair tonic, hair cream, hair pack, hair lotion, hair essence, hair nutrition, hair shampoo, hair rinse, hair conditioner, hair spray, hair gel, hair treatment, hair oil, hair wax, hair bleach and hair dye.

The anti-dandruff hair care cosmetic composition or the anti-fungal composition may be present in an amount of 0.01 to 2.0% by weight based on the hair care cosmetic product, and even when present in a small amount, the anti-dandruff hair care cosmetic composition or the anti-fungal composition exhibits excellent sterilizing power against Malassezia sp. strains. The anti-dandruff hair care cosmetic composition or antifungal composition contains the compound represented by Formula 1 and a compound represented by Formula 5, specifically piroctone olamine, zinc pyrithione, or climbazole.

The compound represented by Formula 1 and the compound represented by Formula 5, specifically, any one compound selected from the group consisting of piroctone olamine, zinc pyrithione, and climbazole, may be present at a weight ratio of 7:3 to 1:9 in terms of dandruff bacteria control, and at a weight ratio of 9:1 to 5:5 in terms of economic efficiency.

Compared to the anti-dandruff hair care cosmetic composition or antifungal composition containing the compound represented by Formula 1 alone, and any one compound selected from the group consisting of piroctone olamine, zinc pyrithione, and climbazole alone, the anti-dandruff hair care cosmetic composition or antifungal composition containing a combination of the compound represented by Formula 1 and any one compound selected from the group consisting of piroctone olamine, zinc pyrithione, and climbazole is effectively capable of controlling *Malassezia* sp. strains at a low dose (concentration).

### Method of sterilizing dandruff bacteria

The present invention provides a method of sterilizing dandruff bacteria including applying the anti-dandruff hair care cosmetic composition or the anti-fungal composition to a subject.

The sterilization method of the present invention includes applying the anti-dandruff hair care cosmetic composition or the anti-fungal composition to the subject.

The dandruff bacteria are strains of the *Malassezia* sp., and specifically include at least one selected from the group consisting of *Malassezia furfur, Malassezia restricta, Malassezia globosa, Malassezia sympodialis, Malassezia slooffiae, Malassezia pachydermatis,* and *Malassezia obtusa* and may be applied without limitation to strains belonging to the genus Malassezia (Malassezia sp.).

Since the composition has strong sterilizing activity against the *Malassezia* sp. strain, the composition may be applied to a subject to sterilize and control dandruff bacteria.

As used herein, the term "application" refers to bringing the anti-dandruff hair care cosmetic composition or anti-fungal composition into contact with the scalp or hair of a subject using any appropriate method. The application enables the ingredients of the composition to act on dandruff bacteria and provide sterilizing activity. The treatment amount of the anti-dandruff hair care cosmetic composition or antifungal composition may be appropriately determined depending on the purpose of use.

The subject to which the anti-dandruff hair care cosmetic composition or antifungal composition of the present invention is applied includes, but is not limited to, a mammal including humans and may be, for example, a cow, a pig, a horse, a rabbit, a rat, or a human.

In addition, the dose of the anti-dandruff hair care cosmetic composition or anti-fungal composition of the present invention may be appropriately adjusted depending on subject differences such as age, degree of lesion, and the like or formulation, and the anti-dandruff hair care cosmetic composition or anti-fungal composition may be applied at an appropriate dose to the scalp once or several times a day over a week to several months.

The details described regarding the anti-dandruff hair care cosmetic composition, the anti-fungal composition or the hair care cosmetic product containing these compositions, and sterilization method according to the present invention are equally applicable unless they contradict each other.

### Antiviral composition

The present invention provides an antiviral composition containing the compound represented by Formula 1. 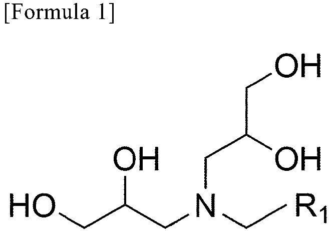 wherein R₁ is substituted or unsubstituted C₇-C₁₇ alkyl or substituted or unsubstituted C₇-C₁₇ alkenyl.

In the present invention, the antiviral composition contains the compound represented by Formula 1 as an active ingredient.

As used herein, the term "antiviral" refers to the ability to reduce, prevent, inhibit or eliminate the proliferation, growth or survival of viruses, or to reduce, inhibit or eliminate the activity of viruses.

The term "antiviral composition" of the present invention acts to reduce, prevent, inhibit or eliminate the proliferation, growth or survival of viruses, or to reduce, inhibit or eliminate the activity of viruses.

In the present invention, the compound represented by Formula 1 is 3,3'-(dodecylimino)bispropane-1,2-diol, has the structure represented by Formula 1-1, and is a nonionic surfactant. 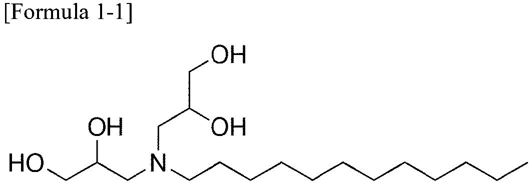 wherein R₁ is C₁₁ alkyl.

The antiviral composition may be used as an aqueous solution of 0.1 to 5% (w/v) prepared by diluting in a liquid such as water. Such an aqueous solution can also exhibit an antiviral effect.

The antiviral composition of the present invention has an antiviral or virucidal effect against viruses.

The virus may include one or more selected from the group consisting of coronavirus, influenza virus, norovirus, rotavirus, respiratory syncytial virus, avian influenza virus, foot-and-mouth disease virus, and African swine fever virus.

Specifically, the viruses include a variety of viruses such as avian influenza (AIV) that spreads to livestock and animals, especially highly pathogenic avian influenza virus, Picornaviridae Aphthovirus, and African swine fever virus (ASFV) as well as human-transmitted viruses, such as norovirus, rotavirus, coronavirus (human coronavirus 229E, OC43), human influenza virus A, B, and C, influenza A virus subtype H1N1, and respiratory syncytial virus (RSV), which cause respiratory infections.

In the present invention, the antiviral composition is also useful as an eco-friendly quarantine composition. As used herein, the term "quarantine" means preventing infectious diseases from occurring or spreading.

### Antiviral method

The present invention provides an antiviral method including treating virus with the antiviral composition.

The bacteria may be treated with the antiviral composition appropriately according to conventional methods well known to those skilled in the art in various fields where antiviral efficacy is required. The amount of the antiviral composition treated may be appropriately determined depending on the purpose of use.

The details described regarding the antiviral composition and antiviral method of the present invention are equally applicable unless they contradict each other.

### [Mode for Invention]

Hereinafter, various products to which the composition of the present invention is applied will be evaluated so that those skilled in the art can easily implement the present invention with reference to preferred embodiments. However, the present invention may be implemented in many different forms and is not limited to the following Example.

### [Test A: Antibacterial effects of compounds represented by Formula 1 and Formula 21

### Examples 1 to 10: Preparation of antibacterial and preservative compositions using compounds represented by Formula 1 and Formula 2

### Example 1.

The 3,3'-(dodecylimino)bispropane-1,2-diol represented by Formula 1 and the o-methoxycinnamaldehyde represented by Formula 2 were mixed at a weight ratio of 2:8, 5:5, or 8:2 to prepare an antibacterial and preservative composition.

### Example 2.

The 3,3'-(dodecylimino)bispropane-1,2-diol represented by Formula 1 and the 4-methoxycinnamaldehyde represented by Formula 2 were mixed at a weight ratio of 2:8, 5:5, or 8:2 to prepare an antibacterial and preservative composition.

### Example 3.

The 3,3'-(dodecylimino)bispropane-1,2-diol represented by Formula 1 and the raspberry ketone represented by Formula 2 were mixed at a weight ratio of 2:8, 5:5, or 8:2 to prepare an antibacterial and preservative composition.

### Example 4.

The 3,3'-(dodecylimino)bispropane-1,2-diol represented by Formula 1 and the 3-phenyl-1-propanol represented by Formula 2 were mixed at a weight ratio of 2:8, 5:5, or 8:2 to prepare an antibacterial and preservative composition.

### Example 5.

The 3,3'-(dodecylimino)bispropane-1,2-diol represented by Formula 1 and the hydroxy acetophenone represented by Formula 2 were mixed at a weight ratio of 2:8, 5:5, or 8:2 to prepare an antibacterial and preservative composition.

### Example 6.

The 3,3'-(dodecylimino)bispropane-1,2-diol represented by Formula 1 and the sodium benzoate represented by Formula 2 were mixed at a weight ratio of 2:8, 5:5, or 8:2 to prepare an antibacterial and preservative composition.

### Example 7.

The 3,3'-(dodecylimino)bispropane-1,2-diol represented by Formula 1 and the phenoxyethanol represented by Formula 2 were mixed at a weight ratio of 2:8, 5:5, or 8:2 to prepare an antibacterial and preservative composition.

### Example 8.

The 3,3'-(dodecylimino)bispropane-1,2-diol represented by Formula 1 and the tropolone represented by Formula 2 were mixed at a weight ratio of 2:8, 5:5, or 8:2 to prepare an antibacterial and preservative composition.

### Example 9.

The 3,3'-(dodecylimino)bispropane-1,2-diol represented by Formula 1 and the piroctone olamine represented by Formula 2 were mixed at a weight ratio of 2:8, 5:5, or 8:2 to prepare an antibacterial and preservative composition.

### Example 10.

The 3,3'-(dodecylimino)bispropane-1,2-diol represented by Formula 1 and the sodium dehydroacetate represented by Formula 2 were mixed at a weight ratio of 2:8, 5:5, or 8:2 to prepare an antibacterial and preservative composition.

### Comparative Examples 1 to 11: Preparation of antibacterial and preservative compositions using compounds represented by Formula 1 or Formula 2

### Comparative Example 1.

The 3,3'-(dodecylimino)bispropane-1,2-diol represented by Formula 1 was used.

### Comparative Example 2.

The o-methoxycinnamaldehyde represented by Formula 2 was used.

### Comparative Example 3.

The 4-methoxycinnamaldehyde represented by Formula 2 was used.

### Comparative Example 4.

The raspberry ketone represented by Formula 2 was used.

### Comparative Example 5.

The 3-phenyl-1-propanol represented by Formula 2 was used.

### Comparative Example 6.

The hydroxy acetophenone represented by Formula 2 was used.

### Comparative Example 7.

The sodium benzoate represented by Formula 2 was used.

### Comparative Example 8.

The phenoxyethanol represented by Formula 2 was used.

### Comparative Example 9.

The tropolone represented by Formula 2 was used.

### Comparative Example 10.

The piroctone olamine represented by Formula 2 was used.

### Comparative Example 11.

The sodium dihydroacetate represented by Formula 2 was used.

### Preparation Examples and Preparation Comparative Examples

Substances used in the following Preparation Examples and Preparation Comparative Examples were purchased from Sigma-Aldrich Korea, TCI, and the like. Cosmetic products, sanitary products, or household chemical products of Preparation Examples were prepared using the antibacterial and preservative compositions of Examples containing the 3,3'-(dodecylimino)bispropane-1,2-diol represented by Formula 1 and the compound represented by Formula 2 at a weight ratio of 8:2. Cosmetic products, sanitary products, or household chemical products of Preparation Comparative Examples were prepared using the antibacterial and preservative compositions of Comparative Examples containing the compound represented by Formula 1 or the compound represented by Formula 2 alone.

### Preparation Examples 1 to 10: Liquid detergent composition for clothes

### Preparation Example 1.

A liquid detergent composition for clothes containing 6% by weight of laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Example 1 was prepared.

### Preparation Examples 2 to 10.

Liquid detergent compositions for clothes according to Preparation Examples 2, 3, 4, 5, 6, 7, 8, 9, and 10 were sequentially prepared in the same manner as in Preparation Example 1, except that the antimicrobial and preservative compositions prepared in accordance with Examples 2, 3, 4, 5, 6, 7, 8, 9 and 10 were used instead of the antimicrobial and preservative composition prepared in accordance with Example 1.

### Preparation Examples 11 to 20: Dishwashing detergent compositions

### Preparation Example 11.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Example 1 was prepared.

### Preparation Examples 12 to 20.

Dishwashing detergent compositions according to Preparation Examples 12, 13, 14, 15, 16, 17, 18, 19, and 20 were sequentially prepared in the same manner as in Preparation Example 11, except that the antimicrobial and preservative compositions prepared in accordance with Examples 2, 3, 4, 5, 6, 7, 8, 9 and 10 were used instead of the antimicrobial and preservative composition prepared in accordance with Example 1.

### Preparation Examples 21 to 30: Stock wet wipe compositions

### Preparation Example 21.

A stock wet wipe composition containing 1% by weight of a natural extract, 98.7% by weight of purified water, and 0.3% by weight of the antimicrobial and preservative composition prepared in accordance with Example 1 was prepared.

### Preparation Examples 22 to 30.

Stock wet wipe compositions according to Preparation Examples 22, 23, 24, 25, 26, 27, 28, 29, and 30 were sequentially prepared in the same manner as in Preparation Example 21, except that the antimicrobial and preservative compositions prepared in accordance with Examples 2, 3, 4, 5, 6, 7, 8, 9 and 10 were used instead of the antimicrobial and preservative composition prepared in accordance with Example 1.

### Preparation Examples 31 to 40: Stock shampoo compositions

### Preparation Example 31.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Example 1 were prepared.

### Preparation Examples 32 to 40.

Stock shampoo compositions according to Preparation Examples 32, 33, 34, 35, 36, 37, 38, 39, and 40 were sequentially prepared in the same manner as in Preparation Example 31, except that the antimicrobial and preservative compositions prepared in accordance with Examples 2, 3, 4, 5, 6, 7, 8, 9 and 10 were used instead of the antimicrobial and preservative composition prepared in accordance with Example 1.

### Preparation Examples 41 to 50: Stock cosmetic cream compositions

### Preparation Example 41.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Example 1 was prepared.

### Preparation Examples 42 to 50.

Stock cosmetic cream compositions according to Preparation Examples 42, 43, 44, 45, 46, 47, 48, 49, and 50 were sequentially prepared in the same manner as in Preparation Example 41, except that the antimicrobial and preservative compositions prepared in accordance with Examples 2, 3, 4, 5, 6, 7, 8, 9 and 10 were used instead of the antimicrobial and preservative composition prepared in accordance with Example 1.

### Preparation Examples 51 to 60: Cosmetic pact (solid color) compositions

### Preparation Example 51.

A pact (solid color) composition containing 25% by weight of a spherical powder (silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Example 1 was prepared.

### Preparation Examples 52 to 60.

Pact (solid color) compositions according to Preparation Examples 52, 53, 54, 55, 56, 57, 58, 59, and 60 were sequentially prepared in the same manner as in Preparation Example 51, except that the antimicrobial and preservative compositions prepared in accordance with Examples 2, 3, 4, 5, 6, 7, 8, 9 and 10 were used instead of the antimicrobial and preservative composition prepared in accordance with Example 1.

### Preparation Comparative Examples 1 to 11: Liquid detergent compositions for clothes

### Preparation Comparative Example 1.

A liquid detergent composition for clothes containing 6% by weight of laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Comparative Example 1 was prepared.

### Preparation Comparative Examples 2 to 11

Liquid detergent compositions for clothes according to Preparation Comparative Examples 2, 3, 4, 5, 6, 7, 8, 9, 10 and 11 were sequentially prepared in the same manner as in Preparation Comparative Example 1, except that the antimicrobial and preservative compositions prepared in accordance with Comparative Examples 2, 3, 4, 5, 6, 7, 8, 9, 10 and 11 were used instead of the antimicrobial and preservative composition prepared in accordance with Comparative Example 1.

### Preparation Comparative Examples 12 to 22: Dishwashing detergent compositions

### Preparation Comparative Example 12.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Comparative Example 1 was prepared.

### Preparation Comparative Examples 13 to 22.

Dishwashing detergent compositions according to Preparation Comparative Examples 13, 14, 15, 16, 17, 18, 19, 20, 21, and 22 were sequentially prepared in the same manner as in Preparation Comparative Example 12, except that the antimicrobial and preservative compositions prepared in accordance with Comparative Examples 2, 3, 4, 5, 6, 7, 8, 9, 10 and 11 were used instead of the antimicrobial and preservative composition prepared in accordance with Comparative Example 1.

### Preparation Comparative Examples 23 to 33: Stock wet wipe compositions

### Preparation Comparative Example 23.

A stock wet wipe composition containing 1% by weight of a natural extract, 98.7% by weight of purified water, and 0.3% by weight of the antimicrobial and preservative composition prepared in accordance with Comparative Example 1 was prepared.

### Preparation Comparative Examples 24 to 33.

Stock wet wipe compositions according to Preparation Comparative Examples 24, 25, 26, 27, 28, 29, 30, 31, 32, and 33 were sequentially prepared in the same manner as in Preparation Comparative Example 23, except that the antimicrobial and preservative compositions prepared in accordance with Comparative Examples 2, 3, 4, 5, 6, 7, 8, 9, 10, and 11 were used instead of the antimicrobial and preservative composition prepared in accordance with Comparative Example 1.

### Preparation Comparative Examples 34 to 44: Stock shampoo compositions

### Preparation Comparative Example 34.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Comparative Example 1 were prepared.

### Preparation Comparative Examples 35 to 44.

Stock shampoo compositions according to Preparation Comparative Examples 35, 36, 37, 38, 39, 40, 41, 42, 43, and 44 were sequentially prepared in the same manner as in Preparation Comparative Example 34, except that the antimicrobial and preservative compositions prepared in accordance with Comparative Examples 2, 3, 4, 5, 6, 7, 8, 9, 10, and 11 were used instead of the antimicrobial and preservative composition prepared in accordance with Comparative Example 1.

### Preparation Comparative Examples 45 to 55: Stock cosmetic cream compositions

### Preparation Comparative Example 45.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Comparative Example 1 was prepared.

### Preparation Comparative Examples 46 to 55.

Stock cosmetic cream compositions according to Preparation Comparative Examples 46, 47, 48, 49, 50, 51, 52, 53, 54, and 55 were sequentially prepared in the same manner as in Preparation Comparative Example 45, except that the antimicrobial and preservative compositions prepared in accordance with Comparative Examples 2, 3, 4, 5, 6, 7, 8, 9, 10, and 11 were used instead of the antimicrobial and preservative composition prepared in accordance with Comparative Example 1.

### Preparation Comparative Examples 56 to 66: Cosmetic pact (solid color) compositions

### Preparation Comparative Example 56.

A pact (solid color) composition containing 25% by weight of a spherical powder (silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Comparative Example 1 was prepared.

### Preparation Comparative Examples 57 to 66.

Pact (solid color) compositions according to Preparation Comparative Examples 57, 58, 59, 60, 61, 62, 63, 64, 65, and 66 were sequentially prepared in the same manner as in Preparation Comparative Example 56, except that the antimicrobial and preservative compositions prepared in accordance with Comparative Examples 2, 3, 4, 5, 6, 7, 8, 9, 10, and 11 were used instead of the antimicrobial and preservative composition prepared in accordance with Comparative Example 1.

### Experimental example

Antibacterial tests against bacteria, molds, and yeasts were performed on the compound represented by Formula 1, each of antibacterial and preservation compositions prepared in accordance with Examples and Comparative Examples, and each of cosmetic products, sanitary products, and household chemical compositions prepared in accordance with Preparation Examples and Preparation Comparative Examples in the following method using the disk diffusion method.

### [Experimental Example 1] Evaluation of antibacterial effect through disk diffusion

### (1) Disk diffusion

Three types of bacteria, namely, *Pseudomonas aeruginosa, Staphylococcus aureus,* and *Escherichia coli,* were cultured in tryptic soy broth media and *Candida albicans* was cultured in Sabouraud dextrose broth medium. *Aspergillus brasiliensis* was subcultured on PDA (potato dextrose agar) medium, and then spores were collected therefrom using sterile saline solution to prepare a bacterial solution. 200 µ1 of each prepared bacterial solution was spread evenly on 4 mm thick TSA (tryptic soy agar) medium and PDA (potato dextrose agar) medium using a spreader. The medium on which the bacterial solution was spread was dried for 5 minutes.

50 µ1 of 3,3'-(dodecylimino)bispropane-1,2-diol represented by Formula 1 was inoculated onto the disk and was then gently pressed onto the dried medium using a sterile stick or ring. At this time, a gap of approximately 20 to 30 mm between the disks was present. Bacteria (*E. coli, S. aureus, P. aeruginosa*) were incubated in the solid medium with the disk in an incubator for 24 to 48 hours at 35±2°C, *Candida albicans* (*C. albicans*) was incubated in the solid medium at 25°C for 24 to 48 hours, and black mold *(A. brasiliensis)* was incubated in the solid medium at 24°C for 48 to 72 hours, and then the clear zone where strain growth was inhibited was measured in mm. The disk diffusion method was repeated three times.

### (2) Results of antibacterial test

Results of antibacterial test show that treatment with 3,3'-(dodecylimino)bispropane-1,2-diol inhibits growth of bacteria such as *E. coli, S. aureus,* and *P. aeruginosa,* and fungi such as *C*. *albicans,* and *A. brasiliensis.* Therefore, it can be seen that 3,3'-(dodecylimino)bispropane-1,2-diol has an antibacterial effect not only against bacteria but also against fungi such as molds and yeasts.

**[Table 1]**

| | **Bacteria** | | | **Mold** | **Yeast** |
|---|---|---|---|---|---|
| | *E. coli* | *S. aureus* | *P. aeruginosa* | *A. brasiliensis* | *C. albicans* |
| 1^{st} | 2.5 mm | 2 mm | 1 mm | 1.5 mm | 5 mm |
| 2^{nd} | 2.5 mm | 3 mm | 1.5 mm | 2 mm | 5 mm |
| 3^{rd} | 3 mm | 2.5 mm | 1 mm | 2 mm | 6 mm |
| Mean | 2.6 mm | 2.5 mm | 1.2 mm | 1.8 mm | 5.3 mm |

### [Experimental Example 2] Antimicrobial test of the antimicrobial and preservative compositions

The antimicrobial activity of the test solution was measured using a broth dilution method. The concentration of the test solution that was treated was diluted from the highest concentration of 5% to the lowest concentration of 0.0001%. The test solution was cultured in the presence of the strain and then the turbidity of the tube was determined. At this time, the concentration of the test solution in the tube where no bacterial growth was observed among the tubes treated with the test solution at each concentration was determined as a minimum inhibitory concentration (MIC), compared to the degree of turbidity observed in the tube inoculated with only the strain without treatment with the test solution. After the primary test described as above, the concentration of the test solution that was treated was further subdivided and a secondary test was conducted to obtain an accurate minimum inhibitory concentration (MIC).

### (1) Pre-culture of test bacteria

Test strains *E. coli, S. aureus,* and *P. aeruginosa* were inoculated into tryptic soy broth and incubated at 35±2°C for 16 to 24 hours. *C. albicans* was inoculated into Sabouraud dextrose broth and cultured at 25°C for 24 hours. *A. brasiliensis* was seeded on potato dextrose agar and cultured at (25±1)°C for 7 days, sterile physiological saline was dispensed into a solid medium on which the bacteria grew, and spores were removed using a spreader and then diluted in sterile physiological saline.

### (2) Preparation of test solution

The antimicrobial and preservative compositions of Examples 1 to 10 were dissolved in dipropylene glycol and the resulting solutions were used as test solutions.

Comparative Examples 1 to 11 were used as comparative solutions.

### (3) Mixing of strain with antimicrobial and preservative composition (test solution)

A liquid medium was put into the tube and the concentration of the test solution that was treated was diluted from the highest concentration of 5% to the lowest concentration of 0.0001%. The liquid media used for *E. coli, S. aureus, and P. aeruginosa* (bacteria) were tryptic soy broth, and the liquid media used for *C*. *albicans,* and *A. brasiliensis* (fungi) were Sabouraud dextrose broth.

### (4) Inoculation of test bacterial solution

The strains of *E. coli, S. aureus,* and *P. aeruginosa* was inoculated in the test tube at a concentration of 1X10⁵ CFU/mL. The tube treated with the test solution was incubated at 35±2°C for 16 to 24 hours. The test tube was inoculated with *C*. *albicans* and *A. brasiliensis* at a concentration of 1X10⁴ CFU/mL. After treatment with the test solution was completed, *C*. *albicans* was cultured at 25°C for 24 to 48 hours and *A. brasiliensis* was cultured at 25°C for 46 to 50 hours. A medium tube, to which the bacterial solution was not added, was used as a negative control, and a tube containing the medium inoculated with only the bacterial solution without the test solution was used as a growth control. In addition, in order to determine the antimicrobial activity of dipropylene glycol itself used as a solvent, the MIC of the test bacteria for the solvent was measured under the same conditions as in the sample.

### (5) Results of antimicrobial test of the antimicrobial and preservative compositions

The test results are shown in [Table 2] to [Table 3], and the test results showed that the antimicrobial and preservative compositions of the present invention effectively controlled bacteria, molds, and yeasts.

### [Tables 2 to 31 Results of antimicrobial tests of antimicrobial and preservative compositions

Table 2 shows the results of the antibacterial test against bacteria, molds, and yeasts of the antibacterial and preservative compositions prepared according to Examples in terms of minimum inhibitory concentration (MIC). Table 3 shows the results of the antibacterial test against bacteria, molds, and yeasts of the antibacterial and preservative compositions prepared according to Comparative Examples in terms of minimum inhibitory concentration (MIC).

As can be seen from Tables 2 and 3, all antimicrobial and preservative compositions prepared in accordance with the present invention exhibited excellent antimicrobial effects against bacteria, molds, and yeasts, compared to the single compound of Table 3, and in particular, the antimicrobial and preservative composition containing a combination of the compound of Formula 1 and the compound of Formula 2 at a ratio of 8:2 most effectively controlled bacteria (*E. coli, S. Aureus, P. Aeruginosa),* mold (*A. brasiliensis*) and yeasts (*C*. *albicans).*

**[Table 2]**

| Example (Formula1:Formula 2) | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Example1(2:8) | 0.02 | 0.015 | 0.09 | 0.02 | 0.002 |
| Example1(5:5) | 0.015 | 0.015 | 0.07 | 0.02 | 0.001 |
| Example1(8:2) | 0.01 | 0.01 | 0.05 | 0.015 | 0.001 |
| Example2(2:8) | 0.05 | 0.015 | 0.1 | 0.02 | 0.001 |
| Example2(5:5) | 0.03 | 0.01 | 0.08 | 0.015 | 0.0008 |
| Example2(8:2) | 0.02 | 0.008 | 0.05 | 0.01 | 0.0005 |
| Example3(2:8) | 0.2 | 0.05 | 0.3 | 0.5 | 0.5 |
| Example3(5:5) | 0.1 | 0.03 | 0.2 | 0.2 | 0.2 |
| Example3(8:2) | 0.07 | 0.02 | 0.1 | 0.08 | 0.03 |
| Example4(2:8) | 0.3 | 0.3 | 0.15 | 0.02 | 0.02 |
| Example4(5:5) | 0.2 | 0.1 | 0.12 | 0.015 | 0.015 |
| Example4(8:2) | 0.08 | 0.05 | 0.1 | 0.01 | 0.01 |
| Example5(2:8) | 0.12 | 0.15 | 0.08 | 0.15 | 0.08 |
| Example5(5:5) | 0.1 | 0.1 | 0.05 | 0.1 | 0.05 |
| Example5(8:2) | 0.05 | 0.05 | 0.03 | 0.08 | 0.03 |
| Example6(2:8) | 0.3 | 0.5 | 0.3 | 0.5 | 0.15 |
| Example6(5:5) | 0.1 | 0.3 | 0.2 | 0.2 | 0.08 |
| Example6(8:2) | 0.05 | 0.08 | 0.1 | 0.08 | 0.03 |
| Example7(2:8) | 0.3 | 0.4 | 0.3 | 0.3 | 0.3 |
| Example7(5:5) | 0.1 | 0.2 | 0.25 | 0.15 | 0.1 |
| Example7(8:2) | 0.05 | 0.05 | 0.1 | 0.05 | 0.03 |
| Example8(2:8) | 0.002 | 0.001 | 0.0025 | 0.0025 | 0.002 |
| Example8(5:5) | 0.0015 | 0.0008 | 0.002 | 0.002 | 0.0018 |
| Example8(8:2) | 0.001 | 0.0005 | 0.0018 | 0.0018 | 0.0015 |
| Example9(2:8) | 0.0018 | 0.005 | 0.005 | 0.0025 | 0.0025 |
| Example9(5:5) | 0.0015 | 0.003 | 0.003 | 0.002 | 0.002 |
| Example9(8:2) | 0.0013 | 0.0025 | 0.0025 | 0.0015 | 0.0015 |
| Example10(2:8) | 0.3 | 0.3 | 0.5 | 0.02 | 0.015 |
| Example10(5:5) | 0.2 | 0.2 | 0.3 | 0.015 | 0.01 |
| Example10(8:2) | 0.05 | 0.05 | 0.15 | 0.01 | 0.01 |

**[Table 3]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Comparative Example1 | 0.1 | 0.1 | 0.25 | 0.1 | 0.05 |
| Comparative Example2 | 0.02 | 0.02 | 0.09 | 0.025 | 0.002 |
| Comparative Example3 | 0.05 | 0.02 | 0.1 | 0.025 | 0.001 |
| Comparative Example4 | 0.3 | 0.05 | 0.5 | 1 | 0.9 |
| Comparative Example5 | 0.38 | 0.38 | 0.19 | 0.025 | 0.025 |
| Comparative Example6 | 0.15 | 0.2 | 0.1 | 0.2 | 0.1 |
| Comparative Example7 | 0.5 | 1 | 0.5 | 1 | 0.25 |
| Comparative Example8 | 0.4 | 0.65 | 0.35 | 0.35 | 0.35 |
| Comparative Example9 | 0.002 | 0.001 | 0.003 | 0.003 | 0.002 |
| Comparative Example10 | 0.002 | 0.006 | 0.006 | 0.003 | 0.003 |
| Comparative Example11 | 0.5 | 0.5 | 1 | 0.02 | 0.02 |

### [Experimental Example 3] Antibacterial test on compositions of cosmetic products, sanitary products, and household chemical products

### (1) Antibacterial test method

The test was conducted based on the Korean Industrial Standard Cosmetics-Microbiology-Evaluation of Preservative Power of Cosmetics (KS M ISO 1930:2014) and the details of the test method are as follows.

Bacterial fluids of *Pseudomonas aeruginosa, Staphylococcus aureus, and Escherichia coli,* and two types of fungal fluids, *Candida albicans* and *Aspergillus brasiliensis,* were prepared. The liquid detergent composition for clothes, dishwashing detergent, stock wet wipe, stock shampoo, stock cosmetic cream, and cosmetic pact formulations prepared in Preparation Examples and Preparation Comparative Examples were divided into five sample bottles, and then were inoculated with the bacteria (*E. coli, S. Aureus,* and *P. Aeruginosa*), Candida (C. albicans), and black mold *(A. brasiliensis).* A sample bottle inoculated with only the bacterial solution without the formulation composition was used as a control group without preservative treatment.

Samples were classified by date as follows: Day-0 was the test start date of inoculation, Day-1 was 24 hours after inoculation, and Day-7 was 7 days after inoculation. Then, each sample was inoculated into TSA (tryptic soy agar) medium and PDA (potato dextrose agar), and bacteria (*E. coli, S. Aureus,* and *P. Aeruginosa*) were incubated in an incubator for 24 to 48 hours at 35±2°C, yeast (*C*. *albicans)* was incubated at 25°C for 24 to 48 hours, and mold (*A. brasiliensis*) was incubated at 24°C for 48 to 72 hours to determine the presence or absence of bacteria.

### (2) Results of antimicrobial test of cosmetic, sanitary or household chemical products

[Tables 4 to 5] Results of antimicrobial tests of liquid detergent composition for clothes

Tables 4 to 5 below show the antibacterial effects of the cosmetic, sanitary and household chemical product compositions of Preparation Examples and Preparation Comparative Examples containing the antibacterial and preservation compositions of Examples and Comparative Examples. After the compositions according to Preparation Examples and Preparation Comparative Examples are applied to bacteria, molds, and yeasts, the number of bacteria remaining after 7 days was expressed as CFU/mL. When the number of remaining bacteria was too great to count, it was expressed as TNTC (too-numerous-to-count).

Table 4 below shows the results of antibacterial tests against bacteria, molds, and yeasts of the liquid detergent compositions for clothes of Preparation Examples 1 to 10 and Table 5 shows the results of the antibacterial tests against bacteria, molds, and yeasts of the liquid detergent compositions for clothes of Preparation Comparative Examples 1 to 11.

As can be seen from Table 4, all of the liquid detergent compositions for clothes of Preparation Examples 1 to 10 effectively control all of bacteria (*E. coli, S. Aureus, P. Aeruginosa*), mold (*A. brasiliensis*) and yeasts (*C*. *albicans*) used for antimicrobial tests.

The liquid detergent compositions for clothes according to Preparation Examples 1 to 10 containing both the compound of Formula 1 and the compound of Formula 2 more effectively control all of bacteria (*E. coli, S. Aureus, P. Aeruginosa*), mold (*A. brasiliensis*) and yeasts (*C*. *albicans*), compared to the liquid detergent compositions for clothes according to Preparation Comparative Examples 1 to 11 containing either the compound of Formula 1 or the compound of Formula 2.

**[Table 4]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example1 | 0 | 0 | 0 | 0 | 0 |
| Preparation Example2 | 0 | 0 | 0 | 0 | 0 |
| Preparation Example3 | 0 | 0 | 0 | 2.9X10 | 0 |
| Preparation Example4 | 0 | 0 | 0 | 0 | 0 |
| Preparation Example5 | 0 | 0 | 0 | 0 | 0 |
| Preparation Example6 | 0 | 2.6X10 | 0 | 1.9X10 | 0 |
| Preparation Example7 | 0 | 0 | 0 | 0 | 0 |
| Preparation Example8 | 0 | 0 | 0 | 0 | 0 |
| Preparation Example9 | 0 | 0 | 0 | 0 | 0 |
| Preparation Example10 | 0 | 0 | 2.7X10 | 0 | 0 |

**[Table 5]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example1 | 0 | 0 | 1.2X10 | 3.9X10 | 0 |
| Preparation Comparative Example2 | 0 | 0 | 2.7X10 | 0 | 0 |
| Preparation Comparative Example3 | 0 | 0 | 2.9X10 | 0 | 0 |
| Preparation Comparative Example4 | 2.6X10 | 1.2X10 | 2.6X10 | 13X10 | 2.9X10 |
| Preparation Comparative Example5 | 1.5X10 | 1.1X10 | 0 | 0 | 0 |
| Preparation Comparative Example6 | 0 | 1.1X10 | 0 | 2.3X10 | 0 |
| Preparation Comparative Example7 | 3.7X10 | 14X10 | 2.3X10 | 12X10 | 0 |
| Preparation Comparative Example8 | 4.6X10 | 1.6X10 | 0 | 0 | 0 |
| Preparation Comparative Example9 | 0 | 0 | 0 | 0 | 0 |
| Preparation Comparative Example10 | 0 | 0 | 0 | 0 | 0 |
| Preparation Comparative Example11 | 1.6X10 | 1.2X10 | 9X10 | 0 | 0 |

### [Tables 6 to 7] Results of antimicrobial tests of dishwashing detergent compositions

Table 6 below shows the results of antibacterial tests against bacteria, molds, and yeasts of dishwashing detergent compositions according to Preparation Examples 11 to 20 and Table 7 shows the results of the antibacterial tests against bacteria, molds, and yeasts of the dishwashing detergent compositions according to Preparation Comparative Examples 12 to 22.

As can be seen from Table 6, the dishwashing detergent compositions according to Preparation Examples 11 to 20 effectively control all of bacteria (*E. coli, S. Aureus, P. Aeruginosa*), mold (*A. brasiliensis*) and yeasts (*C*. *albicans*) used for antimicrobial tests.

The dishwashing detergent compositions according to Preparation Examples 11 to 20 containing both the compound of Formula 1 and the compound of Formula 2 more effectively controlled all of bacteria (*E. coli, S. Aureus, P. Aeruginosa*)*,* mold (*A. brasiliensis*) and yeasts (*C*. *albicans*), compared to the dishwashing detergent compositions according to Preparation Comparative Examples 12 to 22 containing either the compound of Formula 1 or the compound of Formula 2.

**[Table 6]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example11 | 0 | 0 | 0 | 0 | 0 |
| Preparation Example12 | 0 | 0 | 0 | 0 | 0 |
| Preparation Example13 | 0 | 0 | 0 | 1.1X10 | 0 |
| Preparation Example14 | 0 | 0 | 0 | 0 | 0 |
| Preparation Example15 | 0 | 0 | 0 | 0 | 0 |
| Preparation Example16 | 0 | 0 | 0 | 0 | 0 |
| Preparation Example17 | 0 | 0 | 0 | 0 | 0 |
| Preparation Example18 | 0 | 0 | 0 | 0 | 0 |
| Preparation Example19 | 0 | 0 | 0 | 0 | 0 |
| Preparation Example20 | 0 | 0 | 3.1X10 | 0 | 0 |

**[Table 7]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example12 | 0 | 0 | 1.2X10 | 1.9X10 | 0 |
| Preparation Comparative Example13 | 0 | 0 | 2X10 | 0 | 0 |
| Preparation Comparative Example14 | 0 | 0 | 1.5X10 | 0 | 0 |
| Preparation Comparative Example15 | 2X10 | 1X10 | 2.5X10 | 11X10 | 2.3X10 |
| Preparation Comparative Example16 | 1X10 | 0 | 0 | 0 | 0 |
| Preparation Comparative Example17 | 0 | 1.1X10 | 0 | 2.3X10 | 0 |
| Preparation Comparative Example18 | 2.5X10 | 11X10 | 2.7X10 | 13X10 | 0 |
| Preparation Comparative Example19 | 4.1X10 | 1.5X10 | 0 | 0 | 0 |
| Preparation Comparative Example20 | 0 | 0 | 0 | 0 | 0 |
| Preparation Comparative Example21 | 0 | 0 | 0 | 0 | 0 |
| Preparation Comparative Example22 | 1.3X10 | 1X10 | 7.1X10 | 0 | 0 |

### [Tables 8 to 91 Results of antimicrobial tests of stock wet wipe compositions

Table 8 below shows the results of antibacterial tests against bacteria, molds, and yeasts of stock wet wipe compositions according to Preparation Examples 21 to 30 and Table 9 shows the results of the antibacterial tests against bacteria, molds, and yeasts of the stock wet wipe compositions according to Preparation Comparative Examples 23 to 33.

As can be seen from Table 8, the stock wet wipe compositions according to Preparation Examples 21 to 30 effectively control all of bacteria (*E. coli, S. Aureus, P. Aeruginosa*), mold (*A. brasiliensis*) and yeasts (C. *albicans*) used for antimicrobial tests.

The stock wet wipe compositions according to Preparation Examples 21 to 30 containing both the compound of Formula 1 and the compound of Formula 2 more effectively control all of bacteria (*E*. *coli, S. Aureus, P. Aeruginosa*), mold (*A. brasiliensis*) and yeasts (*C*. *albicans*), compared to the stock wet wipe compositions according to Preparation Comparative Examples 23 to 33 containing either the compound of Formula 1 or the compound of Formula 2.

**[Table 8]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example21 | 0 | 0 | 0 | 0 | 0 |
| Preparation Example22 | 0 | 0 | 0 | 0 | 0 |
| Preparation Example23 | 0 | 0 | 0 | 0 | 0 |
| Preparation Example24 | 0 | 0 | 0 | 0 | 0 |
| Preparation Example25 | 0 | 0 | 0 | 0 | 0 |
| Preparation Example26 | 0 | 0 | 0 | 1X10 | 0 |
| Preparation Example27 | 0 | 0 | 0 | 0 | 0 |
| Preparation Example28 | 0 | 0 | 0 | 0 | 0 |
| Preparation Example29 | 0 | 0 | 0 | 0 | 0 |
| Preparation Example30 | 0 | 0 | 1.9X10 | 0 | 0 |

**[Table 9]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example23 | 0 | 0 | 1X10 | 2.1X10 | 0 |
| Preparation Comparative Example24 | 0 | 0 | 2.5X10 | 0 | 0 |
| Preparation Comparative Example25 | 0 | 0 | 2X10 | 0 | 0 |
| Preparation Comparative Example26 | 2.1X10 | 1X10 | 2.4X10 | 10X10 | 2.5X10 |
| Preparation Comparative Example27 | 1.4X10 | 1X10 | 0 | 0 | 0 |
| Preparation Comparative Example28 | 0 | 1X10 | 0 | 2.8X10 | 0 |
| Preparation Comparative Example29 | 3X10 | 11X10 | 2.8X10 | 10X10 | 0 |
| Preparation Comparative Example30 | 3.5X10 | 1.8X10 | 0 | 0 | 0 |
| Preparation Comparative Example31 | 0 | 0 | 0 | 0 | 0 |
| Preparation Comparative Example32 | 0 | 0 | 0 | 0 | 0 |
| Preparation Comparative Example33 | 1.8X10 | 1.1X10 | 6X10 | 0 | 0 |

### [Tables 10 to 11] Results of antimicrobial tests of stock shampoo compositions

Table 10 below shows the results of antibacterial tests against bacteria, molds, and yeasts of stock shampoo compositions according to Preparation Examples 31 to 40 and Table 11 shows the results of the antibacterial tests against bacteria, molds, and yeasts of the stock shampoo compositions according to Preparation Comparative Examples 34 to 44.

As can be seen from Table 10, the stock shampoo compositions according to Preparation Examples 31 to 40 effectively controlled all of bacteria *(E. coli, S. Aureus, P. Aeruginosa),* mold *(A. brasiliensis)* and yeasts (C. *albicans)* used for antimicrobial tests.

The stock shampoo compositions according to Preparation Examples 31 to 40 containing both the compound of Formula 1 and the compound of Formula 2 more effectively controlled all of bacteria (*E. coli, S. Aureus, P. Aeruginosa*), mold (*A. brasiliensis*) and yeasts (*C*. *albicans*)*,* compared to the stock shampoo compositions according to Preparation Comparative Examples 34 to 44 containing either the compound of Formula 1 or the compound of Formula 2.

**[Table 10]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example31 | 0 | 0 | 0 | 0 | 0 |
| Preparation Example32 | 0 | 0 | 0 | 0 | 0 |
| Preparation Example33 | 0 | 0 | 0 | 0 | 0 |
| Preparation Example34 | 0 | 0 | 0 | 0 | 0 |
| Preparation Example35 | 0 | 0 | 0 | 0 | 0 |
| Preparation Example36 | 0 | 0 | 0 | 0 | 0 |
| Preparation Example37 | 0 | 0 | 0 | 0 | 0 |
| Preparation Example38 | 0 | 0 | 0 | 0 | 0 |
| Preparation Example39 | 0 | 0 | 0 | 0 | 0 |
| Preparation Example40 | 0 | 0 | 1.5X10 | 0 | 0 |

**[Table 11]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example34 | 0 | 0 | 2.2X10 | 2.5X10 | 0 |
| Preparation Comparative Example35 | 0 | 0 | 2.7X10 | 0 | 0 |
| Preparation Comparative Example36 | 0 | 0 | 2.9X10 | 0 | 0 |
| Preparation Comparative Example37 | 2.6X10 | 1.2X10 | 2.6X10 | TNTC | 2.9X10 |
| Preparation Comparative Example38 | 1.5X10 | 1.1X10 | 0 | 0 | 0 |
| Preparation Comparative Example39 | 0 | 1.1X10 | 0 | 2.3X10 | 0 |
| Preparation Comparative Example40 | 3.7X10 | TNTC | 2.3X10 | TNTC | 0 |
| Preparation Comparative Example41 | 4.6X10 | 1.6X10 | 0 | 0 | 0 |
| Preparation Comparative Example42 | 0 | 0 | 0 | 0 | 0 |
| Preparation Comparative Example43 | 0 | 0 | 0 | 0 | 0 |
| Preparation Comparative Example44 | 1X10 | 0 | 5.9X10 | 0 | 0 |

### [Tables 12 to 13] Results of antimicrobial tests of stock cosmetic cream compositions

Table 12 below shows the results of antibacterial tests against bacteria, molds, and yeasts of stock cosmetic cream compositions according to Preparation Examples 41 to 50 and Table 13 shows the results of the antibacterial tests against bacteria, molds, and yeasts of the stock cosmetic cream compositions according to Preparation Comparative Examples 45 to 55.

As can be seen from Table 12, the stock cosmetic cream compositions according to Preparation Examples 41 to 50 effectively controlled all of bacteria (*E. coli, S. Aureus, P. Aeruginosa*), mold (*A. brasiliensis*) and yeasts (*C*. *albicans*) used for antimicrobial tests.

The stock cosmetic cream compositions according to Preparation Examples 41 to 50 containing both the compound of Formula 1 and the compound of Formula 2 more effectively controlled all of bacteria (*E. coli, S. Aureus, P. Aeruginosa*), mold (*A. brasiliensis*) and yeasts (C. *albicans*), compared to the stock cosmetic cream compositions according to Preparation Comparative Examples 45 to 55 containing either the compound of Formula 1 or the compound of Formula 2.

**[Table 12]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example41 | 0 | 0 | 0 | 0 | 0 |
| Preparation Example42 | 0 | 0 | 0 | 0 | 0 |
| Preparation Example43 | 0 | 0 | 0 | 0 | 0 |
| Preparation Example44 | 0 | 0 | 0 | 0 | 0 |
| Preparation Example45 | 0 | 0 | 0 | 1.1X10 | 0 |
| Preparation Example46 | 0 | 0 | 0 | 1.5X10 | 0 |
| Preparation Example47 | 0 | 0 | 0 | 0 | 0 |
| Preparation Example48 | 0 | 0 | 0 | 0 | 0 |
| Preparation Example49 | 0 | 0 | 0 | 0 | 0 |
| Preparation Example50 | 0 | 0 | 1.2X10 | 0 | 0 |

**[Table 13]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example45 | 0 | 0 | 1X10 | 1.8X10 | 0 |
| Preparation Comparative Example46 | 0 | 0 | 2X10 | 0 | 0 |
| Preparation Comparative Example47 | 0 | 0 | 2.1X10 | 0 | 0 |
| Preparation Comparative Example48 | 2.4X10 | 1X10 | 2X10 | TNTC | 1.8X10 |
| Preparation Comparative Example49 | 1X10 | 1.5X10 | 0 | 0 | 0 |
| Preparation Comparative Example50 | 0 | 1X10 | 0 | 2.7X10 | 0 |
| Preparation Comparative Example51 | 3.5X10 | TNTC | 2.6X10 | TNTC | 0 |
| Preparation Comparative Example52 | 4X10 | 1.4X10 | 0 | 0 | 0 |
| Preparation Comparative Example53 | 0 | 0 | 0 | 0 | 0 |
| Preparation Comparative Example54 | 0 | 0 | 0 | 0 | 0 |
| Preparation Comparative Example55 | 2.1X10 | 0 | 4.8X10 | 0 | 0 |

### [Tables 14 to 15] Results of antimicrobial tests of cosmetic pact compositions

Table 14 below shows the results of antibacterial tests against bacteria, molds, and yeasts of cosmetic pact compositions according to Preparation Examples 51 to 60 and Table 15 shows the results of the antibacterial tests against bacteria, molds, and yeasts of the cosmetic pact compositions according to Preparation Comparative Examples 56 to 66.

As can be seen from Table 14, the cosmetic pact compositions according to Preparation Examples 51 to 60 effectively controlled all of bacteria (*E. coli, S. Aureus, P. Aeruginosa*), mold (*A. brasiliensis*) and yeasts (*C*. *albicans*) used for antimicrobial tests.

The stock wet wipe compositions according to Preparation Examples 51 to 60 containing both the compound of Formula 1 and the compound of Formula 2 more effectively controlled all of bacteria (*E. coli, S. Aureus, P. Aeruginosa*), mold (*A. brasiliensis*) and yeasts (*C*. *albicans*), compared to the stock wet wipe compositions according to Preparation Comparative Examples 56 to 66 containing either the compound of Formula 1 or the compound of Formula 2.

**[Table 14]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example51 | 0 | 0 | 0 | 0 | 0 |
| Preparation Example52 | 0 | 0 | 0 | 0 | 0 |
| Preparation Example53 | 0 | 0 | 0 | 1X10 | 0 |
| Preparation Example54 | 0 | 0 | 0 | 0 | 0 |
| Preparation Example55 | 0 | 0 | 0 | 0 | 0 |
| Preparation Example56 | 0 | 0 | 0 | 1.2X10 | 0 |
| Preparation Example57 | 0 | 0 | 0 | 0 | 0 |
| Preparation Example58 | 0 | 0 | 0 | 0 | 0 |
| Preparation Example59 | 0 | 0 | 0 | 0 | 0 |
| Preparation Example60 | 0 | 0 | 1.4X10 | 0 | 0 |

**[Table 15]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example56 | 0 | 0 | 0 | 1X10 | 0 |
| Preparation Comparative Example57 | 0 | 0 | 0 | 0 | 0 |
| Preparation Comparative Example58 | 0 | 0 | 0 | 0 | 0 |
| Preparation Comparative Example59 | 0 | 0 | 1.1X10 | 4.1X10 | 1.5X10 |
| Preparation Comparative Example60 | 0 | 1X10 | 0 | 0 | 0 |
| Preparation Comparative Example61 | 0 | 0 | 0 | 2X10 | 0 |
| Preparation Comparative Example62 | 1X10 | 4.8X10 | 1.2X10 | 6.5X10 | 0 |
| Preparation Comparative Example63 | 2.6X10 | 0 | 0 | 0 | 0 |
| Preparation Comparative Example64 | 0 | 0 | 0 | 0 | 0 |
| Preparation Comparative Example65 | 0 | 0 | 0 | 0 | 0 |
| Preparation Comparative Example66 | 1.4X10 | 0 | 3X10 | 0 | 0 |

### [Test B: Antibacterial effects of compounds represented by Formula 1 and Formula 31

### Examples 1 to 7: Preparation of antibacterial and preservative compositions using compounds represented by Formula 1 and Formula 3

### Example 1.

The 3,3'-(dodecylimino)bispropane-1,2-diol represented by Formula 1 and the lauroyl arginine methyl ester represented by Formula 3 were mixed at a weight ratio of 2:8, 5:5, or 8:2 to prepare an antibacterial and preservative composition.

### Example 2.

The 3,3'-(dodecylimino)bispropane-1,2-diol represented by Formula 1 and the lauroyl lysine methyl ester represented by Formula 3 were mixed at a weight ratio of 2:8, 5:5, or 8:2 to prepare an antibacterial and preservative composition.

### Example 3.

The 3,3'-(dodecylimino)bispropane-1,2-diol represented by Formula 1 and the lauroyl arginine ethyl ester represented by Formula 3 were mixed at a weight ratio of 2:8, 5:5, or 8:2 to prepare an antibacterial and preservative composition.

### Example 4.

The 3,3'-(dodecylimino)bispropane-1,2-diol represented by Formula 1 and the iodopropynyl butylcarbamate represented by Formula 3 were mixed at a weight ratio of 2:8, 5:5, or 8:2 to prepare an antibacterial and preservative composition.

### Example 5.

The 3,3'-(dodecylimino)bispropane-1,2-diol represented by Formula 1 and the polylysine represented by Formula 3 were mixed at a weight ratio of 2:8, 5:5, or 8:2 to prepare an antibacterial and preservative composition.

### Example 6.

The 3,3'-(dodecylimino)bispropane-1,2-diol represented by Formula 1 and the lauroyl lysine represented by Formula 3 were mixed at a weight ratio of 2:8, 5:5, or 8:2 to prepare an antibacterial and preservative composition.

### Example 7.

The 3,3'-(dodecylimino)bispropane-1,2-diol represented by Formula 1 and the caprylhydroxamic acid represented by Formula 3 were mixed at a weight ratio of 2:8, 5:5, or 8:2 to prepare an antibacterial and preservative composition.

### Comparative Examples 1 to 8: Preparation of antibacterial and preservative compositions using compounds represented by Formula 1 or Formula 3

### Comparative Example 1.

The 3,3'-(dodecylimino)bispropane-1,2-diol represented by Formula 1 was used.

### Comparative Example 2.

The lauroyl arginine methyl ester represented by Formula 3 was used.

### Comparative Example 3.

The lauroyl lysine methyl ester represented by Formula 3 was used.

### Comparative Example 4.

The lauroyl arginine ethyl ester represented by Formula 3 was used.

### Comparative Example 5.

The iodopropynyl butylcarbamate represented by Formula 3 was used.

### Comparative Example 6.

The polylysine represented by Formula 3 was used.

### Comparative Example 7.

The lauroyl lysine represented by Formula 3 was used.

### Comparative Example 8.

The caprylhydroxamic acid represented by Formula 3 was used.

### Preparation Examples and Preparation Comparative Examples

Substances used in the following Preparation Examples and Preparation Comparative Examples were purchased from Sigma-Aldrich Korea, TCI, and the like. Cosmetic products, sanitary products, or household chemical products of Preparation Examples were prepared using the antibacterial and preservative compositions of Examples containing the 3,3'-(dodecylimino)bispropane-1,2-diol represented by Formula 1 and the compound represented by Formula 3 at a weight ratio of 8:2. Cosmetic products, sanitary products, or household chemical products of Preparation Comparative Examples were prepared using the antibacterial and preservative compositions of Comparative Examples containing the compound represented by Formula 1 or the compound represented by Formula 3 alone.

### Preparation Examples 1 to 7: Liquid detergent composition for clothes

### Preparation Example 1.

A liquid detergent composition for clothes containing 6% by weight of laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Example 1 was prepared.

### Preparation Examples 2 to 7.

Liquid detergent compositions for clothes according to Preparation Examples 2, 3, 4, 5, 6, and 7 were sequentially prepared in the same manner as in Preparation Example 1, except that the antimicrobial and preservative compositions prepared in accordance with Examples 2, 3, 4, 5, 6, and 7 were used instead of the antimicrobial and preservative composition prepared in accordance with Example 1.

### Preparation Examples 8 to 14: Dishwashing detergent compositions

### Preparation Example 8.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water, and 0.3% by weight of the antimicrobial and preservative composition prepared in accordance with Example 1 was prepared.

### Preparation Examples 9 to 14.

Dishwashing detergent compositions according to Preparation Examples 9, 10, 11, 12, 13, and 14 were sequentially prepared in the same manner as in Preparation Example 8, except that the antimicrobial and preservative compositions prepared in accordance with Examples 2, 3, 4, 5, 6, and 7 were used instead of the antimicrobial and preservative composition prepared in accordance with Example 1.

### Preparation Examples 15 to 21: Stock wet wipe compositions

### Preparation Example 15.

A stock wet wipe composition containing 1% by weight of a natural extract, 98.7% by weight of purified water, and 0.3% by weight of the antimicrobial and preservative composition prepared in accordance with Example 1 was prepared.

### Preparation Examples 16 to 21.

Stock wet wipe compositions according to Preparation Examples 16, 17, 18, 19, 20, and 21 were sequentially prepared in the same manner as in Preparation Example 15, except that the antimicrobial and preservative compositions prepared in accordance with Examples 2, 3, 4, 5, 6, and 7 were used instead of the antimicrobial and preservative composition prepared in accordance with Example 1.

### Preparation Examples 22 to 28: Stock shampoo compositions

### Preparation Example 22.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Example 1 were prepared.

### Preparation Examples 23 to 28.

Stock shampoo compositions according to Preparation Examples 23, 24, 25, 26, 27, and 28 were sequentially prepared in the same manner as in Preparation Example 22, except that the antimicrobial and preservative compositions prepared in accordance with Examples 2, 3, 4, 5, 6, and 7 were used instead of the antimicrobial and preservative composition prepared in accordance with Example 1.

### Preparation Examples 29 to 35: Stock cosmetic cream compositions

### Preparation Example 29.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Example 1 was prepared.

### Preparation Examples 30 to 35.

Stock cosmetic cream compositions according to Preparation Examples 30, 31, 32, 33, 34, and 35 were sequentially prepared in the same manner as in Preparation Example 29, except that the antimicrobial and preservative compositions prepared in accordance with Examples 2, 3, 4, 5, 6, and 7 were used instead of the antimicrobial and preservative composition prepared in accordance with Example 1.

### Preparation Examples 36 to 42: Cosmetic pact (solid color) compositions

### Preparation Example 36.

A pact (solid color) composition containing 25% by weight of a spherical powder (silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Example 1 was prepared.

### Preparation Examples 37 to 42.

Pact (solid color) compositions according to Preparation Examples 37, 38, 39, 40, 41, and 42 were sequentially prepared in the same manner as in Preparation Example 36, except that the antimicrobial and preservative compositions prepared in accordance with Examples 2, 3, 4, 5, 6, and 7 were used instead of the antimicrobial and preservative composition prepared in accordance with Example 1.

### Preparation Comparative Examples 1 to 8: Liquid detergent compositions for clothes

### Preparation Comparative Example 1.

A liquid detergent composition for clothes containing 6% by weight of laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Comparative Example 1 was prepared.

### Preparation Comparative Examples 2 to 8

Liquid detergent compositions for clothes according to Preparation Comparative Examples 2, 3, 4, 5, 6, 7, and 8 were sequentially prepared in the same manner as in Preparation Comparative Example 1, except that the antimicrobial and preservative compositions prepared in accordance with Comparative Examples 2, 3, 4, 5, 6, 7, and 8 were used instead of the antimicrobial and preservative composition prepared in accordance with Comparative Example 1.

### Preparation Comparative Examples 9 to 16: Dishwashing detergent compositions

### Preparation Comparative Example 9.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Comparative Example 1 was prepared.

### Preparation Comparative Examples 10 to 16.

Dishwashing detergent compositions according to Preparation Comparative Examples 10, 11, 12, 13, 14, 15, and 16 were sequentially prepared in the same manner as in Preparation Comparative Example 9, except that the antimicrobial and preservative compositions prepared in accordance with Comparative Examples 2, 3, 4, 5, 6, 7, and 8 were used instead of the antimicrobial and preservative composition prepared in accordance with Comparative Example 1.

### Preparation Comparative Examples 17 to 24: Stock wet wipe compositions

### Preparation Comparative Example 17.

A stock wet wipe composition containing 1% by weight of a natural extract, 98.7% by weight of purified water, and 0.3% by weight of the antimicrobial and preservative composition prepared in accordance with Comparative Example 1 was prepared.

### Preparation Comparative Examples 18 to 24.

Stock wet wipe compositions according to Preparation Comparative Examples 18, 19, 20, 21, 22, 23, and 24 were sequentially prepared in the same manner as in Preparation Comparative Example 17, except that the antimicrobial and preservative compositions prepared in accordance with Comparative Examples 2, 3, 4, 5, 6, 7, and 8 were used instead of the antimicrobial and preservative composition prepared in accordance with Comparative Example 1.

### Preparation Comparative Examples 25 to 32: Stock shampoo compositions

### Preparation Comparative Example 25.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Comparative Example 1 were prepared.

### Preparation Comparative Examples 26 to 32.

Stock shampoo compositions according to Preparation Comparative Examples 26, 27, 28, 29, 30, 31, and 32 were sequentially prepared in the same manner as in Preparation Comparative Example 25, except that the antimicrobial and preservative compositions prepared in accordance with Comparative Examples 2, 3, 4, 5, 6, 7, and 8 were used instead of the antimicrobial and preservative composition prepared in accordance with Comparative Example 1.

### Preparation Comparative Examples 33 to 40: Stock cosmetic cream compositions

### Preparation Comparative Example 33.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Comparative Example 1 was prepared.

### Preparation Comparative Examples 34 to 40.

Stock cosmetic cream compositions according to Preparation Comparative Examples 34, 35, 36, 37, 38, 39 and 40 were sequentially prepared in the same manner as in Preparation Comparative Example 33, except that the antimicrobial and preservative compositions prepared in accordance with Comparative Examples 2, 3, 4, 5, 6, 7, and 8 were used instead of the antimicrobial and preservative composition prepared in accordance with Comparative Example 1.

### Preparation Comparative Examples 41 to 48: Cosmetic pact (solid color) compositions

### Preparation Comparative Example 41.

A pact (solid color) composition containing 25% by weight of a spherical powder (silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Comparative Example 1 was prepared.

### Preparation Comparative Examples 42 to 48.

Pact (solid color) compositions according to Preparation Comparative Examples 42, 43, 44, 45, 46, 47, and 48 were sequentially prepared in the same manner as in Preparation Comparative Example 41, except that the antimicrobial and preservative compositions prepared in accordance with Comparative Examples 2, 3, 4, 5, 6, 7, and 8 were used instead of the antimicrobial and preservative composition prepared in accordance with Comparative Example 1.

### Experimental example

Antibacterial tests against bacteria, molds, and yeasts were performed on each of antibacterial and preservation compositions prepared using the compound represented by Formula 1 and the compound represented by Formula 3 in accordance with Examples and Comparative Examples, and each of cosmetic products, sanitary products, and household chemical compositions prepared in accordance with Preparation Examples and Preparation Comparative Examples in the following method using the disk diffusion method.

### [Experimental Example 1] Antibacterial tests of antibacterial and preservation compositions

The antibacterial test was conducted in the same manner as the antibacterial test for the antibacterial and preservation composition described above.

The test results are shown in [Table 16] to [Table 17], and the test results showed that the antimicrobial and preservative compositions of the present invention, and cosmetic, sanitary and household chemical product compositions including the antimicrobial and preservative composition of the present invention effectively controlled bacteria, molds, and yeasts.

### [Tables 16-17] Results of antimicrobial tests of antimicrobial and preservative compositions

Table 16 shows the results of the antibacterial test against bacteria, molds, and yeasts of the antibacterial and preservative compositions prepared according to Examples in terms of minimum inhibitory concentration (MIC). Table 17 shows the results of the antibacterial test against bacteria, molds, and yeasts of the antibacterial and preservative compositions prepared according to Comparative Examples in terms of minimum inhibitory concentration (MIC).

As can be seen from Tables 16 and 17, all antimicrobial and preservative compositions prepared in accordance with the present invention exhibited excellent antimicrobial effects against bacteria, molds, and yeasts, compared to the single compound of Table 17, and in particular, the antimicrobial and preservative composition containing a combination of the compound of Formula 1 and the compound of Formula 3 at a ratio of 8:2 most effectively controlled bacteria (*E. coli, S. Aureus, P. Aeruginosa*), mold (*A. brasiliensis*) and yeasts (*C. albicans*).

**[Table 16]**

| Example(Formula1 :Formula3) | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Example1(2:8) | 0.0018 | 0.0055 | 0.005 | 0.0065 | 0.0055 |
| Example1(5:5) | 0.0015 | 0.005 | 0.0045 | 0.005 | 0.005 |
| Example1(8:2) | 0.001 | 0.004 | 0.003 | 0.0045 | 0.004 |
| Example2(2:8) | 0.0035 | 0.0005 | 0.003 | 0.0035 | 0.0015 |
| Example2(5:5) | 0.003 | 0.00045 | 0.0025 | 0.003 | 0.001 |
| Example2(8:2) | 0.002 | 0.0004 | 0.002 | 0.002 | 0.0008 |
| Example3(2:8) | 0.003 | 0.0007 | 0.0025 | 0.0025 | 0.0013 |
| Example3(5:5) | 0.0025 | 0.0005 | 0.002 | 0.002 | 0.0012 |
| Example3(8:2) | 0.002 | 0.0004 | 0.0015 | 0.0015 | 0.001 |
| Example4(2:8) | 0.00025 | 0.00015 | 0.00015 | 0.00008 | 0.00007 |
| Example4(5:5) | 0.0002 | 0.0001 | 0.00013 | 0.00007 | 0.00005 |
| Example4(8:2) | 0.00015 | 0.00008 | 0.0001 | 0.00005 | 0.00002 |
| Example5(2:8) | 0.0045 | 0.0018 | 0.0025 | 0.0028 | 0.022 |
| Example5(5:5) | 0.004 | 0.0015 | 0.0022 | 0.0025 | 0.02 |
| Example5(8:2) | 0.003 | 0.0013 | 0.002 | 0.002 | 0.015 |
| Example6(2:8) | 0.005 | 0.004 | 0.004 | 0.0045 | 0.025 |
| Example6(5:5) | 0.003 | 0.003 | 0.0035 | 0.004 | 0.02 |
| Example6(8:2) | 0.002 | 0.0035 | 0.003 | 0.003 | 0.015 |
| Example7(2:8) | 0.05 | 0.05 | 0.1 | 0.02 | 0.025 |
| Example7(5:5) | 0.04 | 0.04 | 0.08 | 0.015 | 0.02 |
| Example7(8:2) | 0.04 | 0.03 | 0.05 | 0.01 | 0.01 |

**[Table 17]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Comparative Example1 | 0.1 | 0.1 | 0.25 | 0.1 | 0.05 |
| Comparative Example2 | 0.002 | 0.006 | 0.006 | 0.008 | 0.006 |
| Comparative Example3 | 0.004 | 0.0006 | 0.005 | 0.004 | 0.002 |
| Comparative Example4 | 0.003 | 0.0008 | 0.003 | 0.003 | 0.0015 |
| Comparative Example5 | 0.0003 | 0.0002 | 0.0002 | 0.0001 | 0.0001 |
| Comparative Example6 | 0.005 | 0.002 | 0.003 | 0.003 | 0.025 |
| Comparative Example7 | 0.008 | 0.005 | 0.005 | 0.005 | 0.03 |
| Comparative Example8 | 0.06 | 0.06 | 0.12 | 0.02 | 0.03 |

### [Experimental Example 2] Antibacterial tests of cosmetic, sanitary and household chemical product compositions

### (1) Antibacterial test method

The antibacterial test was conducted in the same manner as the antibacterial test for cosmetic, sanitary and household chemical product compositions described above.

### (2) Results of antimicrobial test of cosmetic, sanitary or household chemical products

### [Tables 18-19] Results of antimicrobial tests of liquid detergent composition for clothes

Tables 18 to 29 below show the antibacterial effects of the cosmetics, sanitary products, and household chemical product compositions of Preparation Examples and Preparation Comparative Examples containing the antibacterial and preservation compositions of Examples and Comparative Examples. After the compositions according to Preparation Examples and Preparation Comparative Examples are applied to bacteria, molds, and yeasts, the number of bacteria remaining after 7 days was expressed as CFU/mL. When the number of remaining bacteria was too great to count, it was expressed as TNTC (too-numerous-to-count).

Table 18 below shows the results of antibacterial tests against bacteria, molds, and yeasts of the liquid detergent compositions for clothes of Preparation Examples 1 to 7 and Table 19 shows the results of the antibacterial tests against bacteria, molds, and yeasts of the liquid detergent compositions for clothes of Preparation Comparative Examples 1 to 8.

As can be seen from Table 18, all of the liquid detergent compositions for clothes of Preparation Examples 1 to 7 effectively control all of bacteria (*E. coli, S. Aureus, P. Aeruginosa*), mold (*A. brasiliensis*) and yeasts (*C. albicans*).

The liquid detergent compositions for clothes according to Preparation Examples 1 to 7 containing both the compound of Formula 1 and the compound of Formula 3 more effectively control all of bacteria (*E. coli, S. Aureus, P. Aeruginosa*), mold (*A. brasiliensis*) and yeasts (*C. albicans*), compared to the liquid detergent compositions for clothes according to Preparation Comparative Examples 1 to 8 containing either the compound of Formula 1 or the compound of Formula 3.

**[Table 18]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example1 | 0 | 0 | 0 | 0 | 0 |
| Preparation Example2 | 0 | 0 | 0 | 0 | 0 |
| Preparation Example3 | 0 | 0 | 0 | 0 | 0 |
| Preparation Example4 | 0 | 0 | 0 | 0 | 0 |
| Preparation Example5 | 0 | 0 | 0 | 0 | 0 |
| Preparation Example6 | 0 | 0 | 0 | 0 | 0 |
| Preparation Example7 | 0 | 0 | 0 | 0 | 0 |

**[Table 19]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example1 | 0 | 0 | 1.2X10 | 3.9X10 | 0 |
| Preparation Comparative Example2 | 2X10 | 0 | 1.1X10 | 0 | 0 |
| Preparation Comparative Example3 | 0 | 0 | 0 | 1.2X10 | 1.3X10 |
| Preparation Comparative Example4 | 1X10 | 0 | 1X10 | 0 | 0 |
| Preparation Comparative Example5 | 0 | 0 | 0 | 1.5X10 | 1.2X10 |
| Preparation Comparative Example6 | 0 | 1.9X10 | 0 | 1.1X10 | 2.5X10 |
| Preparation Comparative Example7 | 0 | 0 | 0 | 0 | 3.8X10 |
| Preparation Comparative Example8 | 0 | 0 | 1.8X10 | 1.2X10 | 0 |

### [Tables 20 to 21] Results of antimicrobial tests of dishwashing detergent compositions

Table 20 below shows the results of antibacterial tests against bacteria, molds, and yeasts of dishwashing detergent compositions according to Preparation Examples 8 to 14 and Table 21 shows the results of the antibacterial tests against bacteria, molds, and yeasts of the dishwashing detergent compositions according to Preparation Comparative Examples 9 to 16.

As can be seen from Table 20, the dishwashing detergent compositions according to Preparation Examples 8 to 14 effectively control all of bacteria (*E. coli, S. Aureus, P. Aeruginosa*), mold (*A. brasiliensis*) and yeasts (*C. albicans*) used for antimicrobial tests.

The dishwashing detergent compositions according to Preparation Examples 8 to 14 containing both the compound of Formula 1 and the compound of Formula 3 more effectively controlled all of bacteria (*E. coli, S. Aureus, P. Aeruginosa*), mold (*A. brasiliensis*) and yeasts (*C. albicans*), compared to the dishwashing detergent compositions according to Preparation Comparative Examples 9 to 16 containing either the compound of Formula 1 or the compound of Formula 3.

**[Table 20]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preparation Example8 | 0 | 0 | 0 | 0 | 0 |
| Preparation Example9 | 0 | 0 | 0 | 0 | 0 |
| Preparation Example10 | 0 | 0 | 0 | 0 | 0 |
| Preparation Example11 | 0 | 0 | 0 | 0 | 0 |
| Preparation Example12 | 0 | 0 | 0 | 0 | 0 |
| Preparation Example 13 | 0 | 0 | 0 | 0 | 0 |
| Preparation Example14 | 0 | 0 | 0 | 0 | 0 |

**[Table 21]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example9 | 0 | 0 | 1.2X10 | 1.9X10 | 0 |
| Preparation Comparative Example10 | 2.8X10 | 0 | 1.3X10 | 0 | 0 |
| Preparation Comparative Example11 | 0 | 0 | 2.7X10 | 1.5X10 | 1.2X10 |
| Preparation Comparative Example12 | 3.1X10 | 0 | 1.8X10 | 0 | 0 |
| Preparation Comparative Example13 | 0 | 0 | 0 | 3.6X10 | 2.2X10 |
| Preparation Comparative Example14 | 0 | 2.4X10 | 0 | 1X10 | 2.1X10 |
| Preparation Comparative Example15 | 0 | 0 | 0 | 1.6X10 | 4.7X10 |
| Preparation Comparative Example16 | 0 | 0 | 3.8X10 | 2.1X10 | 0 |

### [Tables 22 to 23] Results of antimicrobial tests of stock wet wipe compositions

Table 22 below shows the results of antibacterial tests against bacteria, molds, and yeasts of stock wet wipe compositions according to Preparation Examples 15 to 21 and Table 23 shows the results of the antibacterial tests against bacteria, molds, and yeasts of the stock wet wipe compositions according to Preparation Comparative Examples 17 to 24.

As can be seen from Table 22, the stock wet wipe compositions according to Preparation Examples 15 to 21 effectively control all of bacteria (*E. coli, S. Aureus, P. Aeruginosa*), mold (*A. brasiliensis*) and yeasts (*C. albicans*) used for antimicrobial tests.

The stock wet wipe compositions according to Preparation Examples 15 to 21 containing both the compound of Formula 1 and the compound of Formula 3 more effectively control all of bacteria (*E. coli, S. Aureus, P. Aeruginosa*), mold (*A. brasiliensis*) and yeasts (*C*. *albicans*), compared to the stock wet wipe compositions according to Preparation Comparative Examples 17 to 24 containing either the compound of Formula 1 or the compound of Formula 3.

**[Table 22]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example15 | 0 | 0 | 0 | 0 | 0 |
| Preparation Example16 | 0 | 0 | 0 | 0 | 0 |
| Preparation Example17 | 0 | 0 | 0 | 0 | 0 |
| Preparation Example18 | 0 | 0 | 0 | 0 | 0 |
| Preparation Example19 | 0 | 0 | 0 | 0 | 0 |
| Preparation Example20 | 0 | 0 | 0 | 0 | 0 |
| Preparation Example21 | 0 | 0 | 0 | 0 | 0 |

**[Table 23]**

| | **Bacteria** | | | **Mold** | **Yeast** |
|---|---|---|---|---|---|
| | *E.coli* | *S.Aureus* | *P.Aeruginosa* | *A. brasiliensis* | *C.albicans* |
| Preservative Untreated Control | TNTC | TNTC | TNTC | TNTC | TNTC |
| Preparation Comparative Example17 | 0 | 0 | 1X10 | 2.1X10 | 0 |
| Preparation Comparative Example18 | 3.2X10 | 1.6X10 | 1.1X10 | 0 | 0 |
| Preparation Comparative Example19 | 0 | 0 | 1.5X10 | 1.1X10 | 2.1X10 |
| Preparation Comparative Example20 | 1.8X10 | 0 | 1.4X10 | 0 | 0 |
| Preparation Comparative Example21 | 0 | 0 | 0 | 3.2X10 | 1.3X10 |
| Preparation Comparative Example22 | 0 | 2.2X10 | 0 | 2X10 | 1X10 |
| Preparation Comparative Example23 | 0 | 0 | 0 | 1.4X10 | 3.2X10 |
| Preparation Comparative Example24 | 1.2X10 | 0 | 2.4X10 | 2.6X10 | 0 |

### [Tables 24 to 25] Results of antimicrobial tests of stock shampoo compositions

Table 24 below shows the results of antibacterial tests against bacteria, molds, and yeasts of stock shampoo compositions according to Preparation Examples 22 to 28 and Table 25 shows the results of the antibacterial tests against bacteria, molds, and yeasts of the stock shampoo compositions according to Preparation Comparative Examples 25 to 32.

As can be seen from Table 24, the stock wet wipe compositions according to Preparation Examples 22 to 28 effectively controlled all of bacteria (*E. coli, S. Aureus, P. Aeruginosa*), mold (*A. brasiliensis*) and yeasts (*C. albicans*) used for antimicrobial tests.

The stock shampoo compositions according to Preparation Examples 22 to 28 containing both the compound of Formula 1 and the compound of Formula 3 more effectively controlled all of bacteria (*E. coli, S. Aureus, P. Aeruginosa*), mold (*A. brasiliensis*) and yeasts (*C. albicans*), compared to the stock shampoo compositions according to Preparation Comparative Examples 25 to 32 containing either the compound of Formula 1 or the compound of Formula 3.

**[Table 24]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example22 | 0 | 0 | 0 | 0 | 0 |
| Preparation Example23 | 0 | 0 | 0 | 0 | 0 |
| Preparation Example24 | 0 | 0 | 0 | 0 | 0 |
| Preparation Example25 | 0 | 0 | 0 | 0 | 0 |
| Preparation Example26 | 0 | 0 | 0 | 0 | 0 |
| Preparation Example27 | 0 | 0 | 0 | 0 | 0 |
| Preparation Example28 | 0 | 0 | 0 | 0 | 0 |

**[Table 25]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example25 | 0 | 0 | 2.2X10 | 2.5X10 | 0 |
| Preparation Comparative Example26 | 1.5X10 | 0 | 1X10 | 0 | 0 |
| Preparation Comparative Example27 | 0 | 0 | 2X10 | 1.1X10 | 1X10 |
| Preparation Comparative Example28 | 2.3X10 | 0 | 1.5X10 | 0 | 0 |
| Preparation Comparative Example29 | 0 | 0 | 0 | 2.5X10 | 1X10 |
| Preparation Comparative Example30 | 0 | 2X10 | 0 | 1.2X10 | 1X10 |
| Preparation Comparative Example31 | 0 | 0 | 0 | 1.3X10 | 2.7X10 |
| Preparation Comparative Example32 | 1.6x10 | 2.8X10 | 3.4X10 | 0 | 0 |

### [Tables 26 to 27] Results of antimicrobial tests of stock cosmetic cream compositions

Table 26 below shows the results of antibacterial tests against bacteria, molds, and yeasts of stock cosmetic cream compositions according to Preparation Examples 29 to 35 and Table 27 shows the results of the antibacterial tests against bacteria, molds, and yeasts of the stock cosmetic cream compositions according to Preparation Comparative Examples 33 to 40.

As can be seen from Table 26, the stock cosmetic cream compositions according to Preparation Examples 29 to 35 effectively controlled all of bacteria (*E. coli, S. Aureus, P. Aeruginosa*), mold (*A. brasiliensis*) and yeasts (*C. albicans*) used for antimicrobial tests.

The stock cosmetic cream compositions according to Preparation Examples 29 to 35 containing both the compound of Formula 1 and the compound of Formula 3 more effectively controlled all of bacteria (*E. coli, S. Aureus, P. Aeruginosa*), mold (*A. brasiliensis*) and yeasts (*C. albicans*), compared to the stock cosmetic cream compositions according to Preparation Comparative Examples 33 to 40 containing either the compound of Formula 1 or the compound of Formula 3.

**[Table 26]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example29 | 0 | 0 | 0 | 0 | 0 |
| Preparation Example30 | 0 | 0 | 0 | 0 | 0 |
| Preparation Example31 | 0 | 0 | 0 | 0 | 0 |
| Preparation Example32 | 0 | 0 | 0 | 0 | 0 |
| Preparation Example33 | 0 | 0 | 0 | 0 | 0 |
| Preparation Example34 | 0 | 0 | 0 | 0 | 0 |
| Preparation Example35 | 0 | 0 | 0 | 0 | 0 |

**[Table 27]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example33 | 0 | 0 | 1X10 | 1.8X10 | 0 |
| Preparation Comparative Example34 | 1X10 | 1.2X10 | 2.8X10 | 0 | 0 |
| Preparation Comparative Example35 | 0 | 0 | 1.6X10 | 2.1X10 | 1.9X10 |
| Preparation Comparative Example36 | 3.7X10 | 0 | 1X10 | 0 | 0 |
| Preparation Comparative Example37 | 0 | 0 | 0 | 2.9X10 | 2.5X10 |
| Preparation Comparative Example38 | 0 | 3.1X10 | 0 | 1.6X10 | 1.4X10 |
| Preparation Comparative Example39 | 0 | 0 | 0 | 1.5X10 | 2.4X10 |
| Preparation Comparative Example40 | 2.7X10 | 0 | 1.9X10 | 3.5X10 | 0 |

### [Tables 28 to 29] Results of antimicrobial tests of cosmetic pact compositions

Table 28 below shows the results of antibacterial tests against bacteria, molds, and yeasts of cosmetic pact compositions according to Preparation Examples 36 to 42 and Table 29 shows the results of the antibacterial tests against bacteria, molds, and yeasts of the cosmetic pact compositions according to Preparation Comparative Examples 41 to 48.

As can be seen from Table 28, the cosmetic pact compositions according to Preparation Examples 36 to 42 effectively controlled all of bacteria (*E. coli, S. Aureus, P. Aeruginosa*), mold (*A. brasiliensis*) and yeasts (*C. albicans*) used for antimicrobial tests.

The cosmetic pact compositions according to Preparation Examples 36 to 42 containing both the compound of Formula 1 and the compound of Formula 3 more effectively controlled all of bacteria (*E. coli, S. Aureus, P. Aeruginosa*), mold (*A. brasiliensis*) and yeasts (*C. albicans*), compared to the cosmetic pact compositions according to Preparation Comparative Examples 41 to 48 containing either the compound of Formula 1 or the compound of Formula 3.

**[Table 28]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example36 | 0 | 0 | 0 | 0 | 0 |
| Preparation Example37 | 0 | 0 | 0 | 0 | 0 |
| Preparation Example38 | 0 | 0 | 0 | 0 | 0 |
| Preparation Example39 | 0 | 0 | 0 | 0 | 0 |
| Preparation Example40 | 0 | 0 | 0 | 0 | 0 |
| Preparation Example41 | 0 | 0 | 0 | 0 | 0 |
| Preparation Example42 | 0 | 0 | 0 | 0 | 0 |

**[Table 29]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example41 | 0 | 0 | 0 | 1X10 | 0 |
| Preparation Comparative Example42 | 0 | 0 | 1X10 | 0 | 0 |
| Preparation Comparative Example43 | 0 | 0 | 1.9X10 | 0 | 0 |
| Preparation Comparative Example44 | 1.2X10 | 0 | 0 | 0 | 0 |
| Preparation Comparative Example45 | 0 | 0 | 0 | 0 | 1.9X10 |
| Preparation Comparative Example46 | 0 | 1.5X10 | 0 | 1X10 | 0 |
| Preparation Comparative Example47 | 0 | 0 | 0 | 0 | 1X10 |
| Preparation Comparative Example48 | 0 | 0 | 1.2X10 | 1X10 | 0 |

### [Test C: Antibacterial effects of compounds represented by Formula 1 and Formula 4]

### Examples 1 to 12: Preparation of antibacterial and preservative compositions using compounds represented by Formula 1 and Formula 4

### Example 1.

The 3,3'-(dodecylimino)bispropane-1,2-diol represented by Formula 1 and the hexane-1,2-diol represented by Formula 4 were mixed at a weight ratio of 2:8, 5:5, or 8:2 to prepare an antibacterial and preservative composition.

### Example 2.

The 3,3'-(dodecylimino)bispropane-1,2-diol represented by Formula 1 and the octane-1,2-diol represented by Formula 4 were mixed at a weight ratio of 2:8, 5:5, or 8:2 to prepare an antibacterial and preservative composition.

### Example 3.

The 3,3'-(dodecylimino)bispropane-1,2-diol represented by Formula 1 and the decane-1,2-diol represented by Formula 4 were mixed at a weight ratio of 2:8, 5:5, or 8:2 to prepare an antibacterial and preservative composition.

### Example 4.

The 3,3'-(dodecylimino)bispropane-1,2-diol represented by Formula 1 and the ethylhexylglycerin represented by Formula 4 were mixed at a weight ratio of 2:8, 5:5, or 8:2 to prepare an antibacterial and preservative composition.

### Example 5.

The 3,3'-(dodecylimino)bispropane-1,2-diol represented by Formula 1 and the glyceryl monocaprylate represented by Formula 4 were mixed at a weight ratio of 2:8, 5:5, or 8:2 to prepare an antibacterial and preservative composition.

### Example 6.

The 3,3'-(dodecylimino)bispropane-1,2-diol represented by Formula 1 and the octyl glycerin represented by Formula 4 were mixed at a weight ratio of 2:8, 5:5, or 8:2 to prepare an antibacterial and preservative composition.

### Example 7.

The 3,3'-(dodecylimino)bispropane-1,2-diol represented by Formula 1 and the hexyl glycerin represented by Formula 4 were mixed at a weight ratio of 2:8, 5:5, or 8:2 to prepare an antibacterial and preservative composition.

### Example 8.

The 3,3'-(dodecylimino)bispropane-1,2-diol represented by Formula 1 and the levulinic acid represented by Formula 4 were mixed at a weight ratio of 2:8, 5:5, or 8:2 to prepare an antibacterial and preservative composition.

### Example 9.

The 3,3'-(dodecylimino)bispropane-1,2-diol represented by Formula 1 and the citric acid represented by Formula 4 were mixed at a weight ratio of 2:8, 5:5, or 8:2 to prepare an antibacterial and preservative composition.

### Example 10.

The 3,3'-(dodecylimino)bispropane-1,2-diol represented by Formula 1 and the sodium anisate represented by Formula 4 were mixed at a weight ratio of 2:8, 5:5, or 8:2 to prepare an antibacterial and preservative composition.

### Example 11.

The 3,3'-(dodecylimino)bispropane-1,2-diol represented by Formula 1 and the disodium EDTA represented by Formula 4 were mixed at a weight ratio of 2:8, 5:5, or 8:2 to prepare an antibacterial and preservative composition.

### Example 12.

The 3,3'-(dodecylimino)bispropane-1,2-diol represented by Formula 1 and the tetrasodium EDTA represented by Formula 4 were mixed at a weight ratio of 2:8, 5:5, or 8:2 to prepare an antibacterial and preservative composition.

### Comparative Examples 1 to 13: Preparation of antibacterial and preservative compositions using compounds represented by Formula 1 or Formula 4

### Comparative Example 1.

The 3,3'-(dodecylimino)bispropane-1,2-diol represented by Formula 1 was used.

### Comparative Example 2.

The hexane-1,2-diol represented by Formula 4 was used.

### Comparative Example 3.

The octane-1,2-diol represented by Formula 4 was used.

### Comparative Example 4.

The decane-1,2-diol represented by Formula 4 was used.

### Comparative Example 5.

The ethylhexylglycerin represented by Formula 4 was used.

### Comparative Example 6.

The glyceryl monocaprylate represented by Formula 4 was used.

### Comparative Example 7.

The octyl glycerin represented by Formula 4 was used.

### Comparative Example 8.

The hexyl glycerin represented by Formula 4 was used.

### Comparative Example 9.

The levulinic acid represented by Formula 4 was used.

### Comparative Example 10.

The citric acid represented by Formula 4 was used.

### Comparative Example 11.

The sodium anisate represented by Formula 4 was used.

### Comparative Example 12.

The disodium EDTA represented by Formula 4 was used.

### Comparative Example 13.

The tetrasodium EDTA represented by Formula 4 was used.

### Preparation Examples and Preparation Comparative Examples

Substances used in the following Preparation Examples and Preparation Comparative Examples were purchased from Sigma-Aldrich Korea, TCI, and the like. Cosmetic products, sanitary products, or household chemical products of Preparation Examples were prepared using the antibacterial and preservative compositions of Examples containing the 3,3'-(dodecylimino)bispropane-1,2-diol represented by Formula 1 and the compound represented by Formula 4 at a weight ratio of 8:2. Cosmetic products, sanitary products, or household chemical products of Preparation Comparative Examples were prepared using the antibacterial and preservative compositions of Comparative Examples containing the compound represented by Formula 1 or the compound represented by Formula 4 alone.

### Preparation Examples 1 to 12: Liquid detergent composition for clothes

### Preparation Example 1.

A liquid detergent composition for clothes containing 6% by weight of laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Example 1 was prepared.

### Preparation Examples 2 to 12.

Liquid detergent compositions for clothes according to Preparation Examples 2, 3, 4, 5, 6, 7, 8, 9, 10, 11 and 12 were sequentially prepared in the same manner as in Preparation Example 1, except that the antimicrobial and preservative compositions prepared in accordance with Examples 2, 3, 4, 5, 6, 7, 8, 9, 10, 11 and 12 were used instead of the antimicrobial and preservative composition prepared in accordance with Example 1.

### Preparation Examples 13 to 24: Dishwashing detergent compositions

### Preparation Example 13.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Example 1 was prepared.

### Preparation Examples 14 to 24.

Dishwashing detergent compositions according to Preparation Examples 14, 15, 16, 17, 18, 19, 20, 21, 22, 23, and 24 were sequentially prepared in the same manner as in Preparation Example 13, except that the antimicrobial and preservative compositions prepared in accordance with Examples 2, 3, 4, 5, 6, 7, 8, 9, 10, 11 and 12 were used instead of the antimicrobial and preservative composition prepared in accordance with Example 1.

### Preparation Examples 25 to 36: Stock wet wipe compositions

### Preparation Example 25.

A stock wet wipe composition containing 1% by weight of a natural extract, 98.7% by weight of purified water, and 0.3% by weight of the antimicrobial and preservative composition prepared in accordance with Example 1 was prepared.

### Preparation Examples 26 to 36.

Stock wet wipe compositions according to Preparation Examples 26, 27, 28, 29, 30, 31, 32, 33, 34, 35, and 36 were sequentially prepared in the same manner as in Preparation Example 25, except that the antimicrobial and preservative compositions prepared in accordance with Examples 2, 3, 4, 5, 6, 7, 8, 9, 10, 11 and 12 were used instead of the antimicrobial and preservative composition prepared in accordance with Example 1.

### Preparation Examples 37 to 48: Stock shampoo compositions

### Preparation Example 37.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Example 1 were prepared.

### Preparation Examples 38 to 48.

Stock shampoo compositions according to Preparation Examples 38, 39, 40, 41, 42, 43, 44, 45, 46, 47, and 48 were sequentially prepared in the same manner as in Preparation Example 37, except that the antimicrobial and preservative compositions prepared in accordance with Examples 2, 3, 4, 5, 6, 7, 8, 9, 10, 11 and 12 were used instead of the antimicrobial and preservative composition prepared in accordance with Example 1.

### Preparation Examples 49 to 60: Stock cosmetic cream compositions

### Preparation Example 49.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Example 1 was prepared.

### Preparation Examples 50 to 60.

Stock cosmetic cream compositions according to Preparation Examples 50, 51, 52, 53, 54, 55, 56, 57, 58, 59, and 60 were sequentially prepared in the same manner as in Preparation Example 49, except that the antimicrobial and preservative compositions prepared in accordance with Examples 2, 3, 4, 5, 6, 7, 8, 9, 10, 11 and 12 were used instead of the antimicrobial and preservative composition prepared in accordance with Example 1.

### Preparation Examples 61 to 72: Cosmetic pact (solid color) compositions

### Preparation Example 61.

A pact (solid color) composition containing 25% by weight of a spherical powder (silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Example 1 was prepared.

### Preparation Examples 62 to 72.

Pact (solid color) compositions according to Preparation Examples 62, 63, 64, 65, 66, 67, 68, 69, 70, 71, and 72 were sequentially prepared in the same manner as in Preparation Example 61, except that the antimicrobial and preservative compositions prepared in accordance with Examples 2, 3, 4, 5, 6, 7, 8, 9, 10, 11 and 12 were used instead of the antimicrobial and preservative composition prepared in accordance with Example 1.

### Preparation Comparative Examples 1 to 13: Liquid detergent compositions for clothes

### Preparation Comparative Example 1.

A liquid detergent composition for clothes containing 6% by weight of laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Comparative Example 1 was prepared.

### Preparation Comparative Examples 2 to 13

Liquid detergent compositions for clothes according to Preparation Comparative Examples 2, 3, 4, 5, 6, 7, 8, 9, 10, 11, 12 and 13 were sequentially prepared in the same manner as in Preparation Comparative Example 1, except that the antimicrobial and preservative compositions prepared in accordance with Comparative Examples 2, 3, 4, 5, 6, 7, 8, 9, 10, 11, 12 and 13 were used instead of the antimicrobial and preservative composition prepared in accordance with Comparative Example 1.

### Preparation Comparative Examples 14 to 26: Dishwashing detergent compositions

### Preparation Comparative Example 14.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Comparative Example 1 was prepared.

### Preparation Comparative Examples 15 to 26.

Dishwashing detergent compositions according to Preparation Comparative Examples 15, 16, 17, 18, 19, 20, 21, 22, 23, 24, 25, and 26 were sequentially prepared in the same manner as in Preparation Comparative Example 14, except that the antimicrobial and preservative compositions prepared in accordance with Comparative Examples 2, 3, 4, 5, 6, 7, 8, 9, 10, 11, 12 and 13 were used instead of the antimicrobial and preservative composition prepared in accordance with Comparative Example 1.

### Preparation Comparative Examples 27 to 39: Stock wet wipe compositions

### Preparation Comparative Example 27.

A stock wet wipe composition containing 1% by weight of a natural extract, 98.7% by weight of purified water, and 0.3% by weight of the antimicrobial and preservative composition prepared in accordance with Comparative Example 1 was prepared.

### Preparation Comparative Examples 28 to 39.

Stock wet wipe compositions according to Preparation Comparative Examples 28, 29, 30, 31, 32, 33, 34, 35, 36, 37, 38, and 39 were sequentially prepared in the same manner as in Preparation Comparative Example 27, except that the antimicrobial and preservative compositions prepared in accordance with Comparative Examples 2, 3, 4, 5, 6, 7, 8, 9, 10, 11, 12 and 13 were used instead of the antimicrobial and preservative composition prepared in accordance with Comparative Example 1.

### Preparation Comparative Examples 40 to 52: Stock shampoo compositions

### Preparation Comparative Example 40.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Comparative Example 1 were prepared.

### Preparation Comparative Examples 41 to 52.

Stock shampoo compositions according to Preparation Comparative Examples 41, 42, 43, 44, 45, 46, 47, 48, 49, 50, 51, and 52 were sequentially prepared in the same manner as in Preparation Comparative Example 40, except that the antimicrobial and preservative compositions prepared in accordance with Comparative Examples 2, 3, 4, 5, 6, 7, 8, 9, 10, 11, 12 and 13 were used instead of the antimicrobial and preservative composition prepared in accordance with Comparative Example 1.

### Preparation Comparative Examples 53 to 65: Stock cosmetic cream compositions

### Preparation Comparative Example 53.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Comparative Example 1 was prepared.

### Preparation Comparative Examples 54 to 65.

Stock cosmetic cream compositions according to Preparation Comparative Examples 54, 55, 56, 57, 58, 59, 60, 61, 62, 63, 64, and 65 were sequentially prepared in the same manner as in Preparation Comparative Example 53, except that the antimicrobial and preservative compositions prepared in accordance with Comparative Examples 2, 3, 4, 5, 6, 7, 8, 9, 10, 11, 12 and 13 were used instead of the antimicrobial and preservative composition prepared in accordance with Comparative Example 1.

### Preparation Comparative Examples 66 to 78: Cosmetic pact (solid color) compositions

### Preparation Comparative Example 66.

A pact (solid color) composition containing 25% by weight of a spherical powder (silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Comparative Example 1 was prepared.

### Preparation Comparative Examples 67 to 78.

Pact (solid color) compositions according to Preparation Comparative Examples 67, 68, 69, 70, 71, 72, 73, 74, 75, 76, 77, and 78 were sequentially prepared in the same manner as in Preparation Comparative Example 66, except that the antimicrobial and preservative compositions prepared in accordance with Comparative Examples 2, 3, 4, 5, 6, 7, 8, 9, 10, 11, 12 and 13 were used instead of the antimicrobial and preservative composition prepared in accordance with Comparative Example 1.

### Experimental Example

Antibacterial tests against bacteria, molds, and yeasts were performed on each of antibacterial and preservation compositions prepared using the compound represented by Formula 1 and the compound represented by Formula 4 in accordance with Examples and Comparative Examples, and each of cosmetic products, sanitary products, and household chemical compositions prepared in accordance with Preparation Examples and Preparation Comparative Examples in the following method using the disk diffusion method.

### [Experimental Example 1] Antibacterial tests of antibacterial and preservation compositions

The antibacterial test was conducted in the same manner as the antibacterial test for the antibacterial and preservation composition described above.

The test results are shown in [Table 30] to [Table 31], and the test results showed that the antimicrobial and preservative compositions of the present invention, and cosmetic, sanitary and household chemical product compositions including the antimicrobial and preservative composition of the present invention effectively controlled bacteria, molds, and yeasts.

### [Tables 30 to 31] Results of antimicrobial tests of antimicrobial and preservative compositions

Table 30 shows the results of the antibacterial test against bacteria, molds, and yeasts of the antibacterial and preservative compositions prepared according to Examples in terms of minimum inhibitory concentration (MIC). Table 31 shows the results of the antibacterial test against bacteria, molds, and yeasts of the antibacterial and preservative compositions prepared according to Comparative Examples in terms of minimum inhibitory concentration (MIC).

As can be seen from Tables 30 and 31, all antimicrobial and preservative compositions prepared in accordance with the present invention exhibited excellent antimicrobial effects against bacteria, molds, and yeasts, compared to the single compound of Table 31, and in particular, the antimicrobial and preservative composition containing a combination of the compound of Formula 1 and the compound of Formula 4 at a ratio of 8:2 most effectively controlled bacteria (*E. coli, S. Aureus, P. Aeruginosa*), mold (*A. brasiliensis*) and yeasts (*C. albicans*).

**[Table 30]**

| Example(Formul a1: Formula 4) | **Bacteria** | | | **Mold** | **Yeast** |
|---|---|---|---|---|---|
| | *E. coli* | *S.Aureus* | *P.Aeruginosa* | *A.brasiliensis* | *C.albicans* |
| Example 1(2:8) | 1 | 1 | 0.5 | 0.5 | 0.8 |
| Example 1(5:5) | 0.3 | 0.4 | 0.25 | 0.2 | 0.1 |
| Example 1(8:2) | 0.07 | 0.08 | 0.2 | 0.07 | 0.03 |
| Example 2(2:8) | 0.1 | 0.3 | 0.35 | 0.1 | 0.2 |
| Example 2(5:5) | 0.08 | 0.15 | 0.2 | 0.08 | 0.1 |
| Example 2(8:2) | 0.05 | 0.08 | 0.15 | 0.05 | 0.03 |
| Example 3(2:8) | 0.03 | 0.03 | 0.03 | 0.02 | 0.02 |
| Example 3(5:5) | 0.025 | 0.02 | 0.025 | 0.015 | 0.02 |
| Example 3(8:2) | 0.02 | 0.015 | 0.02 | 0.01 | 0.01 |
| Example 4(2:8) | 0.2 | 0.2 | 1.25 | 0.1 | 0.15 |
| Example 4(5:5) | 0.1 | 0.15 | 0.5 | 0.08 | 0.07 |
| Example 4(8:2) | 0.05 | 0.05 | 0.15 | 0.05 | 0.03 |
| Example 5(2:8) | 0.03 | 0.03 | 0.08 | 0.2 | 0.2 |
| Example 5(5:5) | 0.02 | 0.02 | 0.05 | 0.15 | 0.1 |
| Example 5(8:2) | 0.01 | 0.01 | 0.03 | 0.08 | 0.03 |
| Example 6(2:8) | 0.3 | 0.3 | 0.3 | 0.3 | 0.2 |
| Example 6(5:5) | 0.15 | 0.1 | 0.15 | 0.1 | 0.1 |
| Example 6(8:2) | 0.08 | 0.08 | 0.1 | 0.08 | 0.03 |
| Example 7(2:8) | 0.2 | 0.5 | 0.5 | 0.3 | 0.2 |
| Example 7(5:5) | 0.1 | 0.2 | 0.2 | 0.1 | 0.1 |
| Example 7(8:2) | 0.05 | 0.08 | 0.15 | 0.08 | 0.02 |
| Example 8(2:8) | 0.2 | 0.1 | 0.15 | 0.25 | 0.1 |
| Example 8(5:5) | 0.1 | 0.05 | 0.1 | 0.1 | 0.05 |
| Example 8(8:2) | 0.05 | 0.03 | 0.08 | 0.05 | 0.01 |
| Example 9(2:8) | 0.15 | 0.2 | 0.15 | 0.3 | 0.3 |
| Example 9(5:5) | 0.1 | 0.1 | 0.1 | 0.15 | 0.1 |
| Example 9(8:2) | 0.05 | 0.05 | 0.08 | 0.05 | 0.02 |
| Example 10(2:8) | 0.3 | 0.3 | 0.2 | 0.3 | 0.15 |
| Example 10(5:5) | 0.15 | 0.1 | 0.15 | 0.1 | 0.08 |
| Example 10(8:2) | 0.08 | 0.05 | 0.1 | 0.08 | 0.03 |
| Example 11(2:8) | 0.08 | 0.07 | 0.25 | 0.3 | 0.15 |
| Example 11(5:5) | 0.05 | 0.05 | 0.2 | 0.2 | 0.08 |
| Example 11(8:2) | 0.05 | 0.03 | 0.15 | 0.08 | 0.03 |
| Example 12(2:8) | 0.08 | 0.05 | 0.15 | 0.25 | 0.1 |
| Example 12(5:5) | 0.05 | 0.03 | 0.1 | 0.1 | 0.05 |
| Example 12(8:2) | 0.02 | 0.02 | 0.08 | 0.05 | 0.02 |

**[Table 31]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Comparative Example1 | 0.1 | 0.1 | 0.25 | 0.1 | 0.05 |
| Comparative Example2 | 1.25 | 2 | 1.25 | 0.65 | 2 |
| Comparative Example3 | 0.15 | 0.35 | 0.4 | 0.1 | 0.25 |
| Comparative Example4 | 0.04 | 0.03 | 0.04 | 0.02 | 0.03 |
| Comparative Example5 | 0.25 | 0.2 | 2 | 0.1 | 0.2 |
| Comparative Example6 | 0.05 | 0.05 | 0.1 | 0.25 | 0.25 |
| Comparative Example7 | 0.5 | 0.5 | 0.5 | 0.5 | 0.25 |
| Comparative Example8 | 0.25 | 0.75 | 0.75 | 0.5 | 0.4 |
| Comparative Example9 | 0.25 | 0.2 | 0.3 | 0.35 | 0.2 |
| Comparative Example10 | 0.3 | 0.25 | 0.3 | 0.5 | 0.5 |
| Comparative Example11 | 0.4 | 0.7 | 0.5 | 0.7 | 0.25 |
| Comparative Example12 | 0.1 | 0.1 | 0.3 | 0.4 | 0.25 |
| Comparative Example13 | 0.1 | 0.1 | 0.2 | 0.35 | 0.2 |

### [Experimental Example 2] Antibacterial tests of cosmetic, sanitary and household chemical product compositions

### (1) Antibacterial test method

The antibacterial test was conducted in the same manner as the antibacterial test for cosmetic, sanitary and household chemical product compositions described above.

### (2) Results of antimicrobial test of cosmetic, sanitary or household chemical products

### [Tables 32 to 33] Results of antimicrobial tests of liquid detergent composition for clothes

Tables 32 to 43 below show the antibacterial effects of the cosmetics, sanitary products, and household chemical product compositions of Preparation Examples and Preparation Comparative Examples containing the antibacterial and preservation compositions of Examples and Comparative Examples. After the compositions according to Preparation Examples and Preparation Comparative Examples are applied to bacteria, molds, and yeasts, the number of bacteria remaining after 7 days was expressed as CFU/mL. When the number of remaining bacteria was too great to count, it was expressed as TNTC (too-numerous-to-count).

Table 32 below shows the results of antibacterial tests against bacteria, molds, and yeasts of the liquid detergent compositions for clothes of Preparation Examples 1 to 12 and Table 33 shows the results of the antibacterial tests against bacteria, molds, and yeasts of the liquid detergent compositions for clothes of Preparation Comparative Examples 1 to 13.

As can be seen from Table 32, all of the liquid detergent compositions for clothes of Preparation Examples 1 to 12 effectively control all of bacteria (*E. coli, S. Aureus, P. Aeruginosa*), mold (*A. brasiliensis*) and yeasts (*C*. *albicans*).

The liquid detergent compositions for clothes according to Preparation Examples 1 to 12 containing both the compound of Formula 1 and the compound of Formula 4 more effectively control all of bacteria (*E. coli, S. Aureus, P. Aeruginosa*), mold (*A. brasiliensis*) and yeasts (*C. albicans*), compared to the liquid detergent compositions for clothes according to Preparation Comparative Examples 1 to 13 containing either the compound of Formula 1 or the compound of Formula 4.

**[Table 32]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example1 | 0 | 0 | 1.9X10 | 0 | 0 |
| Preparation Example2 | 0 | 0 | 0 | 0 | 0 |
| Preparation Example3 | 0 | 0 | 0 | 0 | 0 |
| Preparation Example4 | 0 | 0 | 1.3X10 | 1X10 | 0 |
| Preparation Example5 | 0 | 0 | 0 | 1.6X10 | 0 |
| Preparation Example6 | 1.2X10 | 0 | 0 | 1X10 | 0 |
| Preparation Example7 | 0 | 0 | 1.6X10 | 0 | 0 |
| Preparation Example8 | 0 | 0 | 0 | 0 | 0 |
| Preparation Example9 | 0 | 0 | 0 | 0 | 0 |
| Preparation Example10 | 0 | 0 | 0 | 1X10 | 0 |
| Preparation Example11 | 0 | 0 | 0 | 0 | 0 |
| Preparation Example12 | 0 | 0 | 0 | 0 | 0 |

**[Table 33]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example1 | 0 | 0 | 1.2X10 | 3.9X10 | 0 |
| Preparation Comparative Example2 | 6.2X10 | TNTC | TNTC | 5.4X10 | TNTC |
| Preparation Comparative Example3 | 2.6X10 | 0 | 1.9X10 | 2.3X10 | 0 |
| Preparation Comparative Example4 | 0 | 0 | 0 | 1X10 | 0 |
| Preparation Comparative Example5 | 2.1X10 | 0 | 17X10 | 4.8X10 | 1.6X10 |
| Preparation Comparative Example6 | 1.8X10 | 0 | 2.8X10 | 3.4X10 | 2.6X10 |
| Preparation Comparative Example7 | 5.6X10 | 1.9X10 | 3.4X10 | 4.9X10 | 1.1X10 |
| Preparation Comparative Example8 | 4.2X10 | 2.8X10 | 4.1X10 | 2.8X10 | 4.1X10 |
| Preparation Comparative Example9 | 1.3X10 | 0 | 1.1X10 | 1.2X10 | 0 |
| Preparation Comparative Example10 | 1.6X10 | 0 | 1.3X10 | 1.2X10 | 0 |
| Preparation Comparative Example11 | 0 | 2.5X10 | 0 | 2.8X10 | 0 |
| Preparation Comparative Example12 | 0 | 0 | 0 | 2.2X10 | 0 |
| Preparation Comparative Example13 | 0 | 0 | 0 | 1.9X10 | 0 |

### [Tables 34 to 35] Results of antimicrobial tests of dishwashing detergent compositions

Table 34 below shows the results of antibacterial tests against bacteria, molds, and yeasts of dishwashing detergent compositions according to Preparation Examples 13 to 24 and Table 35 shows the results of the antibacterial tests against bacteria, molds, and yeasts of the dishwashing detergent compositions according to Preparation Comparative Examples 14 to 26.

As can be seen from Table 34, the dishwashing detergent compositions according to Preparation Examples 13 to 24 effectively control all of bacteria (*E. coli, S. Aureus, P. Aeruginosa*), mold (*A. brasiliensis*) and yeasts (*C*. *albicans*) used for antimicrobial tests.

The dishwashing detergent compositions according to Preparation Examples 13 to 24 containing both the compound of Formula 1 and the compound of Formula 4 more effectively controlled all of bacteria (*E. coli, S. Aureus, P. Aeruginosa*), mold (*A. brasiliensis*) and yeasts (*C. albicans*), compared to the dishwashing detergent compositions according to Preparation Comparative Examples 14 to 26 containing either the compound of Formula 1 or the compound of Formula 4.

**[Table 34]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example13 | 0 | 1.2X10 | 4.1X10 | 1X10 | 0 |
| Preparation Example14 | 0 | 0 | 0 | 0 | 0 |
| Preparation Example15 | 0 | 0 | 0 | 0 | 0 |
| Preparation Example16 | 0 | 0 | 2.6X10 | 0 | 0 |
| Preparation Example17 | 0 | 0 | 0 | 1X10 | 0 |
| Preparation Example18 | 2.3X10 | 0 | 1.1X10 | 0 | 0 |
| Preparation Example19 | 0 | 0 | 1.5X10 | 0 | 1.2X10 |
| Preparation Example20 | 0 | 0 | 0 | 0 | 0 |
| Preparation Example21 | 0 | 0 | 0 | 0 | 0 |
| Preparation Example22 | 0 | 0 | 0 | 0 | 0 |
| Preparation Example23 | 0 | 0 | 0 | 0 | 0 |
| Preparation Example24 | 0 | 0 | 0 | 0 | 0 |

**[Table 35]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example14 | 0 | 0 | 1.2X10 | 1.9X10 | 0 |
| Preparation Comparative Example15 | 4.1X10 | TNTC | TNTC | 4.9X10 | TNTC |
| Preparation Comparative Example16 | 2.4X10 | 0 | 1.7X10 | 2.5X10 | 0 |
| Preparation Comparative Example17 | 0 | 0 | 0 | 0 | 0 |
| Preparation Comparative Example18 | 2X10 | 0 | 15X10 | 4.1X10 | 1.1X10 |
| Preparation Comparative Example19 | 1.5X10 | 0 | 2.6X10 | 3.2X10 | 2.9X10 |
| Preparation Comparative Example20 | 5X10 | 1.2X10 | 3X10 | 4X10 | 1.3X10 |
| Preparation Comparative Example21 | 3X10 | 2X10 | 3.5X10 | 2.6X10 | 3.2X10 |
| Preparation Comparative Example22 | 1.2X10 | 0 | 1X10 | 1X10 | 0 |
| Preparation Comparative Example23 | 1.4X10 | 0 | 1.7X10 | 1.2X10 | 0 |
| Preparation Comparative Example24 | 0 | 2.4X10 | 0 | 2.6X10 | 0 |
| Preparation Comparative Example25 | 0 | 0 | 0 | 2X10 | 0 |
| Preparation Comparative Example26 | 0 | 0 | 0 | 1.5X10 | 0 |

### [Tables 36 to 37] Results of antimicrobial tests of stock wet wipe compositions

Table 36 below shows the results of antibacterial tests against bacteria, molds, and yeasts of stock wet wipe compositions according to Preparation Examples 25 to 36 and Table 37 shows the results of the antibacterial tests against bacteria, molds, and yeasts of the stock wet wipe compositions according to Preparation Comparative Examples 27 to 39.

As can be seen from Table 36, the stock wet wipe compositions according to Preparation Examples 25 to 36 effectively control all of bacteria (*E. coli, S. Aureus, P. Aeruginosa*), mold (*A. brasiliensis*) and yeasts (*C*. *albicans*) used for antimicrobial tests.

The stock wet wipe compositions according to Preparation Examples 25 to 36 containing both the compound of Formula 1 and the compound of Formula 4 more effectively control all of bacteria (*E. coli, S. Aureus, P. Aeruginosa*)*,* mold (*A. brasiliensis*) and yeasts (*C. albicans*), compared to the stock wet wipe compositions according to Preparation Comparative Examples 27 to 39 containing either the compound of Formula 1 or the compound of Formula 4.

**[Table 36]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example25 | 0 | 0 | 1X10 | 0 | 0 |
| Preparation Example26 | 0 | 0 | 0 | 0 | 0 |
| Preparation Example27 | 0 | 0 | 0 | 0 | 0 |
| Preparation Example28 | 0 | 0 | 1X10 | 0 | 0 |
| Preparation Example29 | 0 | 0 | 0 | 1.6X10 | 0 |
| Preparation Example30 | 1X10 | 0 | 0 | 0 | 0 |
| Preparation Example31 | 0 | 0 | 0 | 1.3X10 | 0 |
| Preparation Example32 | 0 | 0 | 0 | 0 | 0 |
| Preparation Example33 | 0 | 0 | 0 | 0 | 0 |
| Preparation Example34 | 0 | 0 | 0 | 1X10 | 0 |
| Preparation Example35 | 0 | 0 | 0 | 0 | 0 |
| Preparation Example36 | 0 | 0 | 0 | 0 | 0 |

**[Table 37]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example27 | 0 | 0 | 1X10 | 2.1X10 | 0 |
| Preparation Comparative Example28 | 6.9X10 | TNTC | TNTC | 4.9X10 | TNTC |
| Preparation Comparative Example29 | 2.9X10 | 0 | 2X10 | 3.1X10 | 0 |
| Preparation Comparative Example30 | 0 | 0 | 0 | 0 | 0 |
| Preparation Comparative Example31 | 2.5X10 | 0 | 12X10 | 4.5X10 | 1X10 |
| Preparation Comparative Example32 | 2X10 | 0 | 2.4X10 | 2.9X10 | 2.3X10 |
| Preparation Comparative Example33 | 4.8X10 | 2X10 | 3.2X10 | 4.5X10 | 1.8X10 |
| Preparation Comparative Example34 | 3.6X10 | 2X10 | 4X10 | 3.5X10 | 1.1X10 |
| Preparation Comparative Example35 | 2X10 | 0 | 1.6X10 | 1.8X10 | 0 |
| Preparation Comparative Example36 | 1.5X10 | 0 | 1X10 | 1.5X10 | 0 |
| Preparation Comparative Example37 | 0 | 2X10 | 0 | 3.1X10 | 0 |
| Preparation Comparative Example38 | 0 | 0 | 0 | 1.5X10 | 0 |
| Preparation Comparative Example39 | 0 | 0 | 0 | 1X10 | 0 |

### [Tables 38 to 39] Results of antimicrobial tests of stock shampoo compositions

Table 38 below shows the results of antibacterial tests against bacteria, molds, and yeasts of stock shampoo compositions according to Preparation Examples 37 to 48 and Table 39 shows the results of the antibacterial tests against bacteria, molds, and yeasts of the stock shampoo compositions according to Preparation Comparative Examples 40 to 52.

As can be seen from Table 38, the stock shampoo compositions according to Preparation Examples 37 to 48 effectively controlled all of bacteria (*E. coli, S. Aureus, P. Aeruginosa*), mold (*A. brasiliensis*) and yeasts (*C. albicans*) used for antimicrobial tests.

The stock shampoo compositions according to Preparation Examples 37 to 48 containing both the compound of Formula 1 and the compound of Formula 4 more effectively controlled all of bacteria (*E. coli, S. Aureus, P. Aeruginosa*), mold (*A. brasiliensis*) and yeasts (*C. albicans*), compared to the stock shampoo compositions according to Preparation Comparative Examples 40 to 52 containing either the compound of Formula 1 or the compound of Formula 4.

**[Table 38]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example37 | 0 | 0 | 1.2X10 | 0 | 0 |
| Preparation Example38 | 0 | 0 | 0 | 0 | 0 |
| Preparation Example39 | 0 | 0 | 0 | 0 | 0 |
| Preparation Example40 | 0 | 0 | 1.4X10 | 1.2X10 | 0 |
| Preparation Example41 | 0 | 0 | 0 | 0 | 0 |
| Preparation Example42 | 0 | 0 | 0 | 1.3X10 | 0 |
| Preparation Example43 | 0 | 0 | 0 | 1X10 | 0 |
| Preparation Example44 | 0 | 0 | 0 | 0 | 0 |
| Preparation Example45 | 0 | 0 | 0 | 0 | 0 |
| Preparation Example46 | 0 | 0 | 0 | 0 | 0 |
| Preparation Example47 | 0 | 0 | 0 | 0 | 0 |
| Preparation Example48 | 0 | 0 | 0 | 0 | 0 |

**[Table 39]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example40 | 0 | 0 | 2.2X10 | 2.5X10 | 0 |
| Preparation Comparative Example41 | 5.8X10 | TNTC | TNTC | 4X10 | TNTC |
| Preparation Comparative Example42 | 2X10 | 0 | 3.1X10 | 3X10 | 0 |
| Preparation Comparative Example43 | 0 | 0 | 0 | 0 | 0 |
| Preparation Comparative Example44 | 3.4X10 | 0 | TNTC | 4.2X10 | 1.9X10 |
| Preparation Comparative Example45 | 2X10 | 0 | 2.4X10 | 4X10 | 2.4X10 |
| Preparation Comparative Example46 | 5.6X10 | 1.9X10 | 3.4X10 | 4.9X10 | 1.1X10 |
| Preparation Comparative Example47 | 4.2X10 | 2.8X10 | 4.1X10 | 2.8X10 | 4.1X10 |
| Preparation Comparative Example48 | 1.3X10 | 0 | 1.1X10 | 1.2X10 | 0 |
| Preparation Comparative Example49 | 1.6X10 | 0 | 1.3X10 | 1.2X10 | 0 |
| Preparation Comparative Example50 | 0 | 2X10 | 0 | 1.9X10 | 0 |
| Preparation Comparative Example51 | 0 | 0 | 0 | 1.5X10 | 0 |
| Preparation Comparative Example52 | 0 | 0 | 0 | 1X10 | 0 |

### [Tables 40 to 41] Results of antimicrobial tests of stock cosmetic cream compositions

Table 40 below shows the results of antibacterial tests against bacteria, molds, and yeasts of stock cosmetic cream compositions according to Preparation Examples 49 to 60 and Table 41 shows the results of the antibacterial tests against bacteria, molds, and yeasts of the stock cosmetic cream compositions according to Preparation Comparative Examples 53 to 65.

As can be seen from Table 40, the stock cosmetic cream compositions according to Preparation Examples 49 to 60 effectively controlled all of bacteria (*E. coli, S. Aureus, P. Aeruginosa*)*,* mold (*A. brasiliensis*) and yeasts (*C*. *albicans*) used for antimicrobial tests.

The stock cosmetic cream compositions according to Preparation Examples 49 to 60 containing both the compound of Formula 1 and the compound of Formula 4 more effectively controlled all of bacteria (*E. coli, S. Aureus, P. Aeruginosa*), mold (*A. brasiliensis*) and yeasts (*C. albicans*), compared to the stock cosmetic cream compositions according to Preparation Comparative Examples 53 to 65 containing either the compound of Formula 1 or the compound of Formula 4.

**[Table 40]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example49 | 0 | 0 | 0 | 1X10 | 0 |
| Preparation Example50 | 0 | 0 | 0 | 0 | 0 |
| Preparation Example51 | 0 | 0 | 0 | 0 | 0 |
| Preparation Example52 | 0 | 0 | 1X10 | 0 | 0 |
| Preparation Example53 | 0 | 0 | 0 | 0 | 0 |
| Preparation Example54 | 0 | 0 | 0 | 1X10 | 0 |
| Preparation Example55 | 0 | 0 | 0 | 1.3X10 | 0 |
| Preparation Example56 | 0 | 0 | 0 | 0 | 0 |
| Preparation Example57 | 0 | 0 | 0 | 0 | 0 |
| Preparation Example58 | 0 | 0 | 0 | 0 | 0 |
| Preparation Example59 | 0 | 0 | 0 | 0 | 0 |
| Preparation Example60 | 0 | 0 | 0 | 0 | 0 |

**[Table 41]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example53 | 0 | 0 | 1X10 | 1.8X10 | 0 |
| Preparation Comparative Example54 | 5X10 | TNTC | TNTC | 3.5X10 | TNTC |
| Preparation Comparative Example55 | 2X10 | 0 | 3X10 | 2.4X10 | 0 |
| Preparation Comparative Example56 | 0 | 0 | 0 | 0 | 0 |
| Preparation Comparative Example57 | 2.4X10 | 0 | TNTC | 3.5X10 | 1.5X10 |
| Preparation Comparative Example58 | 1.6X10 | 0 | 2.2X10 | 2.8X10 | 1.9X10 |
| Preparation Comparative Example59 | 4.6X10 | 1X10 | 2.6X10 | 4.1X10 | 1X10 |
| Preparation Comparative Example60 | 3.2X10 | 2.6X10 | 2.4X10 | 2X10 | 3X10 |
| Preparation Comparative Example61 | 1.2X10 | 0 | 1X10 | 1X10 | 0 |
| Preparation Comparative Example62 | 2X10 | 0 | 1.5X10 | 1X10 | 0 |
| Preparation Comparative Example63 | 0 | 2.6X10 | 0 | 1.5X10 | 0 |
| Preparation Comparative Example64 | 0 | 0 | 0 | 1X10 | 0 |
| Preparation Comparative Example65 | 0 | 0 | 0 | 1X10 | 0 |

### [Tables 42 to 43] Results of antimicrobial tests of cosmetic pact compositions

Table 42 below shows the results of antibacterial tests against bacteria, molds, and yeasts of cosmetic pact compositions according to Preparation Examples 61 to 72 and Table 43 shows the results of the antibacterial tests against bacteria, molds, and yeasts of the cosmetic pact compositions according to Preparation Comparative Examples 66 to 78.

As can be seen from Table 42, the cosmetic pact compositions according to Preparation Examples 61 to 72 effectively controlled all of bacteria (*E. coli, S. Aureus, P. Aeruginosa*), mold (*A. brasiliensis*) and yeasts (*C. albicans*) used for antimicrobial tests.

The cosmetic pact compositions according to Preparation Examples 61 to 72 containing both the compound of Formula 1 and the compound of Formula 4 more effectively controlled all of bacteria (*E. coli, S. Aureus, P. Aeruginosa*), mold (*A. brasiliensis*) and yeasts (*C. albicans*), compared to the cosmetic pact compositions according to Preparation Comparative Examples 66 to 78 containing either the compound of Formula 1 or the compound of Formula 4.

**[Table 42]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preparation Example61 | 0 | 0 | 0 | 0 | 1X10 |
| Preparation Example62 | 0 | 0 | 0 | 0 | 0 |
| Preparation Example63 | 0 | 0 | 0 | 0 | 0 |
| Preparation Example64 | 0 | 0 | 1X10 | 0 | 0 |
| Preparation Example65 | 0 | 0 | 0 | 0 | 0 |
| Preparation Example66 | 0 | 0 | 0 | 0 | 0 |
| Preparation Example67 | 0 | 0 | 0 | 1.1X10 | 0 |
| Preparation Example68 | 0 | 0 | 0 | 0 | 0 |
| Preparation Example69 | 0 | 0 | 0 | 0 | 0 |
| Preparation Example70 | 0 | 0 | 0 | 0 | 0 |
| Preparation Example71 | 0 | 0 | 0 | 0 | 0 |
| Preparation Example72 | 0 | 0 | 0 | 0 | 0 |

**[Table 43]**

| | **Bacteria** | | | **Mold** | **Yeast** |
|---|---|---|---|---|---|
| | *E.coli* | *S.Aureus* | *P.Aeruginosa* | *A.brasiliensis* | *C.albicans* |
| Preservative Untreated Control | TNTC | TNTC | TNTC | TNTC | TNTC |
| Preparation Comparative Example66 | 0 | 0 | 0 | 1X10 | 0 |
| Preparation Comparative Example67 | 0 | 0 | 1X10 | 2.8X10 | 1X10 |
| Preparation Comparative Example68 | 1X10 | 0 | 0 | 2X10 | 0 |
| Preparation Comparative Example69 | 0 | 0 | 0 | 0 | 0 |
| Preparation Comparative Example70 | 2X10 | 0 | 7.6X10 | 2.8X10 | 1.1X10 |
| Preparation Comparative Example71 | 0 | 0 | 1X10 | 0 | 0 |
| Preparation Comparative Example72 | 0 | 0 | 1.6X10 | 1.1X10 | 0 |
| Preparation Comparative Example73 | 1X10 | 0 | 0 | 2X10 | 0 |
| Preparation Comparative Example74 | 0 | 0 | 0 | 1X10 | 0 |
| Preparation Comparative Example75 | 0 | 0 | 0 | 1X10 | 0 |
| Preparation Comparative Example76 | 0 | 1.4X10 | 0 | 1.2X10 | 0 |
| Preparation Comparative Example77 | 0 | 0 | 0 | 0 | 0 |
| Preparation Comparative Example78 | 0 | 0 | 0 | 0 | 0 |

### [Test D: Anti-dandruff effect]

### Experimental Example 1: Preparation of anti-dandruff hair care (antifungal) composition using Formula 1 and piroctone olamine and evaluation of sterilizing power

### 1-1. Preparation of anti-dandruff hair care (antifungal) composition

3,3'-(dodecylimino)bispropane-1,2-diol represented by Formula 1 and piroctone olamine (PO) were mixed at different mixing ratios to prepare an anti-dandruff hair care (antifungal) composition.

### 1-2. Test of sterilizing power

### (1) Pre-culture of test bacteria

The test strains, *Malassezia furfur, Malassezia restricta,* and *Malassezia globosa,* were inoculated into Leeming&Notman Broth and cultured at 30±2°C for 72 to 96 hours.

### (2) Preparation of test solution

The prepared anti-dandruff hair care (antifungal) composition was dissolved in DMSO (dimethyl sulfoxide), which was used as a test solution.

### (3) Mixture of strains and anti-dandruff hair care (antifungal) composition (test solution)

Liquid medium was added to the tube and the test solution was diluted from the highest concentration of 5% to the lowest concentration of 0.0001%. Leeming & Notman Broth was used as a liquid medium for *Malassezia furfur, Malassezia restricta,* and *Malassezia globosa.*

### (4) Inoculation of test bacterial solution

The test bacterial solution was inoculated such that the concentration of strains, *Malassezia furfur, Malassezia restricta,* and *Malassezia globosa,* in the test tube was 1X10⁵ CFU/mL. The tubes that were completely treated with the test solution were incubated at 30±2°C for 72 to 96 hours. A medium tube, to which the bacterial solution was not added, was used as a negative control, and a tube containing the medium inoculated with only the bacterial solution without the test solution was used as a growth control. In addition, in order to determine the sterilizing power of DMSO (dimethyl sulfoxide) used as a solvent, the MIC of the test bacteria for the solvent was measured under the same conditions as in the sample.

The sterilizing power of the test solution was measured using a broth dilution method. The treatment concentration of the test solution was diluted from the highest concentration of 5% to the lowest concentration of 0.0001%. The test solution and the strain were incubated together and then the turbidity of the tube was determined. At this time, compared to the degree of turbidity observed in tubes inoculated with only the strain without treating the medium with the test solution, the concentration of the test solution in the tube in which no bacterial growth was observed among the tubes treated with the test solution at each concentration was determined as the minimum inhibitory concentration (MIC). After the primary test, the secondary test was conducted at further different concentrations of the treated test solution to obtain an accurate minimum inhibitory concentration (MIC).

### (5) Sterilization test results for anti-dandruff hair care (antifungal) composition

The test results are shown in [Table 44] to [Table 46].

Table 44 shows the sterilizing effect against *Malassezia furfur* of an anti-dandruff hair care (antifungal) composition prepared by mixing 3,3'-(dodecylimino)bispropane-1,2-diol represented by Formula 1 with piroctone olamine (PO) at different mixing ratios.

**[Table 44]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | PO | 0 | 10 | 20 | 30 | 40 | 50 | 60 | 70 | 80 | 90 | 100 |
| MIC (*µg*/ml) | | 60 | 50 | 30 | 30 | 20 | 15 | 7 | 7 | 7 | 20 | 30 |

The result shows that the compound of Formula 1 alone exhibits the lowest sterilizing effect and a combination of a reduced amount of the compound of Formula 1 with piroctone olamine (PO) exhibits increased sterilizing effect. A combination of 3,3'-(dodecylimino)bispropane-1,2-diol represented by Formula 1 and piroctone olamine (PO) at a ratio of 8:2 to 1:9 exhibits higher sterilization effect against *Malassezia furfur.*

Table 45 shows the sterilizing effect against *Malassezia restricta* of the anti-dandruff hair care (antifungal) composition prepared by mixing 3,3'-(dodecylimino)bispropane-1,2-diol represented by Formula 1 with piroctone olamine (PO) at different mixing ratios.

**[Table 45]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | PO | 0 | 10 | 20 | 30 | 40 | 50 | 60 | 70 | 80 | 90 | 100 |
| MIC (*µg*/ml) | | 60 | 36 | 22 | 18 | 18 | 12 | 10 | 10 | 18 | 20 | 18 |

The result shows that the compound of Formula 1 alone exhibits the lowest sterilizing effect and a combination of a reduced amount of the compound of Formula 1 with piroctone olamine (PO) exhibits an increased sterilizing effect. A combination of 3,3'-(dodecylimino)bispropane-1,2-diol represented by Formula 1 and piroctone olamine (PO) at a ratio of 9:1 to 1:9 exhibits higher sterilization effect against *Malassezia restricta.*

Table 46 shows the sterilizing effect against *Malassezia globosa* of the anti-dandruff hair care (antifungal) composition prepared by mixing 3,3'-(dodecylimino)bispropane-1,2-diol represented by Formula 1 with piroctone olamine (PO) at different mixing ratios.

**[Table 46]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | PO | 0 | 10 | 20 | 30 | 40 | 50 | 60 | 70 | 80 | 90 | 100 |
| MIC (*µg*/ml) | | 60 | 30 | 22 | 12 | 4 | 4 | 3 | 3 | 3 | 3 | 4 |

The result shows that the compound of Formula 1 alone exhibits the lowest sterilizing effect and a combination of a reduced amount of the compound of Formula 1 with piroctone olamine (PO) exhibits an increased sterilizing effect. A combination of 3,3'-(dodecylimino)bispropane-1,2-diol represented by Formula 1 and piroctone olamine (PO) at a ratio of 9:1 to 1:9 exhibits the highest sterilization effect against *Malassezia globosa.*

As evaluated through test results, the anti-dandruff hair care (antifungal) composition using the compound of Formula 1 and piroctone olamine (PO) of the present invention is capable of effectively controlling the dandruff bacteria, namely, *Malassezia furfur, Malassezia restricta,* and *Malassezia globosa,* although the content of piroctone olamine (PO) is decreased and the content of the compound of Formula 1 is increased, with respect to the total weight of the composition.

### Experimental Example 2: Preparation of anti-dandruff hair care (antifungal) composition using Formula 1 and zinc pyrithione and evaluation of sterilizing power

### 2-1. Preparation of anti-dandruff hair care (antifungal) composition

3,3'-(dodecylimino)bispropane-1,2-diol represented by Formula 1 and zinc pyrithione (ZPT) were mixed at different mixing ratios to prepare an anti-dandruff hair care (antifungal) composition.

### 2-2. Evaluation of sterilizing power

The sterilizing power was evaluated in the same manner as in Experimental Example 1-2.

The test results are shown in [Table 47] to [Table 49].

Table 47 shows the sterilizing effect against *Malassezia furfur* of an anti-dandruff hair care (antifungal) composition prepared by mixing 3,3'-(dodecylimino)bispropane-1,2-diol represented by Formula 1 with zinc pyrithione (ZPT) at different mixing ratios.

**[Table 47]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | ZPT | 0 | 10 | 20 | 30 | 40 | 50 | 60 | 70 | 80 | 90 | 100 |
| MIC (*µg*/ml) | | 60 | 55 | 40 | 30 | 25 | 20 | 6 | 5 | 5 | 7 | 7 |

The result shows that the compound of Formula 1 alone exhibits the lowest sterilizing effect and a combination of a reduced amount of the compound of Formula 1 with zinc pyrithione (ZPT) exhibits increased sterilizing effect. A combination of 3,3'-(dodecylimino)bispropane-1,2-diol represented by Formula 1 and zinc pyrithione (ZPT) at a ratio of 8:2 to 1:9 exhibits the highest sterilization effect against *Malassezia furfur.*

Table 48 shows the sterilizing effect against *Malassezia restricta* of the anti-dandruff hair care (antifungal) composition prepared by mixing 3,3'-(dodecylimino)bispropane-1,2-diol represented by Formula 1 with zinc pyrithione (ZPT) at different mixing ratios.

**[Table 48]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| mixing ratio (%) | ZPT | 0 | 10 | 20 | 30 | 40 | 50 | 60 | 70 | 80 | 90 | 100 |
| MIC (*µg*/ml) | | 60 | 48 | 35 | 15 | 4 | 4 | 3 | 3 | 3 | 4 | 4 |

The result shows that the compound of Formula 1 alone exhibits the lowest sterilizing effect and a combination of a reduced amount of the compound of Formula 1 with zinc pyrithione (ZPT) exhibits increased sterilizing effect. A combination of 3,3'-(dodecylimino)bispropane-1,2-diol represented by Formula 1 and zinc pyrithione (ZPT) at a ratio of 9:1 to 1:9 exhibits higher sterilization effect against *Malassezia restricta.*

Table 49 shows the sterilizing effect against *Malassezia globosa* of the anti-dandruff hair care (antifungal) composition prepared by mixing 3,3'-(dodecylimino)bispropane-1,2-diol represented by Formula 1 with zinc pyrithione (ZPT) at different mixing ratios.

**[Table 49]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | ZPT | 0 | 10 | 20 | 30 | 40 | 50 | 60 | 70 | 80 | 90 | 100 |
| MIC (*µg*/ml) | | 60 | 40 | 20 | 8 | 4 | 1 | 1 | 1 | 1 | 1 | 1 |

The result shows that the compound of Formula 1 alone exhibits the lowest sterilizing effect and a combination of a reduced amount of the compound of Formula 1 with zinc pyrithione (ZPT) exhibits increased sterilizing effect. A combination of 3,3'-(dodecylimino)bispropane-1,2-diol represented by Formula 1 and zinc pyrithione (ZPT) at a ratio of 9:1 to 1:9 exhibits higher sterilization effect against *Malassezia globosa.*

As evaluated through test results, the anti-dandruff hair care (antifungal) composition using the compound of Formula 1 and zinc pyrithione (ZPT) of the present invention although the content of zinc pyrithione (ZPT) is decreased and the content of the compound of Formula 1 is increased, with respect to the total weight of the composition, is capable of effectively controlling the dandruff bacteria, namely, *Malassezia furfur, Malassezia restricta,* and *Malassezia globosa.*

### Experimental Example 3: Preparation of anti-dandruff hair care (antifungal) composition using Formula 1 and climbazole and evaluation of sterilizing power

### 3-1. Preparation of anti-dandruff hair care (antifungal) composition

3,3'-(dodecylimino)bispropane-1,2-diol represented by Formula 1 and climbazole (CB) were mixed at different mixing ratios to prepare an anti-dandruff hair care (antifungal) composition.

### 3-2. Evaluation of sterilizing power

The sterilizing power was evaluated in the same manner as in Experimental Example 1-2.

The test results are shown in [Table 50] to [Table 52].

Table 50 shows the sterilizing effect against *Malassezia furfur* of the anti-dandruff hair care (antifungal) composition prepared by mixing 3,3'-(dodecylimino)bispropane-1,2-diol represented by Formula 1 with climbazole (CB) at different mixing ratios.

**[Table 50]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | CB | 0 | 10 | 20 | 30 | 40 | 50 | 60 | 70 | 80 | 90 | 100 |
| MIC (*µg*/ml) | | 60 | 45 | 40 | 40 | 30 | 15 | 6 | 10 | 15 | 20 | 20 |

The result shows that the compound of Formula 1 alone exhibits the lowest sterilizing effect and a combination of a reduced amount of the compound of Formula 1 with climbazole (CB) exhibits increased sterilizing effect. A combination of 3,3'-(dodecylimino)bispropane-1,2-diol represented by Formula 1 and climbazole (CB) at a ratio of 9:1 to 1:9 exhibits higher sterilization effect against *Malassezia furfur.*

Table 51 shows the sterilizing effect against *Malassezia restricta* of the anti-dandruff hair care (antifungal) composition prepared by mixing 3,3'-(dodecylimino)bispropane-1,2-diol represented by Formula 1 with climbazole (CB) at different mixing ratios.

**[Table 51]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | CB | 0 | 10 | 20 | 30 | 40 | 50 | 60 | 70 | 80 | 90 | 100 |
| MIC (*µg*/ml) | | 60 | 48 | 32 | 40 | 8 | 4 | 4 | 4 | 4 | 4 | 4 |

The result shows that the compound of Formula 1 alone exhibits the lowest sterilizing effect and a combination of a reduced amount of the compound of Formula 1 with climbazole (CB) exhibits increased sterilizing effect. A combination of 3,3'-(dodecylimino)bispropane-1,2-diol represented by Formula 1 and climbazole (CB) at a ratio of 9:1 to 1:9 exhibits higher sterilization effect against *Malassezia restricta.*

Table 52 shows the sterilizing effect against *Malassezia globosa* of the anti-dandruff hair care (antifungal) composition prepared by mixing 3,3'-(dodecylimino)bispropane-1,2-diol represented by Formula 1 with climbazole (CB) at different mixing ratios.

**[Table 52]**

| mixing ratio (%) | Formula1 | 100 | 90 | 80 | 70 | 60 | 50 | 40 | 30 | 20 | 10 | 0 |
|---|---|---|---|---|---|---|---|---|---|---|---|---|
| | CB | 0 | 10 | 20 | 30 | 40 | 50 | 60 | 70 | 80 | 90 | 100 |
| MIC (*µg*/ml) | | 60 | 55 | 30 | 12 | 4 | 1 | 1 | 1 | 1 | 1 | 1 |

The result shows that the compound of Formula 1 alone exhibits the lowest sterilizing effect and a combination of a reduced amount of the compound of Formula 1 with climbazole (CB) exhibits increased sterilizing effect. In particular, a combination of 3,3'-(dodecylimino)bispropane-1,2-diol represented by Formula 1 and climbazole (CB) at a ratio of 8:2 to 1:9 exhibits higher sterilization effect against *Malassezia globosa.*

As evaluated through test results, the anti-dandruff hair care (antifungal) composition using the compound of Formula 1 and climbazole (CB) of the present invention, although the content of climbazole (CB) is decreased and the content of the compound of Formula 1 is increased, with respect to the total weight of the composition, is capable of effectively controlling dandruff bacteria, namely, *Malassezia furfur, Malassezia restricta,* and *Malassezia globosa.*

### Experimental Example 4: Evaluation of sterilizing power of shampoo composition containing anti-dandruff hair care (antifungal) composition

### 4-1: Preparation of shampoo composition

32% by weight of sodium laureth sulfate, 17% by weight of cocamidopropyl betaine, 7% by weight of coco-glucoside, 0.3% by weight of dimethicone, 0.3% by weight of polyquaternium-10, 0.3% by weight of tetrasodium EDTA, 42.6% by weight of purified water, and 0.5% by weight of the anti-dandruff hair care (antifungal) composition prepared according to Experimental Example 1 were added to prepare a shampoo composition.

The anti-dandruff hair care (antifungal) compositions prepared according to Experimental Example 1 were an anti-dandruff hair care (antifungal) composition containing the 3,3'-(dodecylimino)bispropane-1,2-diol represented by Formula 1 alone, an anti-dandruff hair care (antifungal) composition containing a combination of the 3,3'-(dodecylimino)bispropane-1,2-diol represented by Formula 1 and piroctone olamine (PO) at a weight ratio of 5:5, an anti-dandruff hair care (antifungal) composition containing a combination of the 3,3'-(dodecylimino)bispropane-1,2-diol represented by Formula 1 and piroctone olamine (PO) at a weight ratio of 2:8, and an anti-dandruff hair care (antifungal) composition containing the piroctone olamine (PO) alone.

The contents of ingredients used to prepare the shampoo composition are shown in Table 53.

**[Table 53]**

| Ingredient | wt% |
|---|---|
| Sodium laureth sulfate | 32 |
| Cocamidopropyl betaine | 17 |
| Coco-glucoside | 7 |
| Dimethicone | 0.3 |
| Polyquaternium-10 | 0.3 |
| Tetrasodium EDTA | 0.3 |
| Purified water | 42.6 |
| Anti-dandruff hair care (antifungal) composition | 0.5 |
| Total | 100 |

### 4-2. Evaluation of sterilizing power of shampoo compositions

All test solutions, test strain suspensions, reagents, and water were maintained at 20°C±1°C in a constant temperature water bath. A shampoo composition formulation containing an anti-dandruff hair care (antifungal) composition was injected into a sterile test tube and the test strain suspension was added and immediately mixed.

The shampoo compositions were brought into contact with the test strain suspension at 20 ± 1°C for 3 min ± 10 s, 5 min ± 10 s, 10 min ± 10 s, and 15 min ± 10 s, and then diluted in a neutralizing agent. 1 ml of the dilution was injected into two Petri dishes and about 15 ml of sterilized Leeming & Notman agar preparation medium stored at 45 ± 1°C was dispensed aseptically, gently rotated left and right while being careful to prevent the medium from sticking to the Petri dish lid and was thoroughly mixed with the medium by tilting, then cooled and solidified.

In order to suppress formation of spreading colonies, 4 to 5 ml of sterilized Leeming & Notman agar medium was injected again and overlaid. The cooled and solidified Petri dishes were inverted and cultured at 30 ± 2°C for 72 to 96 hours, and the presence or absence of bacteria present in each Petri dish was detected to determine the effect of removing dandruff bacteria.

As such, sterilization test was conducted by bringing the dandruff bacteria, *Malassezia furfur,* into contact with a shampoo composition formulation containing an anti-dandruff hair care (antifungal) composition for 3 minutes, 5 minutes, 10 minutes, and 15 minutes, and whether or not *Malassezia furfur* remained was determined and marked with O and X. The case where *Malassezia furfur* bacteria remained was marked as "O" and the case where all *Malassezia furfur* bacteria were removed was marked as "X".

### (1) Shampoo composition formulation using Formula 1 and piroctone olamine

The test results are shown in [Table 54] to [Table 56].

Table 54 shows the sterilizing effect against *Malassezia furfur* of the anti-dandruff hair care (antifungal) composition prepared by mixing 3,3'-(dodecylimino)bispropane-1,2-diol represented by Formula 1 and piroctone olamine (PO) at different mixing ratios.

**[Table 54]**

| ratio of anti-dandruff hair care (antifungal) composition | Content in shampoo formulation (wt%) | 3 minutes | 5 minutes | 10 minutes | 15 minutes |
|---|---|---|---|---|---|
| Formula 1 | 0.5 wt% | O | O | O | X |
| Formula 1: PO =5:5 | 0.5 wt% | X | X | X | X |
| Formula 1: PO =2:8 | 0.5 wt% | X | X | X | X |
| PO | 0.5 wt% | X | X | X | X |

The result shows that the shampoo composition formulation prepared using the anti-dandruff hair care composition containing 3,3'-(dodecylimino)bispropane-1,2-diol represented by Formula 1 and piroctone olamine (PO) at a ratio of 5:5 or 2:8 and the anti-dandruff hair care (antifungal) composition containing piroctone olamine (PO) alone eliminated *Malassezia furfur* within 3 minutes and thus exhibited better sterilizing power against *Malassezia furfur* compared to an anti-dandruff hair care (antifungal) composition containing only the 3,3'-(dodecylimino)bispropane-1,2-diol represented by Formula 1.

Therefore, rather than the expensive piroctone olamine (PO) compound alone, the combination of the 3,3'-(dodecylimino)bispropane-1,2-diol represented by Formula 1 and piroctone olamine (PO) exhibits excellent sterilizing effect against the dandruff bacteria *Malassezia furfur* and thus the compound of Formula 1 may be used instead of 50% of piroctone olamine to remove dandruff bacteria.

### (2) Shampoo composition formulation using Formula 1 and zinc pyrithione

Table 55 shows the sterilizing effect against *Malassezia furfur* of the shampoo composition using the anti-dandruff hair care (antifungal) composition containing 3,3'-(dodecylimino)bispropane-1,2-diol represented by Formula 1 and zinc pyrithione at different mixing ratios.

**[Table 55]**

| ratio of anti-dandruff hair care (antifungal) composition | Content in shampoo formulation (wt%) | 3 minutes | 5 minutes | 10 minutes | 15 minutes |
|---|---|---|---|---|---|
| Formula 1 | 0.5 wt% | O | O | O | X |
| Formula 1: ZPT =5:5 | 0.5 wt% | X | X | X | X |
| Formula 1: ZPT =2:8 | 0.5 wt% | O | X | X | X |
| ZPT | 0.5 wt% | X | X | X | X |

The result showed that the shampoo composition formulation prepared using the anti-dandruff hair care containing 3,3'-(dodecylimino)bispropane-1,2-diolrepresented by Formula 1 and zinc pyrithione (ZPT) at a ratio of 5:5 and the shampoo composition formulation prepared using the anti-dandruff hair care (antifungal) composition containing zinc pyrithione (ZPT) only eliminated *Malassezia furfur* within 3 minutes and thus exhibited better sterilizing power against *Malassezia furfur* compared to the shampoo composition formulation prepared using the anti-dandruff hair care (antifungal) composition containing only the 3,3'-(dodecylimino)bispropane-1,2-diol represented by Formula 1.

Therefore, rather than the expensive zinc pyrithione (ZPT) alone, the combination of the 3,3'-(dodecylimino)bispropane-1,2-diol represented by Formula 1 and zinc pyrithione (ZPT) exhibits excellent sterilizing effect against the dandruff bacteria *Malassezia furfur* and thus the compound of Formula 1 may be used instead of 50% of zinc pyrithione (ZPT) to remove dandruff bacteria.

### (3) Shampoo composition formulation using Formula 1 and climbazole

Table 56 shows the sterilizing effect against *Malassezia furfur* of the shampoo composition using the anti-dandruff hair care (antifungal) composition prepared by mixing 3,3'-(dodecylimino)bispropane-1,2-diol represented by Formula 1 and climbazole (CB) at different mixing ratios.

**[Table 56]**

| ratio of anti-dandruff hair care (antifungal) composition | Content in shampoo formulation (wt%) | 3 minutes | 5 minutes | 10 minutes | 15 minutes |
|---|---|---|---|---|---|
| Formula 1 | 0.5 wt% | O | O | O | X |
| Formula 1: CB =5:5 | 0.5 wt% | X | X | X | X |
| Formula 1: CB =2:8 | 0.5 wt% | X | X | X | X |
| CB | 0.5 wt% | X | X | X | X |

The result showed that the shampoo composition formulation prepared using the anti-dandruff hair care containing 3,3'-(dodecylimino)bispropane-1,2-diol represented by Formula 1 and climbazole (CB) at a ratio of 5:5 or 2:8, and the shampoo composition formulation prepared using the anti-dandruff hair care (antifungal) composition containing climbazole (CB) alone eliminated *Malassezia furfur* within 3 minutes and thus exhibited better sterilizing power against *Malassezia furfur,* compared to the shampoo composition formulation prepared using the anti-dandruff hair care (antifungal) composition containing the 3,3'-(dodecylimino)bispropane-1,2-diol represented by Formula 1 alone.

Therefore, rather than the expensive climbazole (CB) alone, the combination of the 3,3'-(dodecylimino)bispropane-1,2-diol represented by Formula 1 and climbazole (CB) exhibits excellent sterilizing effect against the dandruff bacteria *Malassezia furfur* and thus the compound of Formula 1 may be used instead of 50% of climbazole (CB) to remove dandruff bacteria.

### Experimental Example 5: Evaluation of sterilizing power of shampoo composition depending on content of anti-dandruff hair care (antifungal) composition

### 5-1. Preparation of shampoo composition

A shampoo composition formulation containing 0.2, 0.3, 0.4, 0.5, 0.6, and 0.7% by weight of an anti-dandruff hair care (antifungal) composition containing the compound of Formula 1 alone, piroctone olamine (PO) alone, zinc pyrithione (ZPT) alone, climbazole (CB) alone, or a combination of the compound of compound 1 and piroctone olamine (PO), zinc pyrithione (ZPT), or climbazole (CB) at 5:5 was prepared in the same manner as in Experimental Example 4-1.

Specifically, 32% by weight of sodium laureth sulfate, 17% by weight of cocamidopropyl betaine, 7% by weight of coco-glucoside, 0.3% by weight of dimethicone, 0.3% by weight of polyquaternium-10, and 0.3% by weight of tetrasodium EDTA, were added, based on 100% by weight of the shampoo composition, like the shampoo composition formulation of Experimental Example 4, but an anti-dandruff hair care (antifungal) composition was added in different amounts of 0.2, 0.3, 0.4, 0.5, 0.6, and 0.7% by weight, and the balance of purified water was added to prepare 100% by weight of a shampoo composition.

Then, the prepared shampoo composition formulation was brought into contact with the dandruff bacteria, *Malassezia furfur, Malassezia restricta,* and *Malassezia globosa* for a constant time of 3 minutes and then the sterilizing effect was measured at each ratio (by weight%) of the added formulation.

### 5-2. Evaluation of sterilizing power of shampoo composition

### (1) Evaluation of bactericidal power against Malassezia furfur

As described above, a sterilization experiment was conducted using a shampoo composition formulation containing an anti-dandruff hair care (antifungal) composition by briefly bringing the shampoo composition formulation into contact with the dandruff bacteria, *Malassezia furfur* for 3 minutes as in Experimental Example 4-2. Whether or not *Malassezia furfur* remained was determined and marked with O and X. The case where *Malassezia furfur* bacteria remained was marked as "O" and the case where all *Malassezia furfur* bacteria were removed was marked as "X".

Table 57 shows the sterilizing effect against *Malassezia furfur* of shampoo composition formulations prepared using different contents of the anti-dandruff hair care (antifungal) composition containing the compound of Formula 1, piroctone olamine (PO), zinc pyrithione (ZPT), or climbazole (CB) alone, or different contents of the anti-dandruff hair care (antifungal) composition containing a combination of the compound of Formula 1 and piroctone olamine (PO), zinc pyrithione (ZPT), or climbazole (CB) at a ratio of 5:5.

**[Table 57]**

| ratio of anti-dandruff hair care (antifungal) composition | 0.2wt% | 0.3wt% | 0.4wt% | 0.5wt% | 0.6wt% | 0.7wt% |
|---|---|---|---|---|---|---|
| Formula1 | O | O | O | O | O | X |
| PO | O | O | X | X | X | X |
| ZPT | O | O | X | X | X | X |
| CB | O | O | X | X | X | X |
| Formula1:PO=5:5 | O | X | X | X | X | X |
| Formula1:ZPT=5:5 | O | O | X | X | X | X |
| Formula1:CB=5:5 | O | X | X | X | X | X |

The result showed that the sterilizing power against *Malassezia furfur* increased as the content of the anti-dandruff hair care (antifungal) composition in the shampoo composition increased. Furthermore, compared to a shampoo composition containing the compound of Formula 1, piroctone olamine (PO), zinc pyrithione (ZPT), or climbazole (CB) alone, a shampoo composition containing a combination of the compound of Formula 1, and piroctone olamine (PO), zinc pyrithione (ZPT) or climbazole (CB) at a weight ratio of 5:5 had excellent overall sterilizing power against *Malassezia furfur.*

Therefore, a combination of the compound of Formula 1, and piroctone olamine (PO), zinc pyrithione (ZPT) or climbazole (CB) at a weight ratio of 5:5 may be used to remove the dandruff bacterium *Malassezia furfur,* rather than using expensive piroctone olamine (PO), zinc pyrithione (ZPT), or climbazole (CB) alone.

### (2) Evaluation of bactericidal power against Malassezia restricta

As described above, a sterilization experiment was conducted using a shampoo composition formulation containing an anti-dandruff hair care (antifungal) composition by briefly bringing the shampoo composition formulation into contact with the dandruff bacteria, *Malassezia restricta* for 3 minutes as in Experimental Example 4-2. Whether or not *Malassezia restricta* remained was determined and marked with O and X. The case where *Malassezia restricta* bacteria remained was marked as "O" and the case where all *Malassezia restricta* bacteria were removed was marked as "X".

Table 58 shows the sterilizing effect against *Malassezia restricta* of shampoo composition formulations prepared using different contents of the anti-dandruff hair care (antifungal) composition containing the compound of Formula 1, piroctone olamine (PO), zinc pyrithione (ZPT), or climbazole (CB) alone, or different contents of the anti-dandruff hair care (antifungal) composition containing a combination of the compound of Formula 1 and piroctone olamine (PO), zinc pyrithione (ZPT), or climbazole (CB) at a ratio of 5:5.

**[Table 58]**

| ratio of anti-dandruff hair care (antifungal) composition | 0.2wt% | 0.3wt% | 0.4wt% | 0.5wt% | 0.6wt% | 0.7wt% |
|---|---|---|---|---|---|---|
| Formula1 | O | O | O | O | X | X |
| PO | O | O | X | X | X | X |
| ZPT | O | X | X | X | X | X |
| CB | O | X | X | X | X | X |
| Formula1:PO=5:5 | O | X | X | X | X | X |
| Formula1:ZPT=5:5 | O | X | X | X | X | X |
| Formula1:CB=5:5 | O | X | X | X | X | X |

The result showed that the sterilizing power against *Malassezia restricta* increased as the content of the anti-dandruff hair care (antifungal) composition in the shampoo composition increased. Furthermore, compared to a shampoo composition containing the compound of Formula 1, piroctone olamine (PO), zinc pyrithione (ZPT), or climbazole (CB) alone, a shampoo composition containing a combination of the compound of Formula 1, and piroctone olamine (PO), zinc pyrithione (ZPT) or climbazole (CB) at a weight ratio of 5:5 had excellent overall sterilizing power against *Malassezia restricta.*

Therefore, a combination of the compound of Formula 1, and piroctone olamine (PO), zinc pyrithione (ZPT) or climbazole (CB) at a weight ratio of 5:5 may be used to remove the dandruff bacterium *Malassezia restricta,* rather than using expensive piroctone olamine (PO), zinc pyrithione (ZPT), or climbazole (CB) alone.

### (3) Evaluation of sterilizing power against Malassezia globose

As described above, a sterilization experiment was conducted using a shampoo composition formulation containing an anti-dandruff hair care (antifungal) composition by briefly bringing the shampoo composition formulation into contact with the dandruff bacteria, *Malassezia globosa* for 3 minutes as in Experimental Example 4-2. Whether or not *Malassezia globosa* remained was determined and marked with O and X. The case where *Malassezia globosa* bacteria remained was marked as "O" and the case where all *Malassezia globosa* bacteria were removed was marked as "X".

Table 59 shows the sterilizing effect against *Malassezia globosa* of shampoo composition formulations prepared using different contents of the anti-dandruff hair care (antifungal) composition containing the compound of Formula 1, piroctone olamine (PO), zinc pyrithione (ZPT), or climbazole (CB) alone, or different contents of the anti-dandruff hair care (antifungal) composition containing a combination of the compound of Formula 1 and piroctone olamine (PO), zinc pyrithione (ZPT), or climbazole (CB).

**[Table 59]**

| ratio of anti-dandruff hair care (antifungal) composition | 0.2wt% | 0.3wt% | 0.4wt% | 0.5wt% | 0.6wt% | 0.7wt% |
|---|---|---|---|---|---|---|
| Formula1 | O | O | O | O | X | X |
| PO | O | O | X | X | X | X |
| ZPT | O | X | X | X | X | X |
| CB | O | X | X | X | X | X |
| Formula1:PO=5:5 | O | X | X | X | X | X |
| Formula1:ZPT=5:5 | O | X | X | X | X | X |
| Formula1:CB=5:5 | O | X | X | X | X | X |

The result showed that the sterilizing power against *Malassezia globosa* increased as the content of the anti-dandruff hair care (antifungal) composition in the shampoo composition increased. Furthermore, compared to a shampoo composition containing the compound of Formula 1, piroctone olamine (PO), zinc pyrithione (ZPT), or climbazole (CB) alone, a shampoo composition containing a combination of the compound of Formula 1, and piroctone olamine (PO), zinc pyrithione (ZPT) or climbazole (CB) at a weight ratio of 5:5 had excellent overall sterilizing power against *Malassezia globosa.*

Therefore, a combination of the compound of Formula 1, and piroctone olamine (PO), zinc pyrithione (ZPT) or climbazole (CB) at a weight ratio of 5:5 may be used to remove the dandruff bacterium *Malassezia globosa,* rather than using expensive piroctone olamine (PO), zinc pyrithione (ZPT), or climbazole (CB) alone.

In conclusion, the present invention is characterized in that the compound represented by Formula 1 is identified to be effective in removing dandruff bacteria. In addition, compared to using the compound represented by Formula 1 alone, a combination of the compound represented by Formula 1 with piroctone olamine, zinc pyrithione, or climbazole exhibits a synergistic effect of controlling dandruff bacteria and is capable of effectively controlling *Malassezia* sp. even at a small dose. Therefore, the present invention has the advantage of reducing the consumption of the compound represented by Formula 1 and piroctone olamine, zinc pyrithione, or climbazole.

In addition, in the present invention, even if more than 50% of piroctone olamine, zinc pyrithione, or climbazole in hair care compositions such as shampoos is replaced with the compound of Formula 1, an effect comparable to when 100% of piroctone olamine, zinc pyrithione, or climbazole can be obtained and dandruff bacteria can be controlled very economically because the compound of Formula 1 can be prepared at a much lower cost.

### [Test E: Antiviral effect]

### Example

### Example 1.

2 g of 3,3'-(dodecylimino)bispropane-1,2-diol represented by Formula 1 was diluted in 1 liter of water.

### Comparative Example

### Comparative Example 1.

2 g of a mixture of n-alkyldimethylethylbenzylammonium chloride and alkylbenzyldimethylammonium chloride in a weight ratio (w/w) of 1:1 was diluted in 1 liter of water.

### Comparative Example 2.

2 g of alkylbenzyldimethylammonium chloride (benzalkonium chloride) was diluted in 1 liter of water.

### Experiment example

### Experimental Example 1. Evaluation of antiviral effect on coronavirus (human coronavirus 229E)

### (1) Experimental method

Viral genomes that have invaded and replicated in host cells grow into viruses, move out from the host cells and then invade new host cells, and continue the replication process. During this process, the host cells that produced the virus die and traces of apoptosis called "plaques" were used as a unit to quantify the virus.

The antiviral test against coronavirus using the liquid samples of Example 1 and Comparative Examples 1 and 2 diluted in water was performed in accordance with ASTM E1052.

The sample was inoculated with a predetermined amount of virus and the contact virus and noncontact virus were serially diluted 10 times each. Viruses prepared by dilution factor were inoculated into host cells HCT-8 (human colon adenocarcinoma). The virus was subjected to adsorption (infection) in order for the virus to effectively penetrate into the host cells through the host cell surface receptor and a solid medium was used to prevent indiscriminate virus infection between host cells and effectively produce plaques.

The infected host cells were cultured on solid medium for a predetermined period of time and then were immobilized, plaques were stained using a dye that stains only active cells, and the number of plaques was counted. The plaque count unit is plaque forming unit (PFU), which was calculated by multiplying the number of plaques by the dilution factor. Antiviral activity is calculated as the log reduction of the virus in contact with the sample (test group) compared to the virus without contact with the sample (control group). A case where the log reduction is 4 or more, corresponding to 99.99% or more, is determined to have antiviral activity.

**[Table 60]**

| **Log reduction** | **Conversion to percentage** | |
|---|---|---|
| 1 or more | 90% or more | Less than 99% |
| 2 or more | 99% or more | Less than 99.9% |
| 3 or more | 99.9% or more | Less than 99.99% |
| 4 or more | 99.99% or more | Less than 99.999% |
| 5 or more | 99.999% or more | Less than 99.9999% |

### (2) Test results

By treating with the liquid samples of Example 1, Comparative Example 1, and Comparative Example 2 diluted in water, all of the liquid samples of Example 1, Comparative Example 1, and Comparative Example 2 exhibit remarkably decreased plaque forming units, compared to the viruses that do not contact the samples (control group) and thus are capable of controlling the coronavirus. In particular, treatment with the sample of Example 1 provides excellent coronavirus control ability and much remarkable coronavirus control effect than quaternary ammonium-based disinfectants of Comparative Examples 1 and 2. The test was repeated three times to determine reproducibility.

The quaternary ammonium-based disinfectants of Comparative Examples 1 and 2 are the most common commercially available cationic surfactant virus disinfectants. Cationic surfactants are known to have a high risk of adsorption in the lungs and to have adverse effects on the respiratory system. On the other hand, the compound of Example 1 represented by Formula 1 is non-ionic, is discharged rather than being adsorbed in the body, and is safe for the human body.

**[Table 61]**

| | | Virus concentration Infectivity titer (PFU/ml) | | Plaque forming unit (PFU) | |
|---|---|---|---|---|---|
| Example | 1 | 4.25X10⁶ | 2.72X10² | 3.42X10² | 3.25X10² |
| Comparative Example | 1 | 4.25X10⁶ | 2.25X10³ | 1.98X10³ | 2.66X10³ |
| | 2 | 4.25X10⁶ | 8.54X10² | 1.22X10³ | 1.86X10³ |

### Experimental Example 2. Evaluation of antiviral effect against influenza A virus (H1N1)

### (1) Test method

The antiviral test against influenza virus type A (H1N1) was conducted on the sample using a plaque reduction test based on ASTM E1052. The plaque reduction test is a method of directly quantifying the inhibition of infectious viruses by a test substance based on the count of plaques isolated from the cell culture. The plaque test is used as the "gold standard" for determining the concentration of infectious lytic virus even as methods and technologies for virus quantification continue to evolve. The virus continues the replication-lysis-infection cycle in the infected cells, destroying surrounding cells and forming increasingly distinct visible areas. At this time, the area of destroyed cells is called a "plaque" and visible plaques are generally formed within 2 to 14 days depending on virus proliferation and host cells. At this time, a monolayer of cells (MDCK cells) infected with the influenza virus is covered with an overlay medium to prevent the viral infection from spreading indiscriminately through mechanical or convective flow of the liquid medium while the virus proliferates, thereby preventing the creation of viral plaques (whether or not plaques are reduced was determined using fixed layer technique).

The plaque count unit is plaque forming unit (PFU), which was calculated by multiplying the number of plaques by the dilution factor as in Experimental Example 1. Antiviral activity is calculated as the log reduction of the virus in contact with the sample (test group) compared to the virus without contact with the sample (control group). A case where the log reduction is 4 or more, corresponding to 99.99% or more, is determined to have antiviral activity.

### (2) Test results

By treating with the liquid samples of Example 1, Comparative Example 1, and Comparative Example 2 diluted in water, all of the liquid samples of Example 1, Comparative Example 1, and Comparative Example 2 exhibited remarkably decreased plaque forming units, compared to the viruses that do not contact the samples (control group) and thus were capable of controlling the influenza A virus (H1N1). In particular, treatment with the sample of Example 1 provided excellent influenza A virus (H1N1) control ability and much remarkable influenza A virus (H1N1) control effect than quaternary ammonium-based disinfectants of Comparative Examples 1 and 2. The test was repeated three times to determine reproducibility.

**[Table 62]**

| | | Virus concentration Infectivity titer (PFU/ml) | | Plaque forming unit (PFU) | |
|---|---|---|---|---|---|
| Example | 1 | 3.98X10⁶ | 2.88X10² | 3.14X10² | 3.26X10² |
| Comparative Example | 1 | 3.98X10⁶ | 2.S1X10³ | 1.87X10³ | 2.46X10³ |
| | 2 | 3.98X10⁶ | 8.67X10² | 1.69X10³ | 1.87X10³ |

### Experimental Example 3. Evaluation of antiviral effect against Avian influenza virus

### (1) Test method

200 PFU/well of avian influenza virus was inoculated into a previously prepared 6-well plate, and 1 hour later, all virus inoculum was removed. The cells (MDCK cells) were washed with serum-free culture medium, the test substances (samples) were dispensed and cultured in a CO₂ incubator at 37°C for 1 hour. To observe normal plaque formation, the treatment time of the test substance was 2 hours or less. The test was repeated twice and included a negative control (virus only) that was not treated with the test substance.

After treatment with the test substance, the test substance solution was removed from the plate, 3 mL of agar medium prepared at 4% FBS DMEM:3% agarose (1:1) was slowly dispensed onto the wall of the plate until the agar was allowed to harden, and the cells were cultured at 37°C in a CO₂ incubator for 3 days. The cells in each well were stained with a crystal violet solution and allowed to stand at room temperature to stain the cells. After at least 6 hours, the staining solution and agar were removed and the plate was washed with water. The plate was sufficiently dried at room temperature and then plaques in each well were observed and counted. The plaque count unit is plaque forming unit (PFU), which was calculated by multiplying the number of plaques by the dilution factor. Antiviral activity is calculated as the log reduction of the virus in contact with the sample (test group) compared to the virus without contact with the sample (control group). A case where the log reduction is 4 or more, corresponding to 99.99% or more, is determined to have antiviral activity.

### (2) Test results

By treating with the liquid samples of Example 1, Comparative Example 1, and Comparative Example 2 diluted in water, all of the liquid samples of Example 1, Comparative Example 1, and Comparative Example 2 exhibited remarkably decreased plaque forming units, compared to the viruses that do not contact the samples (control group), and thus were capable of controlling Avian influenza virus. In particular, treatment with the sample of Example 1 provided excellent Avian influenza virus control ability and much remarkable Avian influenza virus control effect than quaternary ammonium-based disinfectants of Comparative Examples 1 and 2. The test was repeated three times to determine reproducibility.

**[Table 63]**

| | | Virus concentration Infectivity titer (PFU/ml) | | Plaque forming unit (PFU) | |
|---|---|---|---|---|---|
| Example | 1 | 4.38X10⁶ | 2.89X10² | 3.42X10² | 3.19X10² |
| Comparative Example | 1 | 4.38X10⁶ | 2.48X10³ | 1.65X10³ | 8.79X10² |
| | 2 | 4.38X10⁶ | 8.56X10² | 7.56X10² | 1.35X10³ |

It will be apparent to those skilled in the art that various modifications and variations can be made in the disclosure without departing from the spirit or scope of the disclosure. Thus, it is intended that the disclosure cover such modifications and variations thereof, provided they fall within the scope of the appended claims and their equivalents.

## Claims

1. An antimicrobial and preservative composition containing a compound represented by Formula 1: 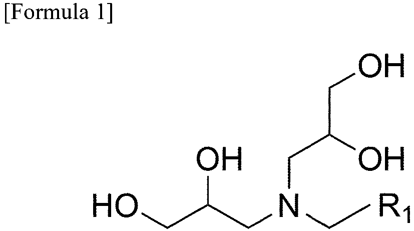 wherein R₁ is substituted or unsubstituted C₇-C₁₇ alkyl or substituted or unsubstituted C₇-C₁₇ alkenyl.

2. The antimicrobial and preservative composition according to claim 1, further comprising a compound represented by Formula 2, Formula 3 or Formula 4. 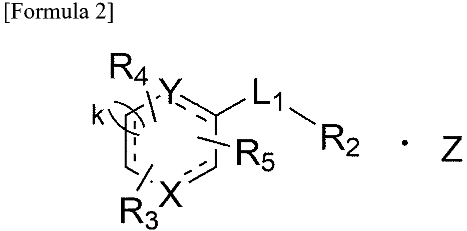 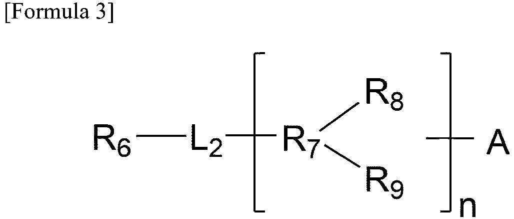 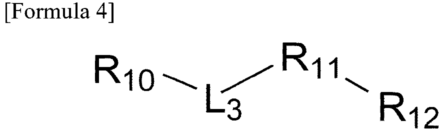 wherein
in Formula 2,
L₁ is -(CH=CH)-, -(CH₂)₁-, -C(=O)- or -O-,
R₂ is -H, -C(=O)-CH₃, -C(=O)H, -OH, -O⁻Na⁺, -(CH₂)ₘ-OH, -CH(CH₃)-CH₂-C(CH₃)₂-CH₃, or substituted or unsubstituted C₁-C₆ alkyl,
R₃, R₄ and R₅ are each independently -H, -OH, C₁-C₃ alkoxy, =O, -O⁻Na⁺, or substituted or unsubstituted C₁-C₆ alkyl,
X and Y are each independently -CH-, -O- or -NRₐ,
Rₐ is -OH,
Z is NH₂-CH₂-CH₂-OH or null,
- - - represents a single or double bond,
K, 1, and m are each independently an integer of 1 to 4,
in Formula 3,
R₆ is -H, substituted or unsubstituted C₂-C₁₈ alkyl, or substituted or unsubstituted C₂-C₁₈ alkenyl,
L₂ is -(CH₂)ₒ-, -O- or -C(=O)-,
R₇ is -NHCH-, -N- or C(=O)CHNH-,
R₈ is -OH, COOH, -COOCH₃, -COOCH₂CH₃, -(CH₂)₄NH- or 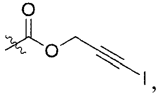
R₉ is -H, -(CH₂)₄NH₂ or -(CH₂)₃C(NH)₂NH₂,
A is -H or null,
n is an integer of 1 to 99, and
o is an integer of 1 to 3, and
in Formula 4,
L₃ is -(CH₂)ₚ-, -O-, -C(=O)- or -O-C(=O)-,
R₁₀ is substituted or unsubstituted C₂-C₁₀ alkyl, -OH or -O⁻Na⁺,
R₁₁ is -(CH₂)_{q}- substituted or unsubstituted with at least one substituent selected from the group consisting of -CH₂-CHOH-CH₂-, hydroxy, unsubstituted C₁-C₃ alkyl, and carboxyl, substituted or unsubstituted -(CH₂)ᵣ-N(R_{b})-(CH₂)ₛ-, or unsubstituted phenylene (-C₆H₄-),
R₁₂ is -OH, -COOH, C₁-C₃ alkoxy or -COO⁻Na⁺,
R_{b} is -(CH₂)ₜ-N[(CH₂)ᵤ-CO₂⁻)]₂, and
p, q, r, s, t, and u are each independently an integer of 1 to 3.

3. The antimicrobial and preservative composition according to claim 1, wherein the antibacterial and preservation composition has an antibacterial or antifungal effect on bacteria or fungi.

4. The antimicrobial and preservative composition according to claim 1, wherein the antibacterial and preservation composition controls at least one microorganism selected from the group consisting *of Escherichia coli, Staphylococcus aureus, Pseudomonas aeruginosa, Klebsiella pneumoniae,* MRSA *(methicillin-resistant Staphylococcus aureus), Bacillus subtilis, Vibrio parahaemolyticus, Salmonella typhimurium, Listeria monocytogenes, Shigella flexneri, Candida albicans, Aspergillus niger,* and *Aspergillus brasiliensis.*

5. The antimicrobial and preservative composition according to claim 2, wherein the compound represented by Formula 1, and the compound represented by Formula 2, Formula 3, or Formula 4 are present in a weight ratio of 0.01:9.99 to 9.99:0.01.

6. The antimicrobial and preservative composition according to claim 1, wherein the compound represented by Formula 1 is 3,3'-(dodecylimino)bispropane-1,2-diol.

7. The antimicrobial and preservative composition according to claim 2, wherein the compound represented by Formula 2 comprises at least one selected from the group consisting of o-methoxycinnamaldehyde, 4-methoxycinnamaldehyde, raspberry ketone, 3-phenyl-1-propanol, hydroxy acetophenone, sodium benzoate, phenoxyethanol, tropolone, piroctone olamine, and sodium dehydroacetate.

8. The antimicrobial and preservative composition according to claim 2, wherein the compound represented by Formula 3 comprises at least one selected from the group consisting of lauroyl arginine methyl ester, lauroyl lysine methyl ester, lauroyl arginine ethyl ester, iodopropynyl butylcarbamate, polylysine, lauroyl lysine, and caprylhydroxamic acid.

9. The antimicrobial and preservative composition according to claim 2, wherein the compound represented by Formula 4 comprises at least one selected from the group consisting of hexane-1,2-diol, octane-1,2-diol, decane-1,2-diol, ethylhexylglycerin, glyceryl monocaprylate, octyl glycerin, hexyl glycerin, levulinic acid, citric acid, sodium anisate, disodium EDTA, and tetrasodium EDTA.

10. A product comprising the antimicrobial and preservative composition according to claim 1 or 2.

11. The product according to claim 10, wherein the product is a cosmetic product, sanitary product or household chemical product.

12. The product according to claim **11,** wherein the cosmetic product is formulated into any one selected from the group consisting of solutions, suspensions, emulsions, pastes, gels, creams, pacts, powders, emulsion foundations, wax foundations, sprays, soaps, face cleansers, body cleansers, hair shampoos and hair rinses.

13. The product according to claim **11,** wherein the sanitary product is selected from the group consisting of dishwashing detergents, dishwasher detergents, and wet wipes.

14. The product according to claim **11,** wherein the household chemical product is selected from the group consisting of laundry detergents, residential cleaners, deodorants, air cleaners, indoor air fresheners, fragrances, fabric softeners, multi-purpose cleaners, disinfectants, antimicrobials, sanitizers, and fungistats.

15. The product according to claim 11, wherein the antimicrobial and preservative composition is present in an amount of 0.01 to 10% by weight with respect to the weight of the cosmetic, sanitary or household chemical product.

16. An antibacterial method comprising treating bacteria with the antimicrobial and preservative composition according to claim 1 or 2.

17. An anti-dandruff hair care cosmetic composition comprising a compound represented by Formula 1: 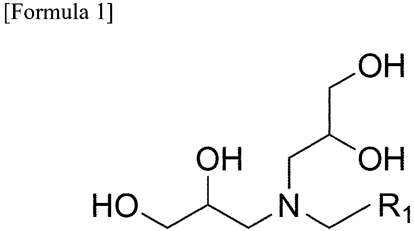 wherein R₁ is linear or branched C₇-C₁₇ alkyl, linear or branched C₇-C₁₇ alkenyl, or C₇-C₁₇ hydroxyalkyl.

18. The anti-dandruff hair care cosmetic composition according to claim 17, further comprising a compound represented by Formula 5 below:
[Formula 5] R₁₃ - L₄ · W
wherein
L₄ is 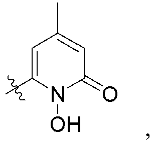 -Zn or 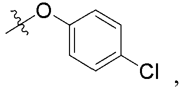
R₁₃ is CH₃-C(CH₃)₂-CH₂-CH(CH₃)-CH₂-, 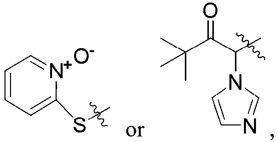
W is NH₂-CH₂-CH₂-OH, 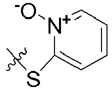 or null.

19. The anti-dandruff hair care cosmetic composition according to claim 18, wherein the compound represented by Formula 1 and the compound represented by Formula 5 are present in the hair care cosmetic composition at a weight ratio of 9:1 to 1:9.

20. The anti-dandruff hair care cosmetic composition according to claim 17, wherein the cosmetic composition has sterilizing activity against strains of *Malassezia* sp.

21. The anti-dandruff hair care cosmetic composition according to claim 17, wherein the anti-dandruff hair care cosmetic composition has sterilizing activity against at least one fungus selected from the group consisting of *Malassezia furfur, Malassezia restricta, Malassezia globosa, Malassezia sympodialis, Malassezia slooffiae, Malassezia pachydermatis,* and *Malassezia obtusa.*

22. The anti-dandruff hair care cosmetic composition according to claim 17, wherein the compound represented by Formula 1 is 3,3'-(dodecylimino)bispropane-1,2-diol.

23. An antifungal composition against strains of *Malassezia sp.,* comprising a compound represented by Formula 1: 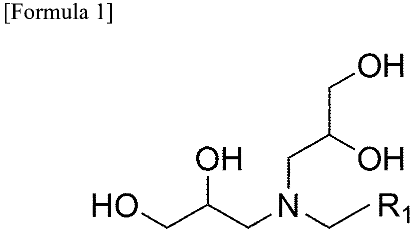 wherein R₁ is linear or branched C₇-C₁₇ alkyl, linear or branched C₇-C₁₇ alkenyl, or C₇-C₁₇ hydroxyalkyl.

24. The antifungal composition according to claim 23, further comprising the compound represented by Formula 5.

25. The antifungal composition according to claim 23, wherein the antifungal composition has sterilizing activity against at least one fungus selected from the group consisting of *Malassezia furfur, Malassezia restricta, Malassezia globosa, Malassezia sympodialis, Malassezia slooffiae, Malassezia pachydermatis, and Malassezia obtusa.*

26. A hair care cosmetic product comprising the composition according to any one of claims 17 to 25.

27. A hair care cosmetic product according to claim 26, wherein the composition is present in an amount of 0.01 to 2.0% by weight with respect to the weight of the hair care cosmetic product.

28. The hair care cosmetic product according to claim 26, wherein the hair care cosmetic product is formulated into any one selected from the group consisting of hair tonic, hair cream, hair pack, hair lotion, hair essence, hair nutrition, hair shampoo, hair rinse, hair conditioner, hair spray, hair gel, hair treatment, hair oil, hair wax, hair bleach and hair dye.

29. A method of sterilizing dandruff bacteria including treating the dandruff bacteria with the composition according to any one of claims 17 to 25.

30. An antiviral composition comprising, as an active ingredient, the compound represented by Formula 1: 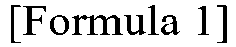 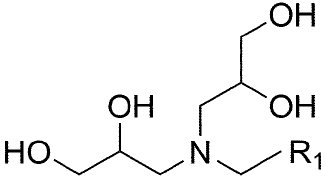 wherein R₁ is substituted or unsubstituted C₇-C₁₇ alkyl or substituted or unsubstituted C₇-C₁₇ alkenyl.

31. The antiviral composition according to claim 30, wherein the compound represented by Formula 1 is 3,3'-(dodecylimino)bispropane-1,2-diol.

32. The antiviral composition according to claim 30, wherein the antiviral compound is diluted as a 0.1 to 5% (w/v) aqueous solution.

33. The antiviral composition according to claim 30, wherein the virus comprises one or more selected from the group consisting of coronavirus, influenza virus, norovirus, rotavirus, respiratory syncytial virus, avian influenza virus, foot-and-mouth disease virus, and African swine fever virus.

34. An antiviral method including treating virus with the antiviral composition according to claim 30.
